(19)

Europäisches Patentamt

European Patent Office

Office européen des brevets

(11) **EP 4 019 047 A1**

(12) **EUROPEAN PATENT APPLICATION**
published in accordance with Art. 153(4) EPC

(43) Date of publication:
**29.06.2022 Bulletin 2022/26**

(21) Application number: **20853868.6**

(22) Date of filing: **21.08.2020**

(51) International Patent Classification (IPC):
*A61K 39/395* (2006.01)   *A61K 9/08* (2006.01)
*A61K 47/12* (2006.01)   *A61K 47/18* (2017.01)
*A61K 47/26* (2006.01)   *A61P 35/00* (2006.01)

(86) International application number:
**PCT/RU2020/050197**

(87) International publication number:
**WO 2021/034228 (25.02.2021 Gazette 2021/08)**

(84) Designated Contracting States:
**AL AT BE BG CH CY CZ DE DK EE ES FI FR GB GR HR HU IE IS IT LI LT LU LV MC MK MT NL NO PL PT RO RS SE SI SK SM TR**
Designated Extension States:
**BA ME**
Designated Validation States:
**KH MA MD TN**

(30) Priority: **22.08.2019 RU 2019126511**

(71) Applicant: **Joint Stock Company "Biocad"**
**St.Petersburg 198515 (RU)**

(72) Inventors:
• **LOMKOVA, Ekaterina Aleksandrovna**
**Saint Petersburg, 196066 (RU)**

• **SHUSTOVA, Mariia Stanislavovna**
**Saint-Petersburg, 196135 (RU)**
• **TSUKUR, Alina Aleksandrovna**
**Saint-Petersburg, 193313 (RU)**
• **IAKOVLEV, Aleksandr Olegovich**
**Moscow, 109651 (RU)**
• **KOZLOVA, Olesya Nikolaevna**
**Telmana, 187032 (RU)**
• **SHITIKOVA, Viktoriia Olegovna**
**Saint- Petersburg, 195213 (RU)**
• **MOROZOV, Dmitry Valentinovich**
**Saint Petersburg, 190000 (RU)**

(74) Representative: **Held, Stephan**
**Meissner Bolte Patentanwälte**
**Rechtsanwälte Partnerschaft mbB**
**Widenmayerstraße 47**
**80538 München (DE)**

(54) **AQUEOUS PHARMACEUTICAL COMPOSITION OF ANTI-PD1 ANTIBODY PROLGOLIMAB AND USE THEREOF**

(57)   The present invention relates to the aqueous pharmaceutical compositions for the anti-PD-1 antibody prolgolimab and to the use of such pharmaceutical compositions as a medicinal agent for the treatment of PD-1-mediated diseases.

EP 4 019 047 A1

**Description**

Field of the invention

The present invention relates to the novel aqueous compositions for the anti-PD-1 antibodies, and in particular to novel aqueous compositions for anti-PD-1 antibody prolgolimab, which can be used as a medicinal agent for the treatment of malignant neoplasms.

Background of the invention

Programmed death 1 (PD-1) protein is an inhibitory member of the CD28 receptor family that also includes the CD28, the CTLA-4, the ICOS and the BTLA. PD-1 is expressed by activated B cells, T cells, and myeloid cells (Agata et al., supra; Okazaki et al. (2002) Curr. Opin. Immunol. 14: 391779-82; Bennet et al. (2003) J Immunol 170:711-8). The initial members of this family, CD28 and ICOS, were detected by functional effects on increase in the T cell proliferation following the addition of monoclonal antibodies (Hutloff et al. (1999) Nature 397:263-266; Hansen et al. (1980) Immunogenics 10:247-260). PD-1 was detected by screening for differential expression in apoptotic cells (Ishida et al. (1992) EMBO J 11:3887-95). Other members of this family, CTLA-4 and BTLA, were detected by screening for the differential expression in cytotoxic T-lymphocytes and TH1 cells, respectively. The CD28, the ICOS and the CTLA-4, all have an unpaired cysteine residue that allows them to homodimerize. In contrast, PD-1 is believed to exist as a monomer, lacking the unpaired cysteine residue characteristic of other members of the CD28 family.

PD-1 is a 55 kDa type I transmembrane protein that is a member of the Ig gene superfamily (Agata et al. (1996) Int Immunol 8:765-72). PD-1 comprises a membrane proximal immunoreceptor tyrosine inhibitory motif (ITIM) and a membrane distal tyrosine-based switch motif (ITSM) (Thomas, M.L. (1995) J Exp Med 181:1953-6; Vivier, E и Daeron, M (1997) Immunol Today 18:286-91). Although structurally similar to CTLA-4, PD-1 lacks the MYPPY motif that is critical for B7-1 and B7-2 binding. It has been detected that PD-1 has two ligands, PD-L1 and PD-L2, which have been shown to negatively regulate T cell activation after binding to PD-1 (Freeman et al. (2000) J Exp Med 192:1027-34; Latchman et al. (2001) Nat Immunol 2:261-8; Carter et al. (2002) Eur J Immunol 32:634-43). Both PD-L1 and PD-L2 are B7 homologs that bind to PD-1, but do not bind to other members of the CD28 family.

One PD-1 ligand, PD-L1, is abundant in various types of the human cancers (Dong et al. (2002) Nat. Med. 8:787-9). The interaction between PD-1 and PD-L1 leads to a reduction in the number of tumor-infiltrating lymphocytes, decrease in T cell receptor-mediated proliferation, and cancer cell escape from immunological surveillance (Dong et al. (2003) J. Mol. Med. 81:281-7; Blank et al. (2005) Cancer Immunol. Immunother. 54:307-314; Konishi et al. (2004) Clin. Cancer Res. 10:5094-100). Immunosuppression may be reversed by inhibiting a local PD-L1/PD-1 interaction, and this effect is additive when blocking the PD-L2/PD-1 interaction (Iwai et al. (2002) Proc. Nat'l. Acad. Sci. USA 99:12293-7; Brown et al. (2003) J. Immunol. 170:1257-66).

PD-1 is an inhibitory member of the CD28 family and is expressed on activated B cells, T cells and myeloid cells (Agata et al., supra; Okazaki et al. (2002) Curr Opin Immunol 14: 391779-82; Bennett et al. (2003) J Immunol 170:711-8). PD-1-deficient animals are prone to develop various autoimmune diseases including autoimmune cardiopathy, and lupus-like syndrome comprised of arthritis and nephritis (Nishimura et al. (1999) Immunity 11:141-51; Nishimura et al. (2001) Science 291:319-22). In addition, PD-1 was found to play a role in autoimmune encephalomyelitis, systemic lupus erythematosus, graft-versus-host disease (GVHD), type I diabetes and rheumatoid arthritis (Salama et al. (2003) J Exp Med 198:71-78; Prokunina and Alarcon-Riquelme (2004) Hum Mol Genet 13:R143; Nielsen et al. (2004) Lupus 13:510). In a murine B cell tumor line, the ITSM of PD-1 was shown to be essential to block BCR-mediated Ca2+-flux and tyrosine phosphorylation of downstream effector molecules (Okazaki et al. (2001) PNAS 98:13866-71).

A number of anti-PD-1 antibodies are known in the art, for example, nivolumab (BMS), pembrolizumab (Merck), which are a human IgG4 monoclonal antibody.

Also known is a novel anti-PDI antibody prolgolimab (also known as BCD-100), which is a monoclonal human antibody of the IgG1 isotype non-effector mutations L234A, L235A. Prolgolimab has shown increased affinity to PD-1, increased aggregation stability as compared to IgG4 antibodies. Furthermore, prolgolimab is currently under clinical trials for various types of malignant neoplasms, including melanoma, including inoperable or metastatic melanoma, early stages of melanoma before and after definitive treatment; lung cancer, non-small cell lung cancer (NSCLC), including inoperable or metastatic non-small cell lung cancer.

In connection with the above, it is relevant to create novel improved stable aqueous pharmaceutical compositions for anti-PD-1 antibody prolgolimab.

Brief description of drawings

The invention will be better understood by way of the following detailed description of embodiments of the invention with reference to the appended drawings, in which:

Figure 1, Figure 2 are diagrams illustrating the dynamics of BCD-100 concentrations in patients' blood serum during 6 administrations (adjusted for coefficient) in μg/ml (BCD-100-1 trial) .
Figure 3 is a diagram illustrating the design of BCD-100-2/MIRACULUM trial.
Figure 4 is a diagram illustrating the schematic of the trial.
Figure 5 is a diagram illustrating overall survival of patients in the 1st group (BCD-100, 1 mg/kg once in 2 weeks) according to the results of the BCD-100-2/MIRACULUM trial.
Figure 6 is a diagram illustrating overall survival of patients in the 2nd group (BCD-100, 3 mg/kg once in 3 weeks) according to the results of the BCD-100-2/MIRACULUM trial.
Figure 7 is a diagram illustrating progression-free survival of patients in the BCD-100 1 mg/kg group (based on irRECIST criteria) according to the results of the BCD-100-2/MIRACULUM trial.
Figure 8 is a diagram illustrating progression-free survival of patients in the BCD-100 3 mg/kg group (based on irRECIST criteria) according to the results of the BCD-100-2/MIRACULUM trial.
Figure 9 is a diagram illustrating BCD-100 concentration in patients who received 1 mg/kg every 2 weeks following administration of a single dose of the product (results of the BCD-100-2/MIRACULUM trial).
Figure 10 is a diagram illustrating BCD-100 concentration in patients who received 3 mg/kg every 3 weeks (results of the BCD-100-2/MIRACULUM trial).
Figure 11 is a diagram illustrating the portion of Th9 in the overall population of helper T cells in subgroups of patients who received BCD-100 at a dose of 1 mg/kg Q2W, exhibiting different types of responses to therapy (results of the BCD-100-2/MIRACULUM trial).
Figure 12 is a diagram illustrating the portion of Th9 in the overall population of helper T cells in subgroups of patients who received BCD-100 at a dose of 3 mg/kg Q3W, exhibiting different types of responses to therapy (results of the BCD-100-2/MIRACULUM trial).

Description of the invention

Definitions:

The terms used in this description generally have their ordinary meanings in the art, within the context of the invention, and in the specific context where each term is used. Certain terms that are used to describe the invention are discussed below, or elsewhere in the description, to provide additional guidance to the practitioner regarding the description of the invention. Synonyms for certain terms are provided. A recital of one or more synonyms does not exclude the use of other synonyms. The use of examples anywhere in this description, including the examples of any terms discussed herein, is illustrative only, and in no way limits the scope and meaning of the invention or of any exemplified term. The invention is not limited to various embodiments given in this description.

"Monoclonal antibody" as used herein relates to a humanized antibody or fully human antibody, unless otherwise stated in the present application. Monoclonal antibodies according to the invention can be obtained using, for example, recombinant technology, phage display technology, synthetic technology or the combinations of these or other technologies well known from the prior art.

The population of "monoclonal antibodies" as used herein refers to a homogenous or essentially homogeneous antibody population (i.e. at least or 96%, but more preferably no less than about 97 or 98%, or further preferably at least 99% of antibodies in the population will compete for the same antigen/epitope in the enzyme-linked immunosorbent assay ELISA, or further preferably antibodies are identical regarding their amino acid sequences).

A native full-size antibody is represented by immunoglobulin molecule comprising four polypeptide chains (two heavy H chains of about 50-70 KDa for the full length, and two light L chains of about 25 KDa for the full length) linked via disulfide bonds. Amino-terminal part of each chain comprises a variable domain of about 100-110 or more amino acids that are responsible for binding an antigen. Carboxyl-terminal domain of each chain determines the constant region that is mostly responsible for the effector function. Light chains are classified as kappa and lambda and characterized by a specific constant region. Each light chain is characterized in comprising a variable N-terminal light chain region (hereafter referred to as VL or VK) and a constant light chain region that consists of a single domain (CL or CK). Heavy chains are classified as $\gamma$, $\delta$, $\alpha$, $\mu$, and $\varepsilon$ and define classes of immunoglobulins: IgG, IgM, IgA, IgD and IgE, respectively; some of them can be additionally divided into sub-classes (isotypes) such as IgG1, IgG2, IgG3, IgG4, IgA1 and IgA2. Each heavy chain type is characterized by a particular constant region, Fc. Each heavy chain comprises a variable N-terminal region (hereafter referred to as VH) and constant region CH. Constant heavy chain region consists of three domains (CH1, CH2 and CH3) for IgG, IgD and IgA, and of 4 domains (CH1, CH2, CH3 and CH4) for IgM and IgE. VH and VL variable domains can also be divided into so-called hypervariable regions (complementarity determining regions, CDR) interspersing with more conservative framework regions (FR). Each variable domain comprises three CDRs and four FRs located in the following order from N-terminus to C-terminus: FR1, CDR1, FR2, CDR2, FR3, CDR3 and FR4.

Variable regions of each light/heavy chain pair form antigen-binding sites of an antibody. Thus, an intact IgG antibody has two binding sites. Except for bi-functional or bi-specific antibodies, the two binding sites are identical. As used herein, "antigen-binding portion" or "antigen-binding region", or "antigen-binding domain", are interchangeable with refer to an antibody region comprising amino acid residues interacting with an antigen and giving the antibody its specificity and affinity to an antigen. This antibody fragment includes the frame amino acid residues necessary for maintaining the proper conformation of antigen-binding residues.

"Antibody fragment" may be represented by an antibody fragment or antibody fragment that has the activity of a full-size antibody. Said antibody fragment may be represented by F(ab')2, F(ab)2, Fab', Fab Fv and scFv.

As used herein, the terms "inhibit" or "neutralize" regarding the activity of an antibody of the present invention shall mean the ability to block, prevent, restrict, slow down, stop, reduce or reverse significantly, for example, the development or severity of inhibition subject, including but not limited to biological activity (such as activity of PD-1) or property, disease or condition. The inhibition or neutralization of activity of PD-1 resulted from binding of an antibody of the invention to PD-1 is preferably at least about 20%, 30%, 40%, 50%, 60%, 70%, 80%, 90%, 95% or higher.

The term "separated" or "isolated" with regard to nucleic acids or protein products (such as an antibody) refers to the nucleic acid molecule or protein molecule that is identified and separated from at least one of contaminating substances to which it is usually combined in the natural source. Preferably, an "isolated antibody" is an antibody that substantially contains no other antibodies that have particular antigenic specificity (for example, pharmaceutical compositions of the present invention contain an isolated antibody that specifically binds PD-1 and substantially contain no antibodies that specifically bind antigens other than PD-1).

Polynucleotide is "functionally bound" if it has functional linkages to other polynucleotide. For example, promoter or enhancer is functionally bound to the coding sequence if it affects the sequence transcription. Polypeptide is "functionally bound" to another polypeptide if polynucleotides coding thereof is functionally bound, preferably if they are located in the same open reading frame.

The term "specific binding" between an antibody and an antigen target (antigen) refers to immunological specificity. Antibody can specifically bind an antigen target if it binds an antigen epitope stronger than other antigen epitopes. Specific binding does not exclude the cross-reactivity with other antigens that carry similar antigen epitopes.

VL domains in antibodies of the invention can be either VL lambda type or VL kappa type. The term "VL domain" covers both VL lambda and VL kappa isotypes that contain one or more amino acid substitutions, insertions or deletions.

The term "pharmaceutical composition" refers to a composition and/or formulation containing a therapeutically effective amount of an antibody of the invention plus excipients (carriers, diluents, vehicles, solvents and other excipients).

The term "buffer" or "buffer solution" refers herein to an aqueous solution comprising a mixture of an acid (typically a weak acid, such as, e.g. acetic acid, citric acid) and a conjugate base thereof (such as e.g. an acetate or citrate salt, e.g. sodium acetate, sodium citrate, as well as hydrates of said salts, e.g. sodium acetate trihydrate) or alternatively a mixture of a base (typically a weak base, e.g. histidine) and conjugate acid thereof (e.g. histidine hydrochloride). The pH of a "buffer solution" will change only slightly upon addition of a small quantity of strong base or strong acid, as well as upon dilution or concentration due to the "buffering effect" imparted by the "buffering agent".

Herein, a "buffer system" comprises one or more buffering agent(s) and/or an acid/base conjugate(s) thereof, and more suitably comprises one or more buffering agent(s) and an acid/base conjugate(s) thereof, and most suitably comprises one buffering agent and an acid/base conjugate thereof. Unless specified otherwise, any concentrations referred herein to a "buffer system" (a buffer concentration) may suitably refer to the combined concentration of buffering agent(s) and/or acid/base conjugate(s) thereof. In other words, concentrations referred herein to a "buffer system" may refer to the combined concentration of all the relevant buffering species (i.e. the species in dynamic equilibrium with one another, e.g. citrate/citric acid). The overall pH of the composition comprising the relevant buffer system is a reflection of the equilibrium concentration of each of the relevant buffering species (i.e. the balance of buffering agent(s) to acid/base conjugate(s) thereof).

The term "buffering agent" refers herein to an acid or base component (typically a weak acid or weak base) of a buffer or buffer solution. A buffering agent helps to maintain the pH of a given solution at or near to a pre-determined value, and the buffering agents are generally chosen to complement the pre-determined value. A buffering agent may be a single compound which gives rise to a desired buffering effect, especially when said buffering agent is mixed with (and suitably capable of proton exchange with) an appropriate amount (depending on the pre-determined pH desired) of its corresponding "acid/base conjugate".

As used herein, the term "solubilizer" refers to a pharmaceutically acceptable non-ionic surfactant. Both one solubilizer and combinations thereof can be used. Exemplary solubilizers are, without limitation, polysorbate 20 or polysorbate 80, Poloxamer 184 or Poloxamer 188, or PLURONIC®.

The terms "osmotic agent" or "tonicity agent", as well as "osmolyte", as used herein, refer to an excipient that can provide the required osmotic pressure of a liquid antibody solution. In some embodiments, a tonicity agent can increase the osmotic pressure of a liquid antibody formulation to isotonic pressure such that said liquid antibody formulation is physiologically compatible with the cells of a tissue of a subject's organism. In another embodiment, a tonicity agent can contribute to the increase in stability of antibodies. "Isotonic" drug is a drug that has an osmotic pressure equivalent to that of human blood. Isotonic drugs typically have an osmotic pressure from about 250 to 350 mOsm/kg. The term "hypotonic" describes a formulation with an osmotic pressure below that of human blood. Correspondingly, the term "hypertonic" is used to describe a formulation with an osmotic pressure above that of human blood. Isotonicity can be measured using, e.g. a vapor pressure or cryoscopic osmometer. A tonicity agent can be in an enantiomeric (e.g. L- or D-enantiomer) or racemic form; in the form of isomers such as alpha or beta, including alpha, alpha; or beta, beta; or alpha, beta; or beta, alpha; in the form of a free acid or free base; in the form of a salt; in a hydrated form (e.g. monohydrate), or in an anhydrous form. Exemplary osmotic agents are but not limited to sugars (trehalose dihydrate, sucrose, glucose), polyols (mannitol, sorbitol), amino acids (proline, arginine, glycine), or salts (sodium chloride, potassium chloride, magnesium chloride).

The term "long-term storage" or "long term stability" is understood to mean that the pharmaceutical composition can be stored for three months or more, for six months or more, and preferably for one year or more, most preferably with a minimum stable shelf life of at least two years. Generally speaking, the terms "long term storage" and "long term stability" further include stable storage durations that are at least comparable to or better than the stable shelf life typically required for currently available commercial formulations of the anti-PD-1 antibody prolgolimab, without losses in stability that would render the formulation unsuitable for its intended pharmaceutical application.

The term "parenteral administration" refers to administration regimens, typically by injection, and includes, in particular intravenous, intramuscular, intraarterial, intratracheal, intracapsular, intraorbital, intracardiac, intradermal, intraperitoneal, transtracheal, subcutaneous, intraarticular, subcapsular, subarchnoid, intraspinal, epidural and intrasternal injection or infusion.

The term "use" applies to the ability of using an antibody of the present invention or a pharmaceutical composition containing thereof to treat, relief the course of the disease, expedite the remission or reduce the recurrence rate for the disease or disorders mediated by receptors with which an antibody of the present invention can bind. Exemplary diseases are but not limited to malignant neoplasms, including melanoma, including inoperable or metastatic melanoma, early stages of melanoma before and after definitive treatment; lung cancer, non-small cell lung cancer (NSCLC), including inoperable or metastatic non-small cell lung cancer; non-squamous non-small cell lung cancer, squamous cell lung cancer; small cell lung cancer, including inoperable or metastatic small cell lung cancer; early stages of lung cancer before and after definitive treatment; cervical cancer, including metastatic cervical cancer, early stages of cervical cancer before and after definitive treatment; head and neck tumors, including head and neck squamous cell cancer; Hodgkin's lymphoma; stomach and bowel tumors, metastatic squamous cell esophageal cancer; bladder cancer, including metastatic urothelial carcinoma, kidney cancer; endometrial cancer, including metastatic endometrial cancer, early stages of endometrial cancer before and after definitive treatment; breast cancer, including metastatic breast cancer, early stages of endometrial cancer before and after definitive treatment; liver cancer, including metastatic or inoperable liver cancer, early stages of liver cancer before and after definitive treatment; inoperable or metastatic solid tumor, including inoperable or metastatic solid tumor with signs of microsatellite instability.

The term "method of treatment" applies to the ability of using an antibody of the present invention or a pharmaceutical composition containing thereof to treat, relief the course of the disease, expedite the remission or reduce the recurrence rate for the disease or disorders associated with PD1 activity. "Treat" or "treatment" of a disease, disorder or condition may comprise the prevention or delay of the onset of clinical symptoms of a disease, disorder or condition developing in human, the inhibition of a disease, disorder or condition, i.e. stop, reduction or delay of the development of a disease or a relapse thereof (in case of maintenance therapy) or at least one clinical or subclinical symptom thereof, or the alleviation or easement of a disease, i.e. the causing of regression of a disease, disorder or condition. Exemplary diseases are but not limited to malignant neoplasms, including melanoma, including inoperable or metastatic melanoma, early stages of melanoma before and after definitive surgical treatment; lung cancer, non-small cell lung cancer (NSCLC), including inoperable or metastatic non-small cell lung cancer.

The term "aqueous composition" as used herein refers to a water-based composition, the water in the composition may be: water, water for injections, physiologic saline (0.9%-1.0% aqueous solution of sodium chloride).

In one embodiment of the invention, the subject of treatment, or patient, is a mammal, preferably a human subject. Said subject may be either male or female, of any age.

As used in the present description, the words "comprise", "have", "include", or variations such as "comprises", "comprising", "has", "having", "includes" or "including", and all grammatical variations thereof will be understood to imply the inclusion of a stated integer or group of integers but not the exclusion of any other integer or group of integers.

## Summary of the invention

The present invention discloses aqueous pharmaceutical compositions of anti-PD-1 antibody prolgolimab that has improved aggregation stability, increased affinity over known anti-PD-1 antibodies that are are based on the human antibody of the IgG4 isotype.

As disclosed in international patent application WO/2018/013017 incorporated herein by reference, the anti-PD-1 antibody prolgolimab that is a monoclonal human antibody of the IgG1 isotype with non-effector mutations L234A, L235A, (referred herein as "antibody of the invention") has shown to have improved aggregation stability, increased affinity and improved pharmacokinetic parameters, such as t1/2β (hour) or Cmax (µg/ml) when compared to known anti-PD-1 antibodies that are based on the human antibody of the IgG4 isotype, such as nivolumab. Prolgolimab has a weight average molecular weight around 146 kDa and is specific for human PD-1. Prolgolimab has a heavy chain that contains 459 amino acids (SEQ ID NO: 1), and has a human light chain containing 214 amino acids (SEQ ID NO: 2), the constant portion (Fc) of prolgolimab comprises L234A, L235A mutations.

As such, it would be advantageous to administer an aqueous composition with the antibody of the invention to a patient having a malignant neoplasm.

A broad aspect of the present invention is an aqueous pharmaceutical composition suitable for administration to a subject for the inhibiting PD-1 protein activity. An aqueous pharmaceutical composition comprises a pharmaceutically effective amount of anti-PD-1 antibody prolgolimab, an effective amount of trehalose dihydrate, an acetate- or histidine-based buffering agent.

According to one broad aspect of the present invention, there is provided an aqueous pharmaceutical composition of anti-PD-1 antibody comprising:

(a) prolgolimab at a concentration from 15 mg/ml to 40 mg/ml as an antibody;
(b) trehalose dihydrate at a concentration from 80 mg/ml to 110 mg/ml;
(c) sodium acetate trihydrate at a concentration from 0.2 mg/ml to 2.5 mg/ml; and
(d) acetic acid to pH from 4.5 to 5.5.

In some embodiments of the invention, said prolgolimab may be present at a concentration from 15 mg/ml to 25 mg/ml.

In some embodiments of the invention, said prolgolimab may be present at a concentration of 20 mg/ml.

In some embodiments of the invention, said trehalose dihydrate may be present at a concentration from 95 mg/ml to 105 mg/ml.

In some embodiments of the invention, said trehalose dihydrate may be present at a concentration of 100 mg/ml.

In some embodiments of the invention, said sodium acetate trihydrate may be present at a concentration from 1.6 mg/ml to 1.9 mg/ml.

In some embodiments of the invention, said sodium acetate trihydrate may be present at a concentration from 1.7 mg/ml to 1.8 mg/ml.

In some embodiments of the invention, said sodium acetate trihydrate may be present at a concentration of 1.742 mg/ml.

In some embodiments of the invention, said acetic acid may be added to pH 5.0.

In some embodiments of the invention, said acetic acid may be present at a concentration from 0.04 mg/ml to 0.77 mg/ml.

In some embodiments of the invention, said acetic acid may be present at a concentration from 0.40 mg/ml to 0.50 mg/ml.

In some embodiments of the invention, said acetic acid may be present at a concentration of 0.43 mg/ml.

According to one broad aspect of the present invention, there is provided an aqueous pharmaceutical composition of anti-PD-1 antibody comprising:

(a) prolgolimab at a concentration from 90 mg/ml to 150 mg/ml as an antibody;
(b) trehalose dihydrate at a concentration from 50 mg/ml to 110 mg/ml;
(c) sodium acetate trihydrate at a concentration from 0.2 mg/ml to 2.5 mg/ml; and
(d) acetic acid to pH from 4.5 to 5.5.

In some embodiments of the invention, said prolgolimab may be present at a concentration from 90 mg/ml to 110 mg/ml.

In some embodiments of the invention, said prolgolimab may be present at a concentration of 100 mg/ml.

In some embodiments of the invention, said trehalose dihydrate may be present at a concentration from 75 mg/ml to 85 mg/ml.

In some embodiments of the invention, said trehalose dihydrate may be present at a concentration of 80 mg/ml.

In some embodiments of the invention, said sodium acetate trihydrate may be present at a concentration from 1.6 mg/ml to 1.9 mg/ml.

In some embodiments of the invention, said sodium acetate trihydrate may be present at a concentration from 1.7 mg/ml to 1.8 mg/ml.

In some embodiments of the invention, said sodium acetate trihydrate may be present at a concentration of 1.742 mg/ml.

In some embodiments of the invention, said acetic acid may be added to pH from 5.0 to 5.5.

In some embodiments of the invention, said acetic acid may be added to pH 5.0.

In some embodiments of the invention, said acetic acid may be present at a concentration from 0.045 mg/ml to 0.77 mg/ml.

In some embodiments of the invention, said acetic acid may be present at a concentration from 0.40 mg/ml to 0.50 mg/ml.

In some embodiments of the invention, said acetic acid may be present at a concentration from 0.43 mg/ml.

According to one broad aspect of the present invention, there is provided an aqueous pharmaceutical composition of anti-PD-1 antibody comprising:

(a) prolgolimab at a concentration from 5 mg/ml to 150 mg/ml as an antibody;
(b) trehalose dihydrate at a concentration from 70 mg/ml to 110 mg/ml;
(c) L-histidine at a concentration from 0.2 to 2.5 mg/ml; and
(d) L-histidine hydrochloride at a concentration from 0.2 to 3.5 mg/ml.

In some embodiments of the invention, said prolgolimab may be present at a concentration from 15 mg/ml to 40 mg/ml.

In some embodiments of the invention, said prolgolimab may be present at a concentration from 15 mg/ml to 25 mg/ml.

In some embodiments of the invention, said prolgolimab may be present at a concentration of 20 mg/ml.

In some embodiments of the invention, said trehalose dihydrate may be present at a concentration from 95 mg/ml to 105 mg/ml.

In some embodiments of the invention, said trehalose dihydrate may be present at a concentration of 100 mg/ml.

In some embodiments of the invention, said L-histidine may be present at a concentration from 0.7 mg/ml to 1.0 mg/ml.

In some embodiments of the invention, said L-histidine may be present at a concentration of 0.92 mg/ml.

In some embodiments of the invention, said L-histidine hydrochloride may be present at a concentration from 2.8 mg/ml to 3.3 mg/ml.

In some embodiments of the invention, said L-histidine hydrochloride may be present at a concentration of 2.96 mg/ml.

In some embodiments of the invention, said composition may have pH from 5.5 to 6.5.

In some embodiments of the invention, said composition may have pH 5.5.

In some embodiments of the invention, said prolgolimab may be present at a concentration from 90 mg/ml to 110 mg/ml.

In some embodiments of the invention, said prolgolimab may be present at a concentration of 100 mg/ml.

In some embodiments of the invention, said trehalose dihydrate may be present at a concentration from 75 mg/ml to 85 mg/ml.

In some embodiments of the invention, said trehalose dihydrate may be present at a concentration of 80 mg/ml.

In some embodiments of the invention, said L-histidine may be present at a concentration from 0.7 mg/ml to 1.0 mg/ml.

In some embodiments of the invention, said L-histidine may be present at a concentration of 0.92 mg/ml.

In some embodiments of the invention, said L-histidine hydrochloride may be present at a concentration from 2.8 mg/ml to 3.3 mg/ml.

In some embodiments of the invention, said L-histidine hydrochloride may be present at a concentration of 2.96 mg/ml.

In some embodiments of the invention, said composition may have pH from 5.5 to 6.5.

In some embodiments of the invention, said composition may have pH from 5.5 to 6.0.

In some embodiments of the invention, said composition may have pH 5.5.

An aqueous pharmaceutical composition of anti-PD-1 antibody prolgolimab of the invention may further comprise a suitable solubilizer.

In some embodiments of the invention, said solubilizer may be Poloxamer 188.

In some embodiments of the invention, said Poloxamer 188 may be present in an amount greater than 0 mg/ml but equal to or less than 1 mg/ml.

In some embodiments of the invention, said Poloxamer 188 may be present in an amount of 0 mg/ml, 0.1 mg/ml, 0.2 mg/ml, 0.3 mg/ml, 0.4 mg/ml, 0.5 mg/ml, 0.6 mg/ml, 0.7 mg/ml, 0.8 mg/ml, 0.9 mg/ml, 1.0 mg/ml.

In one embodiment, the present invention relates to an aqueous pharmaceutical composition of anti-PD-1 antibody comprising:

    (a) prolgolimab at a concentration of 20 mg/ml as an antibody;
    (b) trehalose dihydrate at a concentration of 100 mg/ml;
    (c) sodium acetate trihydrate at a concentration from 0.2 to 2.5 mg/ml; and
    (d) acetic acid to pH from 4.5 to 5.5.

In one embodiment, the present invention relates to an aqueous pharmaceutical composition of anti-PD-1 antibody comprising:

    (a) prolgolimab at a concentration of 20 mg/ml as an antibody;
    (b) trehalose dihydrate at a concentration of 100 mg/ml;
    (c) sodium acetate trihydrate at a concentration from 1.7 mg/ml to 1.8 mg/ml; and
    (d) acetic acid to pH 5.0.

In one embodiment, the present invention relates to an aqueous pharmaceutical composition of anti-PD-1 antibody comprising:

    (a) prolgolimab at a concentration of 20 mg/ml as an antibody;
    (b) trehalose dihydrate at a concentration of 100 mg/ml;
    (c) sodium acetate trihydrate at a concentration of 1.742 mg/ml; and
    (d) acetic acid to pH 5.0.

In one embodiment, the present invention relates to an aqueous pharmaceutical composition of anti-PD-1 antibody comprising:

    (a) prolgolimab at a concentration of 100 mg/ml as an antibody;
    (b) trehalose dihydrate at a concentration of 80 mg/ml;
    (c) sodium acetate trihydrate at a concentration from 0.2 to 2.5 mg/ml; and
    (d) acetic acid to pH from 4.5 to 5.5.

In one embodiment, the present invention relates to an aqueous pharmaceutical composition of anti-PD-1 antibody comprising:

    (a) prolgolimab at a concentration of 100 mg/ml as an antibody;
    (b) trehalose dihydrate at a concentration of 80 mg/ml;
    (c) sodium acetate trihydrate at a concentration from 1.7 mg/ml to 1.8 mg/ml; and
    (d) acetic acid to pH from 5.0 to 5.5.

In one embodiment, the present invention relates to an aqueous pharmaceutical composition of anti-PD-1 antibody comprising:

    (a) prolgolimab at a concentration of 100 mg/ml as an antibody;
    (b) trehalose dihydrate at a concentration of 80 mg/ml;
    (c) sodium acetate trihydrate at a concentration from 1.742 mg/ml; and
    (d) acetic acid to pH from 5.0 to 5.5.

In one embodiment, the present invention relates to an aqueous pharmaceutical composition of anti-PD-1 antibody comprising:

    (a) prolgolimab at a concentration of 100 mg/ml as an antibody;
    (b) trehalose dihydrate at a concentration of 80 mg/ml;
    (c) sodium acetate trihydrate at a concentration from 1.742 mg/ml; and

(d) acetic acid to pH 5.0.

In one embodiment, the present invention relates to an aqueous pharmaceutical composition of anti-PD-1 antibody comprising:

(a) prolgolimab at a concentration of 20 mg/ml as an antibody;
(b) trehalose dihydrate at a concentration of 100 mg/ml;
(c) L-histidine at a concentration from 0.2 to 2.5 mg/ml; and
(d) L-histidine hydrochloride at a concentration from 0.2 to 3.5 mg/ml;
(e) wherein said composition has pH from 5.5 to 6.5.

In one embodiment, the present invention relates to an aqueous pharmaceutical composition of anti-PD-1 antibody comprising:

(a) prolgolimab at a concentration of 20 mg/ml as an antibody;
(b) trehalose dihydrate at a concentration of 100 mg/ml;
(c) L-histidine at a concentration of 0.92 mg/ml; and
(d) L-histidine hydrochloride at a concentration of 2.96 mg/ml;
(e) wherein said composition has pH 5.5.

In one embodiment, the present invention relates to an aqueous pharmaceutical composition of anti-PD-1 antibody comprising:

(a) prolgolimab at a concentration of 100 mg/ml as an antibody;
(b) trehalose dihydrate at a concentration of 80 mg/ml;
(c) L-histidine at a concentration from 0.2 to 2.5 mg/ml; and
(d) L-histidine hydrochloride at a concentration from 0.2 to 3.5 mg/ml;
(e) wherein said composition has pH from 5.5 to 6.5.

In one embodiment, the present invention relates to an aqueous pharmaceutical composition of anti-PD-1 antibody comprising:

(a) prolgolimab at a concentration of 100 mg/ml as an antibody;
(b) trehalose dihydrate at a concentration of 80 mg/ml;
(c) L-histidine at a concentration of 0.92 mg/ml; and
(d) L-histidine hydrochloride at a concentration of 2.96 mg/ml;
(e) wherein said composition has pH from 5.5 to 6.0.

In one embodiment, the present invention relates to an aqueous pharmaceutical composition of anti-PD-1 antibody comprising:

(a) prolgolimab at a concentration of 100 mg/ml as an antibody;
(b) trehalose dihydrate at a concentration of 80 mg/ml;
(c) L-histidine at a concentration of 0.92 mg/ml; and
(d) L-histidine hydrochloride at a concentration of 2.96 mg/ml;
(e) wherein said composition has pH 5.5.

In one embodiment, the present invention relates to an aqueous pharmaceutical composition of anti-PD-1 antibody comprising:
I. Composition per 1 mL:

| | |
|---|---|
| Prolgolimab | 20.0 mg |
| Sodium acetate trihydrate | 1.742 mg |
| Trehalose dihydrate | 100 mg |
| Acetic acid glac. | to pH 5.0 |
| Water for injections | up to 1 ml. |

In one embodiment, the present invention relates to an aqueous pharmaceutical composition of anti-PD-1 antibody comprising:

II. Composition per 1 mL:

| | |
|---|---|
| Prolgolimab | 20.0 mg |
| Trehalose dihydrate | 100 mg |
| L-histidine | 0.92 mg |
| L-histidine hydrochloride | 2.96 mg |
| Water for injections | up to 1 ml. |

An aqueous pharmaceutical composition of anti-PD-1 antibody prolgolimab of the invention may further comprise a suitable solubilizer.

In some embodiments of the invention, said solubilizer may be Poloxamer 188.

In some embodiments of the invention, said Poloxamer 188 may be present in an amount greater than 0 mg/ml but equal to or less than 1 mg/ml.

In some embodiments of the invention, said Poloxamer 188 may be present in an amount of 0 mg/ml, 0.1 mg/ml, 0.2 mg/ml, 0.3 mg/ml, 0.4 mg/ml, 0.5 mg/ml, 0.6 mg/ml, 0.7 mg/ml, 0.8 mg/ml, 0.9 mg/ml, 1.0 mg/ml.

In one embodiment, the present invention relates to an aqueous pharmaceutical composition of anti-PD-1 antibody comprising:

(a) prolgolimab at a concentration of 20 mg/ml as an antibody;
(b) trehalose dihydrate at a concentration of 100 mg/ml;
(c) sodium acetate trihydrate at a concentration from 0.2 to 2.5 mg/ml; and
(d) acetic acid to pH from 4.5 to 5.5;
(e) Poloxamer 188 at a concentration of greater than 0 mg/ml, but equal to or less than 1 mg/ml.

In one embodiment, the present invention relates to an aqueous pharmaceutical composition of anti-PD-1 antibody comprising:

(a) prolgolimab at a concentration of 20 mg/ml as an antibody;
(b) trehalose dihydrate at a concentration of 100 mg/ml;
(c) sodium acetate trihydrate at a concentration from 1.7 mg/ml to 1.8 mg/ml; and
(d) acetic acid to pH 5.0.
(e) Poloxamer 188 at a concentration of greater than 0 mg/ml, but equal to or less than 1 mg/ml.

In one embodiment, the present invention relates to an aqueous pharmaceutical composition of anti-PD-1 antibody comprising:

(a) prolgolimab at a concentration of 20 mg/ml as an antibody;
(b) trehalose dihydrate at a concentration of 100 mg/ml;
(c) sodium acetate trihydrate at a concentration of 1.742 mg/ml; and
(d) acetic acid to pH 5.0.
(e) Poloxamer 188 at a concentration of greater than 0 mg/ml, but equal to or less than 1 mg/ml.

In one embodiment, the present invention relates to an aqueous pharmaceutical composition of anti-PD-1 antibody comprising:

(a) prolgolimab at a concentration of 100 mg/ml as an antibody;
(b) trehalose dihydrate at a concentration of 80 mg/ml;
(c) sodium acetate trihydrate at a concentration from 0.2 to 2.5 mg/ml; and
(d) acetic acid to pH from 4.5 to 5.5;
(e) Poloxamer 188 at a concentration of greater than 0 mg/ml, but equal to or less than 1 mg/ml.

In one embodiment, the present invention relates to an aqueous pharmaceutical composition of anti-PD-1 antibody comprising:

(a) prolgolimab at a concentration of 100 mg/ml as an antibody;

(b) trehalose dihydrate at a concentration of 80 mg/ml;

(c) sodium acetate trihydrate at a concentration from 1.7 mg/ml to 1.8 mg/ml; and

(d) acetic acid to pH from 5.0 to 5.5;

(e) Poloxamer 188 at a concentration of greater than 0 mg/ml, but equal to or less than 1 mg/ml.

In one embodiment, the present invention relates to an aqueous pharmaceutical composition of anti-PD-1 antibody comprising:

(a) prolgolimab at a concentration of 100 mg/ml as an antibody;

(b) trehalose dihydrate at a concentration of 80 mg/ml;

(c) sodium acetate trihydrate at a concentration of 1.742 mg/ml; and

(d) acetic acid to pH from 5.0 to 5.5;

(e) Poloxamer 188 at a concentration of greater than 0 mg/ml, but equal to or less than 1 mg/ml.

In one embodiment, the present invention relates to an aqueous pharmaceutical composition of anti-PD-1 antibody comprising:

(a) prolgolimab at a concentration of 100 mg/ml as an antibody;

(b) trehalose dihydrate at a concentration of 80 mg/ml;

(c) sodium acetate trihydrate at a concentration from 1.742 mg/ml; and

(d) acetic acid to pH 5.0.

(e) Poloxamer 188 at a concentration of greater than 0 mg/ml, but equal to or less than 1 mg/ml.

In one embodiment, the present invention relates to an aqueous pharmaceutical composition of anti-PD-1 antibody comprising:

(a) prolgolimab at a concentration of 20 mg/ml as an antibody;

(b) trehalose dihydrate at a concentration of 100 mg/ml;

(c) L-histidine at a concentration from 0.2 to 2.5 mg/ml; and

(d) L-histidine hydrochloride at a concentration from 0.2 to 3.5 mg/ml;

(e) wherein said composition has pH from 5.5 to 6.5;

(f) Poloxamer 188 at a concentration of greater than 0 mg/ml, but equal to or less than 1 mg/ml.

In one embodiment, the present invention relates to an aqueous pharmaceutical composition of anti-PD-1 antibody comprising:

(a) prolgolimab at a concentration of 20 mg/ml as an antibody;

(b) trehalose dihydrate at a concentration of 100 mg/ml;

(c) L-histidine at a concentration of 0.92 mg/ml; and

(d) L-histidine hydrochloride at a concentration of 2.96 mg/ml;

(e) wherein said composition has pH 5.5;

(f) Poloxamer 188 at a concentration of greater than 0 mg/ml, but equal to or less than 1 mg/ml.

In one embodiment, the present invention relates to an aqueous pharmaceutical composition of anti-PD-1 antibody comprising:

(a) prolgolimab at a concentration of 100 mg/ml as an antibody;

(b) trehalose dihydrate at a concentration of 80 mg/ml;

(c) L-histidine at a concentration from 0.2 to 2.5 mg/ml; and

(d) L-histidine hydrochloride at a concentration from 0.2 to 3.5 mg/ml;

(e) wherein said composition has pH from 5.5 to 6.5;

(f) Poloxamer 188 at a concentration of greater than 0 mg/ml, but equal to or less than 1 mg/ml.

In one embodiment, the present invention relates to an aqueous pharmaceutical composition of anti-PD-1 antibody comprising:

(a) prolgolimab at a concentration of 100 mg/ml as an antibody;

(b) trehalose dihydrate at a concentration of 80 mg/ml;

(c) L-histidine at a concentration of 0.92 mg/ml; and

(d) L-histidine hydrochloride at a concentration of 2.96 mg/ml;

(e) wherein said composition has pH from 5.5 to 6.0;

(f) Poloxamer 188 at a concentration of greater than 0 mg/ml, but equal to or less than 1 mg/ml.

In one embodiment, the present invention relates to an aqueous pharmaceutical composition of anti-PD-1 antibody comprising:

(a) prolgolimab at a concentration of 100 mg/ml as an antibody;

(b) trehalose dihydrate at a concentration of 80 mg/ml;

(c) L-histidine at a concentration of 0.92 mg/ml; and

(d) L-histidine hydrochloride at a concentration of 2.96 mg/ml;

(e) wherein said composition has pH 5.5;

(f) Poloxamer 188 at a concentration of greater than 0 mg/ml, but equal to or less than 1 mg/ml.

In some embodiments of the present invention, said Poloxamer 188 may be present in an amount of 0 mg/ml, 0.1 mg/ml, 0.2 mg/ml, 0.3 mg/ml, 0.4 mg/ml, 0.5 mg/ml, 0.6 mg/ml, 0.7 mg/ml, 0.8 mg/ml, 0.9 mg/ml, 1.0 mg/ml.

In some embodiments, an aqueous pharmaceutical composition of anti-PD-1 antibody of the invention may be administered parenterally.

In some embodiments, an aqueous pharmaceutical composition of anti-PD-1 antibody of the invention may be administered intramuscularly.

In some embodiments, an aqueous pharmaceutical composition of anti-PD-1 antibody of the invention may be administered subcutaneously.

In some embodiments, an aqueous pharmaceutical composition of anti-PD-1 antibody of the invention may be administered intravenously.

In some embodiments, an aqueous pharmaceutical composition of anti-PD-1 antibody of the invention may be administered intravenously as an infusion.

In some embodiments, an aqueous pharmaceutical composition of anti-PD-1 antibody of the invention may be administered intravenously as an infusion over 60 minutes; in case of good tolerability, the infusion time may be shortened to 30 minutes.

In one embodiment, an aqueous pharmaceutical composition of anti-PD-1 antibody prolgolimab of the invention may be present in a vial.

In some embodiments, said vial may be a glass vial.

In some embodiments, said vial may have a volume from 1 ml to 50 ml.

In some embodiments, said vial may have a volume from 1 ml to 20 ml.

In some embodiments, said vial may have a volume of 1 ml, 2 ml, 3 ml, 4 ml, 5 ml, 6 ml, 7 ml, 8 ml, 9 ml, 10 ml, 15 ml, 20 ml, 25 ml, 30 ml, 35 ml, 40 ml, 45 ml or 50 ml.

In one embodiment, an aqueous pharmaceutical composition of anti-PD-1 antibody prolgolimab of the present invention may be present in a syringe.

In some embodiments, said syringe may have a capacity of 1 ml.

In some embodiments, said syringe may have a capacity of 2 ml.

In one embodiment, an aqueous pharmaceutical composition of anti-PD-1 antibody prolgolimab of the invention may be present in a pre-filled syringe.

In some embodiments, said pre-filled syringe may have a capacity of 1 ml.

In some embodiments, said pre-filled syringe may have a capacity of 2 ml.

Another broad aspect of the present invention is a method of producing an aqueous pharmaceutical composition suitable for administration to a subject for the inhibiting PD-1 protein activity. The method includes combining a pharmaceutically effective amount of anti-PD-1 antibody prolgolimab with an acetate-based buffering agent; and an effective amount of trehalose. The method includes combining a pharmaceutically effective amount of anti-PD-1 antibody prolgolimab with a histidine-based buffering agent; and an effective amount of trehalose.

In some embodiments, Poloxamer 188 may be added as a solubilizer.

Another broad aspect of the present invention is the use of an aqueous pharmaceutical composition of anti-PD-1 antibody prolgolimab, as defined herein, for treating malignant neoplasms.

In some embodiments of the invention, the use of an aqueous pharmaceutical composition of anti-PD-1 antibody prolgolimab is for treating malignant neoplasms that may be selected from the group comprising melanoma, including inoperable or metastatic melanoma, early stages of melanoma before and after definitive treatment; lung cancer, non-small cell lung cancer (NSCLC), including inoperable or metastatic non-small cell lung cancer; non-squamous non-small cell lung cancer, squamous cell lung cancer; small cell lung cancer, including inoperable or metastatic small cell lung cancer; early stages of lung cancer before and after definitive treatment; cervical cancer, including metastatic cervical cancer, early stages of cervical cancer before and after definitive treatment; head and neck tumors, including head and neck squamous cell cancer; Hodgkin's lymphoma; stomach and bowel tumors, metastatic squamous cell esophageal cancer; bladder cancer, including metastatic urothelial carcinoma, kidney cancer; endometrial cancer, including metastatic endometrial cancer, early stages of endometrial cancer before and after definitive treatment; breast cancer, including metastatic breast cancer, early stages of endometrial cancer before and after definitive treatment; liver cancer, including metastatic or inoperable liver cancer, early stages of liver cancer before and after definitive treatment; inoperable or metastatic solid tumor, including inoperable or metastatic solid tumor with signs of microsatellite instability.

Another broad aspect of the present invention is the use of an aqueous pharmaceutical composition of anti-PD-1 antibody prolgolimab for treating a malignant neoplasm comprising administering a pharmaceutically effective amount of an aqueous pharmaceutical composition as defined herein.

Another broad aspect of the present invention is the use of an aqueous pharmaceutical composition of anti-PD-1 antibody comprising:

    (a) prolgolimab at a concentration from 15 mg/ml to 40 mg/ml as an antibody;
    (b) trehalose dihydrate at a concentration from 80 mg/ml to 110 mg/ml;
    (c) sodium acetate trihydrate at a concentration from 0.2 mg/ml to 2.5 mg/ml; and
    (d) acetic acid to pH 4.5-5.5,
    for treating a malignant neoplasm in a subject in need thereof.

In some embodiments of the invention, said prolgolimab may be present at a concentration from 15 mg/ml to 25 mg/ml.

In some embodiments of the invention, said prolgolimab may be present at a concentration of 20 mg/ml.

In some embodiments of the invention, said trehalose dihydrate may be present at a concentration from 95 mg/ml to 105 mg/ml.

In some embodiments of the invention, said trehalose dihydrate may be present at a concentration of 100 mg/ml.

In some embodiments of the invention, said sodium acetate trihydrate may be present at a concentration from 1.6 mg/ml to 1.9 mg/ml.

In some embodiments of the invention, said sodium acetate trihydrate may be present at a concentration from 1.7 mg/ml to 1.8 mg/ml.

In some embodiments of the invention, said sodium acetate trihydrate may be present at a concentration of 1.742 mg/ml.

In some embodiments of the invention, said acetic acid may be added to pH 5.0.

In some embodiments of the invention, said acetic acid may be present at a concentration from 0.04 mg/ml to 0.77 mg/ml.

In some embodiments of the invention, said acetic acid may be present at a concentration from 0.40 mg/ml to 0.50 mg/ml.

In some embodiments of the invention, said acetic acid may be present at a concentration of 0.43 mg/ml.

Another broad aspect of the present invention is the use of an aqueous pharmaceutical composition of anti-PD-1 antibody comprising:

    (a) prolgolimab at a concentration from 5 mg/ml to 40 mg/ml as an antibody;
    (b) trehalose dihydrate at a concentration from 70 mg/ml to 110 mg/ml;
    (c) L-histidine at a concentration from 0.2 to 2.5 mg/ml; and
    (d) L-histidine hydrochloride at a concentration from 0.2 to 3.5 mg/ml.
    for treating a malignant neoplasm in a subject in need thereof.

In some embodiments of the invention, said prolgolimab may be present at a concentration from 15 mg/ml to 25 mg/ml.

In some embodiments of the invention, said prolgolimab may be present at a concentration of 20 mg/ml.

In some embodiments of the invention, said trehalose dihydrate may be present at a concentration from 95 mg/ml to 105 mg/ml.

In some embodiments of the invention, said trehalose dihydrate may be present at a concentration of 100 mg/ml.

In some embodiments of the invention, said L-histidine may be present at a concentration from 0.7 mg/ml to 1.0 mg/ml.

In some embodiments of the invention, said L-histidine may be present at a concentration of 0.92 mg/ml.

In some embodiments of the invention, said L-histidine hydrochloride may be present at a concentration from 2.8 mg/ml to 3.3 mg/ml.

In some embodiments of the invention, said L-histidine hydrochloride may be present at a concentration of 2.96 mg/ml.

In some embodiments of the invention, said composition may have pH from 5.5 to 6.5.

In some embodiments of the invention, said composition may have pH 5.5.

In one embodiment, there is provided the use of an aqueous pharmaceutical composition of anti-PD-1 antibody comprising:

(a) prolgolimab at a concentration of 20 mg/ml as an antibody;
(b) trehalose dihydrate at a concentration of 100 mg/ml;
(c) sodium acetate trihydrate at a concentration from 0.2 to 2.5 mg/ml; and
(d) acetic acid to pH from 4.5 to 5.5;
for treating a malignant neoplasm in a subject in need thereof.

In one embodiment, there is provided the use of an aqueous pharmaceutical composition of anti-PD-1 antibody comprising:

(a) prolgolimab at a concentration of 20 mg/ml as an antibody;
(b) trehalose dihydrate at a concentration of 100 mg/ml;
(c) sodium acetate trihydrate at a concentration from 1.7 mg/ml to 1.8 mg/ml; and
(d) acetic acid to pH 5.0.
for treating a malignant neoplasm in a subject in need thereof.

In one embodiment, there is provided the use of an aqueous pharmaceutical composition of anti-PD-1 antibody comprising:

(a) prolgolimab at a concentration of 20 mg/ml as an antibody;
(b) trehalose dihydrate at a concentration of 100 mg/ml;
(c) sodium acetate trihydrate at a concentration of 1.742 mg/ml; and
(d) acetic acid to pH 5.0.
for treating a malignant neoplasm in a subject in need thereof.

In one embodiment, there is provided the use of an aqueous pharmaceutical composition of anti-PD-1 antibody comprising:

(a) prolgolimab at a concentration of 20 mg/ml as an antibody;
(b) trehalose dihydrate at a concentration of 100 mg/ml;
(c) L-histidine at a concentration from 0.2 to 2.5 mg/ml; and
(d) L-histidine hydrochloride at a concentration from 0.2 to 3.5 mg/ml;
(e) wherein said composition has pH from 5.5 to 6.5;
for treating a malignant neoplasm in a subject in need thereof.

In one embodiment, there is provided the use of an aqueous pharmaceutical composition of anti-PD-1 antibody comprising:

(a) prolgolimab at a concentration of 20 mg/ml as an antibody;
(b) trehalose dihydrate at a concentration of 100 mg/ml;
(c) L-histidine at a concentration of 0.92 mg/ml; and
(d) L-histidine hydrochloride at a concentration of 2.96 mg/ml;
(e) wherein said composition has pH 5.5;
for treating a malignant neoplasm in a subject in need thereof.

In one embodiment, there is provided the use of an aqueous pharmaceutical composition of anti-PD-1 antibody comprising:

I. Composition per 1 mL:

| | |
|---|---|
| Prolgolimab | 20.0 mg |
| Sodium acetate trihydrate | 1.742 mg |

(continued)

| | |
|---|---|
| Trehalose dihydrate | 100 mg |
| Acetic acid glac. | to pH 5.0 |
| Water for injections | up to 1 ml; |

for treating a malignant neoplasm in a subject in need thereof.

In one embodiment, there is provided the use of an aqueous pharmaceutical composition of anti-PD-1 antibody comprising:

II. Composition per 1 mL:

| | |
|---|---|
| Prolgolimab | 20.0 mg |
| Trehalose dihydrate | 100 mg |
| L-histidine | 0.92 mg |
| L-histidine hydrochloride | 2.96 mg |
| Water for injections | up to 1 ml; |

for treating a malignant neoplasm in a subject in need thereof.

An aqueous pharmaceutical composition of anti-PD-1 antibody prolgolimab of the invention may further comprise a suitable solubilizer.

In some embodiments of the invention, said solubilizer may be Poloxamer 188.

In some embodiments of the invention, said Poloxamer 188 may be present in an amount greater than 0 mg/ml but equal to or less than 1 mg/ml.

In some embodiments of the invention, said Poloxamer 188 may be present in an amount of 0 mg/ml, 0.1 mg/ml, 0.2 mg/ml, 0.3 mg/ml, 0.4 mg/ml, 0.5 mg/ml, 0.6 mg/ml, 0.7 mg/ml, 0.8 mg/ml, 0.9 mg/ml, 1.0 mg/ml.

In some embodiments of the invention, the use of said aqueous pharmaceutical composition of anti-PD-1 antibody prolgolimab may comprise administering said composition at a dose of anti-PD-1 antibody prolgolimab of 1 mg/kg of body weight.

In some embodiments of the invention, the use of said aqueous pharmaceutical composition of anti-PD-1 antibody prolgolimab may comprise administering said composition at a dose of anti-PD-1 antibody prolgolimab of 3 mg/kg of body weight.

In some embodiments of the invention, the use of said aqueous pharmaceutical composition of anti-PD-1 antibody prolgolimab may comprise administering said composition every 2 weeks.

In some embodiments of the invention, the use of said aqueous pharmaceutical composition of anti-PD-1 antibody prolgolimab may comprise administering said composition once in 2 weeks.

In some embodiments of the invention, the use of said aqueous pharmaceutical composition of anti-PD-1 antibody prolgolimab may comprise administering said composition every 3 weeks.

In some embodiments of the invention, the use of said aqueous pharmaceutical composition of anti-PD-1 antibody prolgolimab may comprise administering said composition once in 3 weeks.

In some embodiments of the invention, the use of said aqueous pharmaceutical composition of anti-PD-1 antibody prolgolimab may comprise administering said composition at a dose of anti-PD-1 antibody prolgolimab of 1 mg/kg of body weight every 2 weeks.

In some embodiments of the invention, the use of said aqueous pharmaceutical composition of anti-PD-1 antibody prolgolimab may comprise administering said composition at a dose of anti-PD-1 antibody prolgolimab of 1 mg/kg of body weight once in 2 weeks.

In some embodiments of the invention, the use of said aqueous pharmaceutical composition of anti-PD-1 antibody prolgolimab may comprise administering said composition at a dose of anti-PD-1 antibody prolgolimab of 3 mg/kg of body weight every 3 weeks.

In some embodiments of the invention, the use of said aqueous pharmaceutical composition of anti-PD-1 antibody prolgolimab may comprise administering said composition at a dose of anti-PD-1 antibody prolgolimab of 3 mg/kg of body weight once in 3 weeks.

In some embodiments of the invention, the use of said aqueous pharmaceutical composition of anti-PD-1 antibody prolgolimab may comprise administering said composition parenterally.

In some embodiments of the invention, said parenteral administration may be intravenous, subcutaneous or intramuscular administration.

In some embodiments of the invention, the use of said aqueous pharmaceutical composition of anti-PD-1 antibody prolgolimab may comprise administering said composition intravenously as an infusion.

In some embodiments, an aqueous pharmaceutical composition of anti-PD-1 antibody prolgolimab of the invention may be administered intravenously as an infusion over 60 minutes; in case of good tolerability, the infusion time may be shortened to 30 minutes.

In some embodiments of the invention, a malignant neoplasm is melanoma, including inoperable or metastatic melanoma, early stages of melanoma before and after definitive treatment; lung cancer, non-small cell lung cancer (NSCLC), including inoperable or metastatic non-small cell lung cancer.

In one embodiment, there is provided a method for treating malignant neoplasms in a subject in need thereof comprising administering a therapeutically effective amount of an aqueous pharmaceutical composition of anti-PD-1 antibody comprising:

(a) prolgolimab at a concentration from 15 mg/ml to 40 mg/ml as an antibody;
(b) trehalose dihydrate at a concentration from 80 mg/ml to 110 mg/ml;
(c) sodium acetate trihydrate at a concentration from 0.2 mg/ml to 2.5 mg/ml; and
(d) acetic acid to pH 4.5-5.5.

In some embodiments of the invention, said prolgolimab may be present at a concentration from 15 mg/ml to 25 mg/ml.

In some embodiments of the invention, said prolgolimab may be present at a concentration of 20 mg/ml.

In some embodiments of the invention, said trehalose dihydrate may be present at a concentration from 95 mg/ml to 105 mg/ml.

In some embodiments of the invention, said trehalose dihydrate may be present at a concentration of 100 mg/ml.

In some embodiments of the invention, said sodium acetate trihydrate may be present at a concentration from 1.6 mg/ml to 1.9 mg/ml.

In some embodiments of the invention, said sodium acetate trihydrate may be present at a concentration from 1.7 mg/ml to 1.8 mg/ml.

In some embodiments of the invention, said sodium acetate trihydrate may be present at a concentration of 1.742 mg/ml.

In some embodiments of the invention, said acetic acid may be added to pH 5.0.

In some embodiments of the invention, said acetic acid may be present at a concentration from 0.04 mg/ml to 0.77 mg/ml.

In some embodiments of the invention, said acetic acid may be present at a concentration from 0.40 mg/ml to 0.50 mg/ml.

In some embodiments of the invention, said acetic acid may be present at a concentration of 0.43 mg/ml.

In one embodiment, there is provided a method for treating malignant neoplasms in a subject in need thereof comprising administering a therapeutically effective amount of an aqueous pharmaceutical composition of anti-PD-1 antibody comprising:

(a) prolgolimab at a concentration from 5 mg/ml to 40 mg/ml as an antibody;
(b) trehalose dihydrate at a concentration from 70 mg/ml to 110 mg/ml;
(c) L-histidine at a concentration from 0.2 to 2.5 mg/ml; and
(d) L-histidine hydrochloride at a concentration from 0.2 to 3.5 mg/ml.

In some embodiments of the invention, said prolgolimab may be present at a concentration from 15 mg/ml to 25 mg/ml.

In some embodiments of the invention, said prolgolimab may be present at a concentration of 20 mg/ml.

In some embodiments of the invention, said trehalose dihydrate may be present at a concentration from 95 mg/ml to 105 mg/ml.

In some embodiments of the invention, said trehalose dihydrate may be present at a concentration of 100 mg/ml.

In some embodiments of the invention, said L-histidine may be present at a concentration from 0.7 mg/ml to 1.0 mg/ml.

In some embodiments of the invention, said L-histidine may be present at a concentration of 0.92 mg/ml.

In some embodiments of the invention, said L-histidine hydrochloride may be present at a concentration from 2.8 mg/ml to 3.3 mg/ml.

In some embodiments of the invention, said L-histidine hydrochloride may be present at a concentration of 2.96 mg/ml.

In some embodiments of the invention, said composition may have pH from 5.5 to 6.5.

In some embodiments of the invention, said composition may have pH 5.5.

In one embodiment, there is provided a method for treating malignant neoplasms in a subject in need thereof comprising administering a therapeutically effective amount of an aqueous pharmaceutical composition of anti-PD-1 antibody comprising:

(a) prolgolimab at a concentration of 20 mg/ml as an antibody;
(b) trehalose dihydrate at a concentration of 100 mg/ml;
(c) sodium acetate trihydrate at a concentration from 0.2 to 2.5 mg/ml; and
(d) acetic acid to pH from 4.5 to 5.5.

In one embodiment, there is provided a method for treating malignant neoplasms in a subject in need thereof comprising administering a therapeutically effective amount of an aqueous pharmaceutical composition of anti-PD-1 antibody comprising:

(a) prolgolimab at a concentration of 20 mg/ml as an antibody;
(b) trehalose dihydrate at a concentration of 100 mg/ml;
(c) sodium acetate trihydrate at a concentration from 1.7 mg/ml to 1.8 mg/ml; and
(d) acetic acid to pH 5.0.

In one embodiment, there is provided a method for treating malignant neoplasms in a subject in need thereof comprising administering a therapeutically effective amount of an aqueous pharmaceutical composition of anti-PD-1 antibody comprising:

(a) prolgolimab at a concentration of 20 mg/ml as an antibody;
(b) trehalose dihydrate at a concentration of 100 mg/ml;
(c) sodium acetate trihydrate at a concentration of 1.742 mg/ml; and
(d) acetic acid to pH 5.0.

In one embodiment, there is provided a method for treating malignant neoplasms in a subject in need thereof comprising administering a therapeutically effective amount of an aqueous pharmaceutical composition of anti-PD-1 antibody comprising:

(a) prolgolimab at a concentration of 20 mg/ml as an antibody;
(b) trehalose dihydrate at a concentration of 100 mg/ml;
(c) L-histidine at a concentration from 0.2 to 2.5 mg/ml; and
(d) L-histidine hydrochloride at a concentration from 0.2 to 3.5 mg/ml;
(e) wherein said composition has pH from 5.5 to 6.5.

In one embodiment, there is provided a method for treating malignant neoplasms in a subject in need thereof comprising administering a therapeutically effective amount of an aqueous pharmaceutical composition of anti-PD-1 antibody comprising:

(a) prolgolimab at a concentration of 20 mg/ml as an antibody;
(b) trehalose dihydrate at a concentration of 100 mg/ml;
(c) L-histidine at a concentration of 0.92 mg/ml; and
(d) L-histidine hydrochloride at a concentration of 2.96 mg/ml;
(e) wherein said composition has pH 5.5.

In one embodiment, there is provided a method for treating malignant neoplasms in a subject in need thereof comprising administering a therapeutically effective amount of an aqueous pharmaceutical composition of anti-PD-1 antibody comprising:

I. Composition per 1 mL:

| | |
|---|---|
| Prolgolimab | 20.0 mg |
| Sodium acetate trihydrate | 1.742 mg |
| Trehalose dihydrate | 100 mg |
| Acetic acid glac. | to pH 5.0 |
| Water for injections | up to 1 ml. |

In one embodiment, there is provided a method for treating malignant neoplasms in a subject in need thereof comprising administering a therapeutically effective amount of an aqueous pharmaceutical composition of anti-PD-1 antibody comprising:

II. Composition per 1 mL:

| | |
|---|---|
| Prolgolimab | 20.0 mg |
| Trehalose dihydrate | 100 mg |
| L-histidine | 0.92 mg |
| L-histidine hydrochloride | 2.96 mg |
| Water for injections | up to 1 ml. |

An aqueous pharmaceutical composition of anti-PD-1 antibody prolgolimab of the invention may further comprise a suitable solubilizer.

In some embodiments of the invention, said solubilizer may be Poloxamer 188.

In some embodiments of the invention, said Poloxamer 188 may be present in an amount greater than 0 mg/ml but equal to or less than 1 mg/ml.

In some embodiments of the invention, said Poloxamer 188 may be present in an amount of 0 mg/ml, 0.1 mg/ml, 0.2 mg/ml, 0.3 mg/ml, 0.4 mg/ml, 0.5 mg/ml, 0.6 mg/ml, 0.7 mg/ml, 0.8 mg/ml, 0.9 mg/ml, 1.0 mg/ml.

In some embodiments of the invention, a method for treating malignant neoplasms in a subject in need thereof may comprise administering a therapeutically effective amount of said aqueous pharmaceutical composition of anti-PD-1 antibody prolgolimab at a dose of anti-PD-1 antibody prolgolimab of 1 mg/kg of body weight.

In some embodiments of the invention, a method for treating malignant neoplasms in a subject in need thereof may comprise administering a therapeutically effective amount of said aqueous pharmaceutical composition of anti-PD-1 antibody prolgolimab at a dose of anti-PD-1 antibody prolgolimab of 3 mg/kg of body weight.

In some embodiments of the invention, a method for treating malignant neoplasms in a subject in need thereof may comprise administering a therapeutically effective amount of said aqueous pharmaceutical composition of anti-PD-1 antibody prolgolimab every 2 weeks.

In some embodiments of the invention, a method for treating malignant neoplasms in a subject in need thereof may comprise administering a therapeutically effective amount of said aqueous pharmaceutical composition of anti-PD-1 antibody prolgolimab once in 2 weeks.

In some embodiments of the invention, a method for treating malignant neoplasms in a subject in need thereof may comprise administering a therapeutically effective amount of said aqueous pharmaceutical composition of anti-PD-1 antibody prolgolimab every 3 weeks.

In some embodiments of the invention, a method for treating malignant neoplasms in a subject in need thereof may comprise administering a therapeutically effective amount of said aqueous pharmaceutical composition of anti-PD-1 antibody prolgolimab once in 3 weeks.

In some embodiments of the invention, a method for treating malignant neoplasms in a subject in need thereof may comprise administering a therapeutically effective amount of said aqueous pharmaceutical composition of anti-PD-1 antibody prolgolimab at a dose of anti-PD-1 antibody prolgolimab of 1 mg/kg of body weight every 2 weeks.

In some embodiments of the invention, a method for treating malignant neoplasms in a subject in need thereof may comprise administering a therapeutically effective amount of said aqueous pharmaceutical composition of anti-PD-1 antibody prolgolimab at a dose of anti-PD-1 antibody prolgolimab of 1 mg/kg of body weight once in 2 weeks.

In some embodiments of the invention, a method for treating malignant neoplasms in a subject in need thereof may comprise administering a therapeutically effective amount of said aqueous pharmaceutical composition of anti-PD-1 antibody prolgolimab at a dose of anti-PD-1 antibody prolgolimab of 3 mg/kg of body weight every 3 weeks.

In some embodiments of the invention, a method for treating malignant neoplasms in a subject in need thereof may comprise administering a therapeutically effective amount of said aqueous pharmaceutical composition of anti-PD-1 antibody prolgolimab at a dose of anti-PD-1 antibody prolgolimab of 3 mg/kg of body weight once in 3 weeks.

In some embodiments of the invention, a method for treating malignant neoplasms in a subject in need thereof may comprise administering a therapeutically effective amount of said aqueous pharmaceutical composition of anti-PD-1 antibody prolgolimab parenterally.

In some embodiments of the invention, said parenteral administration may be intravenous, subcutaneous or intramuscular administration.

In some embodiments of the invention, a method for treating malignant neoplasms in a subject in need thereof may comprise administering a therapeutically effective amount of said aqueous pharmaceutical composition of anti-PD-1 antibody prolgolimab intravenously as an infusion.

In some embodiments, an aqueous pharmaceutical composition of anti-PD-1 antibody prolgolimab of the invention may be administered intravenously as an infusion over 60 minutes; in case of good tolerability, the infusion time may be shortened to 30 minutes.

In some embodiments of the invention, a malignant neoplasm is melanoma, including inoperable or metastatic melanoma, early stages of melanoma before and after definitive treatment; lung cancer, non-small cell lung cancer (NSCLC), including inoperable or metastatic non-small cell lung cancer.

In one embodiment, there is provided a method for treating malignant neoplasms in a subject in need thereof comprising administering a therapeutically effective amount of prolgolimab.

In some embodiments of the invention, a method for treating malignant neoplasms in a subject in need thereof may comprise administering a therapeutically effective amount of prolgolimab at a dose of 1 mg/kg.

In some embodiments of the invention, a method for treating malignant neoplasms in a subject in need thereof may comprise administering a therapeutically effective amount of prolgolimab at a dose of 3 mg/kg.

In some embodiments of the invention, a method for treating malignant neoplasms in a subject in need thereof may comprise administering a therapeutically effective amount of prolgolimab every 2 weeks.

In some embodiments of the invention, a method for treating malignant neoplasms in a subject in need thereof may comprise administering a therapeutically effective amount of prolgolimab once in 2 weeks.

In some embodiments of the invention, a method for treating malignant neoplasms in a subject in need thereof may comprise administering a therapeutically effective amount of prolgolimab every 3 weeks.

In some embodiments of the invention, a method for treating malignant neoplasms in a subject in need thereof may comprise administering a therapeutically effective amount of prolgolimab once in 3 weeks.

In some embodiments of the invention, a method for treating malignant neoplasms in a subject in need thereof may comprise administering a therapeutically effective amount of prolgolimab at a dose of 1 mg/kg every 2 weeks.

In some embodiments of the invention, a method for treating malignant neoplasms in a subject in need thereof may comprise administering a therapeutically effective amount of prolgolimab at a dose of 1 mg/kg once in 2 weeks.

In some embodiments of the invention, a method for treating malignant neoplasms in a subject in need thereof may comprise administering a therapeutically effective amount of prolgolimab at a dose of 3 mg/kg every 3 weeks.

In some embodiments of the invention, a method for treating malignant neoplasms in a subject in need thereof may comprise administering a therapeutically effective amount of prolgolimab at a dose of 3 mg/kg once in 3 weeks.

In some embodiments of the invention, a method for treating malignant neoplasms in a subject in need thereof may comprise administering a therapeutically effective amount of prolgolimab parenterally.

In some embodiments of the invention, said parenteral administration may be intravenous, subcutaneous or intramuscular administration.

In some embodiments of the invention, a method for treating malignant neoplasms in a subject in need thereof may comprise administering a therapeutically effective amount of prolgolimab intravenously as an infusion.

In some embodiments, prolgolimab may be administered intravenously as an infusion over 60 minutes; in case of good tolerability, the infusion time may be shortened to 30 minutes.

In some embodiments of the invention, a malignant neoplasm is melanoma, including inoperable or metastatic melanoma, early stages of melanoma before and after definitive treatment; lung cancer, non-small cell lung cancer (NSCLC), including inoperable or metastatic non-small cell lung cancer.

In one embodiment of the invention, there is provided a pharmaceutical composition suitable for administration to a subject for the inhibiting PD-1 protein activity, wherein, for each 1 mL of the pharmaceutical composition, the pharmaceutical composition comprises:

| | |
|---|---|
| Prolgolimab | 20.0 mg |
| Sodium acetate trihydrate | 1.742 mg |
| Trehalose dihydrate | 100 mg |
| Acetic acid glac. | to pH 5.0 |
| Water for injections | up to 1 ml. |

In one embodiment of the invention, there is provided a pharmaceutical composition suitable for administration to a subject for the inhibiting PD-1 protein activity, wherein, for each 1 mL of the pharmaceutical composition, the pharmaceutical composition comprises:

| | |
|---|---|
| Prolgolimab | 20.0 mg |
| Trehalose dihydrate | 100 mg |
| L-histidine | 0.92 mg |
| L-histidine hydrochloride | 2.96 mg |

(continued)

| Water for injections | up to 1 ml. |
|---|---|

In some embodiments of the invention, said aqueous pharmaceutical composition suitable for administration to a subject for the inhibiting PD-1 protein activity may be administered at a dose of anti-PD-1 antibody prolgolimab of 1 mg/kg of body weight.

In some embodiments of the invention, said aqueous pharmaceutical composition suitable for administration to a subject for the inhibiting PD-1 protein activity may be administered at a dose of anti-PD-1 antibody prolgolimab of 3 mg/kg of body weight.

In some embodiments of the invention, said aqueous pharmaceutical composition suitable for administration to a subject for the inhibiting PD-1 protein activity may be administered every 2 weeks.

In some embodiments of the invention, said aqueous pharmaceutical composition suitable for administration to a subject for the inhibiting PD-1 protein activity may be administered once in 2 weeks.

In some embodiments of the invention, said aqueous pharmaceutical composition suitable for administration to a subject for the inhibiting PD-1 protein activity may be administered once in 3 weeks.

In some embodiments of the invention, said aqueous pharmaceutical composition suitable for administration to a subject for the inhibiting PD-1 protein activity may be administered at a dose of anti-PD-1 antibody prolgolimab of 1 mg/kg of body weight every 2 weeks.

In some embodiments of the invention, said aqueous pharmaceutical composition suitable for administration to a subject for the inhibiting PD-1 protein activity may be administered at a dose of anti-PD-1 antibody prolgolimab of 1 mg/kg of body weight once in 2 weeks.

In some embodiments of the invention, said aqueous pharmaceutical composition suitable for administration to a subject for the inhibiting PD-1 protein activity may be administered at a dose of anti-PD-1 antibody prolgolimab of 3 mg/kg of body weight every 3 weeks.

In some embodiments of the invention, said aqueous pharmaceutical composition suitable for administration to a subject for the inhibiting PD-1 protein activity may be administered at a dose of anti-PD-1 antibody prolgolimab of 3 mg/kg of body weight once in 3 weeks.

In some embodiments of the invention, a method for treating malignant neoplasms in a subject in need thereof may comprise administering a therapeutically effective amount of prolgolimab parenterally.

In some embodiments of the invention, said parenteral administration may be intravenous, subcutaneous or intramuscular administration.

In some embodiments of the invention, a method for treating malignant neoplasms in a subject in need thereof may comprise administering a therapeutically effective amount of prolgolimab intravenously as an infusion.

In some embodiments, prolgolimab may be administered intravenously as an infusion over 60 minutes; in case of good tolerability, the infusion time may be shortened to 30 minutes.

In some embodiments of the invention, a malignant neoplasm is melanoma, including inoperable or metastatic melanoma, early stages of melanoma before and after definitive treatment; lung cancer, non-small cell lung cancer (NSCLC), including inoperable or metastatic non-small cell lung cancer.

EXEMPLARY EMBODIMENTS:

The present invention relates to suitable aqueous pharmaceutical compositions of anti-PD-1 antibody prolgolimab. In one embodiment of the invention, an aqueous pharmaceutical composition of prolgolimab may comprise an acetate-based buffer and trehalose. Poloxamer 188 may be added as a solubilizer. In another one embodiment of the invention, an aqueous pharmaceutical composition of prolgolimab may comprise a histidine-based buffer and trehalose. Poloxamer 188 may be added as a solubilizer.

The histidine-based buffer may be the result of combining L-histidine with histidine hydrochloride or, additionally, with hydrochloric acid, or other acids. It will be understood that even though histidine hydrochloride may be used as a salt for the histidine-based buffer, any other histidine-based salt may be used for the histidine-based buffer without departing from the present teachings.

The acetate-based buffer may be the result of combining acetic acid with sodium acetate trihydrate. It will be understood that even though sodium acetate trihydrate may be used as a salt for the acetate-based buffer, any other acetate salt, such as potassium acetate, may be used for the acetate-based buffer without departing from the present teachings.

Moreover, the composition of the present invention may further include one or more other suitable excipients that are well known to those skilled in the art.

In some embodiments, the implementation of the liquid pharmaceutical composition is stable during storage in the sense that no further processes of protein aggregation or its modifications occur in comparison with the indicator of stability at zero time point.

In one embodiment, the inventors have surprisingly obtained highly concentrated aqueous pharmaceutical compositions of anti-PD-1 antibody prolgolimab, wherein prolgolimab may be present at a concentration from 90 mg/ml to 150 mg/ml. In some embodiments of the present invention, said prolgolimab may be present at a concentration of 90 mg/ml, 95 mg/ml, 100 mg/ml, 105 mg/ml, 110 mg/ml, 115 mg/ml, 120 mg/ml, 125 mg/ml, 130 mg/ml, 135 mg/ml, 140 mg/ml, 145 mg/ml, 150 mg/ml.

Such highly concentrated aqueous pharmaceutical compositions of anti-PD-1 antibody prolgolimab of the present invention have exhibited colloidal stability while stirring vigorously (800 rpm) for 120 hours and high thermal stability while heating at 50°C and 37°C, as well as a viscosity of less than 50 cP that is suitable for parenteral administration.

The above compositions are suitable for parenteral administration, such as intravenous, subcutaneous, intradermal, intra-arterial, intrathecal, intraperitoneal, intra-articular and/or intramuscular administration.

Provided pharmaceutical compositions can be administered to an individual in need of treatment through a systemic injection, for example, by intravenous or subcutaneous or intramuscular injection; or by injection or application to an appropriate site, for example, by direct injection or direct application to the site, when the site is available for surgery; or through topical use.

The above compositions may be administered to a subject in need thereof intravenously as an infusion.

In some embodiments, an aqueous pharmaceutical composition of anti-PD-1 antibody prolgolimab of the invention may be administered intravenously as an infusion over 60 minutes; in case of good tolerability, the infusion time may be shortened to 30 minutes.

An aqueous pharmaceutical composition of anti-PD-1 antibody prolgolimab may be used after dilution. To this end, the required volume of the composition is transferred from a vial to an infusion container comprising a sterile 0.9% sodium chloride solution or a sterile 5% dextrose solution. The concentration of said composition in the resulting solution may range from 0.5 mg/ml to 10 mg/ml. The resulting solution is stirred by gently turning the infusion container over to avoid foaming.

Methods of treatment, and use of aqueous composition

In another embodiment, the present invention relates to a method for treating a mammal, comprising administering to the mammal a therapeutically effective amount of the pharmaceutical compositions of the present invention, wherein the concentration of anti-PDI antibody prolgolimab is from 15 mg/ml to 40 mg/ml, wherein the mammal may have a disease or disorder that can be effectively treated with the anti-PD-1 antibody prolgolimab of the invention.

In another embodiment, the present invention relates to a method for treating a mammal, comprising administering to the mammal a therapeutically effective amount of the pharmaceutical compositions of the present invention, wherein the concentration of anti-PD-1 antibody prolgolimab is 20 mg/ml, wherein the mammal may have a disease or disorder that can be effectively treated with the anti-PD-1 antibody prolgolimab of the invention.

In a preferred embodiment, the mammal is a human.

In another embodiment, the present invention relates to a method for treating a subject in need thereof, comprising administering to the subject a therapeutically effective amount of the pharmaceutical compositions of the present invention, wherein the concentration of anti-PD-1 antibody prolgolimab is from 15 mg/ml to 40 mg/ml, wherein the subject may have a disease or disorder that can be effectively treated with the anti-PD-1 antibody prolgolimab of the invention.

In another embodiment, the present invention relates to a method for treating a subject in need thereof, comprising administering to the subject a therapeutically effective amount of the pharmaceutical compositions of the present invention, wherein the concentration of anti-PD-1 antibody prolgolimab is 20 mg/ml, wherein the subject may have a disease or disorder that can be effectively treated with the anti-PD-1 antibody prolgolimab of the invention.

In a preferred embodiment, the subject is a human.

In one of the embodiments, the invention relates to a method for treating melanoma, comprising administering to a subject in need thereof a therapeutically effective amount of one of the provided compositions, wherein the concentration of anti-PD-1 antibody prolgolimab is from 15 mg/ml to 40 mg/ml, of the present invention.

In one of the embodiments, the invention relates to a method for treating melanoma, comprising administering to a subject in need thereof a therapeutically effective amount of one of the provided compositions, wherein the concentration of anti-PD-1 antibody prolgolimab is 20 mg/ml, of the present invention.

In one of the embodiments, the invention relates to a method for treating inoperable melanoma, comprising administering to a subject in need thereof a therapeutically effective amount of one of the provided compositions of anti-PD-1 antibody prolgolimab, wherein the concentration of anti-PD-1 antibody prolgolimab is from 15 mg/ml to 40 mg/ml, of the present invention.

In one of the embodiments, the invention relates to a method for treating inoperable melanoma, comprising administering to a subject in need thereof a therapeutically effective amount of one of the provided compositions of anti-PD-1 antibody prolgolimab, wherein the concentration of anti-PD-1 antibody prolgolimab is 20 mg/ml, of the present invention.

In one of the embodiments, the invention relates to a method for treating metastatic melanoma, comprising administering to a subject in need thereof a therapeutically effective amount of one of the provided compositions of anti-PD-1 antibody prolgolimab, wherein the concentration of anti-PD-1 antibody prolgolimab is from 15 mg/ml to 40 mg/ml, of the present invention.

In one of the embodiments, the invention relates to a method for treating metastatic melanoma, comprising administering to a subject in need thereof a therapeutically effective amount of one of the provided compositions of anti-PD-1 antibody prolgolimab, wherein the concentration of anti-PD-1 antibody prolgolimab is 20 mg/ml, of the present invention.

In one of the embodiments, the invention relates to a method for treating early stages of melanoma before and after definitive treatment, comprising administering to a subject in need thereof a therapeutically effective amount of one of the provided compositions of anti-PD-1 antibody prolgolimab, wherein the concentration of anti-PD-1 antibody prolgolimab is from 15 mg/ml to 40 mg/ml, of the present invention.

In one of the embodiments, the invention relates to a method for treating early stages of melanoma before and after definitive treatment, comprising administering to a subject in need thereof a therapeutically effective amount of one of the provided compositions of anti-PD-1 antibody prolgolimab, wherein the concentration of anti-PD-1 antibody prolgolimab is 20 mg/ml, of the present invention.

In one of the embodiments, the invention relates to a method for treating lung cancer, comprising administering to a subject in need thereof a therapeutically effective amount of one of the provided compositions of anti-PD-1 antibody prolgolimab, wherein the concentration of anti-PD-1 antibody prolgolimab is from 15 mg/ml to 40 mg/ml, of the present invention.

In one of the embodiments, the invention relates to a method for treating lung cancer, comprising administering to a subject in need thereof a therapeutically effective amount of one of the provided compositions of anti-PD-1 antibody prolgolimab, wherein the concentration of anti-PD-1 antibody prolgolimab is 20 mg/ml, of the present invention.

In one of the embodiments, the invention relates to a method for treating non-small cell lung cancer (NSCLC), comprising administering to a subject in need thereof a therapeutically effective amount of one of the provided compositions of anti-PD-1 antibody prolgolimab, wherein the concentration of anti-PD-1 antibody prolgolimab is from 15 mg/ml to 40 mg/ml, of the present invention.

In one of the embodiments, the invention relates to a method for treating non-small cell lung cancer (NSCLC), comprising administering to a subject in need thereof a therapeutically effective amount of one of the provided compositions of anti-PD-1 antibody prolgolimab, wherein the concentration of anti-PD-1 antibody prolgolimab is 20 mg/ml, of the present invention.

In one of the embodiments, the invention relates to a method for treating inoperable or metastatic non-small cell lung cancer, comprising administering to a subject in need thereof a therapeutically effective amount of one of the provided compositions of anti-PD-1 antibody prolgolimab, wherein the concentration of anti-PD-1 antibody prolgolimab is from 15 mg/ml to 40 mg/ml, of the present invention.

In one of the embodiments, the invention relates to a method for treating inoperable or metastatic non-small cell lung cancer, comprising administering to a subject in need thereof a therapeutically effective amount of one of the provided compositions of anti-PD-1 antibody prolgolimab, wherein the concentration of anti-PD-1 antibody prolgolimab is 20 mg/ml, of the present invention.

In one embodiment, there is provided a method for treating melanoma in a subject in need thereof comprising administering a therapeutically effective amount of an aqueous pharmaceutical composition of anti-PD-1 antibody comprising:

I. Composition per 1 mL:

| | |
|---|---|
| Prolgolimab | 20.0 mg |
| Sodium acetate trihydrate | 1.742 mg |
| Trehalose dihydrate | 100 mg |
| Acetic acid glac. | to pH 5.0 |
| Water for injections | up to 1 ml. |

In one embodiment, there is provided a method for treating melanoma in a subject in need thereof comprising administering a therapeutically effective amount of an aqueous pharmaceutical composition of anti-PD-1 antibody comprising:

II. Composition per 1 mL:

| Prolgolimab | 20.0 mg |
|---|---|
| Trehalose dihydrate | 100 mg |

| L-histidine | 0.92 mg |
|---|---|
| L-histidine hydrochloride | 2.96 mg |
| Water for injections | up to 1 ml. |

In one embodiment, there is provided a method for treating inoperable melanoma in a subject in need thereof comprising administering a therapeutically effective amount of an aqueous pharmaceutical composition of anti-PD-1 antibody comprising:

I. Composition per 1 mL:

| Prolgolimab | 20.0 mg |
|---|---|
| Sodium acetate trihydrate | 1.742 mg |
| Trehalose dihydrate | 100 mg |
| Acetic acid glac. | to pH 5.0 |
| Water for injections | up to 1 ml. |

In one embodiment, there is provided a method for treating inoperable melanoma in a subject in need thereof comprising administering a therapeutically effective amount of an aqueous pharmaceutical composition of anti-PD-1 antibody comprising:

II. Composition per 1 mL:

| Prolgolimab | 20.0 mg |
|---|---|
| Trehalose dihydrate | 100 mg |
| L-histidine | 0.92 mg |
| L-histidine hydrochloride | 2.96 mg |
| Water for injections | up to 1 ml. |

In one embodiment, there is provided a method for treating metastatic melanoma in a subject in need thereof comprising administering a therapeutically effective amount of an aqueous pharmaceutical composition of anti-PD-1 antibody comprising:

I. Composition per 1 mL:

| Prolgolimab | 20.0 mg |
|---|---|
| Sodium acetate trihydrate | 1.742 mg |
| Trehalose dihydrate | 100 mg |
| Acetic acid glac. | to pH 5.0 |
| Water for injections | up to 1 ml. |

In one embodiment, there is provided a method for treating metastatic melanoma in a subject in need thereof comprising administering a therapeutically effective amount of an aqueous pharmaceutical composition of anti-PD-1 antibody comprising:

II. Composition per 1 mL:

| Prolgolimab | 20.0 mg |
|---|---|
| Trehalose dihydrate | 100 mg |
| L-histidine | 0.92 mg |
| L-histidine hydrochloride | 2.96 mg |

| Water for injections | up to 1 ml. |
|---|---|

In one embodiment, there is provided a method for treating early stages of melanoma before and after definitive treatment in a subject in need thereof comprising administering a therapeutically effective amount of an aqueous pharmaceutical composition of anti-PD-1 antibody comprising:

I. Composition per 1 mL:

| Prolgolimab | 20.0 mg |
|---|---|
| Sodium acetate trihydrate | 1.742 mg |
| Trehalose dihydrate | 100 mg |
| Acetic acid glac. | to pH 5.0 |
| Water for injections | up to 1 ml. |

In one embodiment, there is provided a method for treating early stages of melanoma before and after definitive treatment in a subject in need thereof comprising administering a therapeutically effective amount of an aqueous pharmaceutical composition of anti-PD-1 antibody comprising:

II. Composition per 1 mL:

| Prolgolimab | 20.0 mg |
|---|---|
| Trehalose dihydrate | 100 mg |
| L-histidine | 0.92 mg |
| L-histidine hydrochloride | 2.96 mg |
| Water for injections | up to 1 ml. |

In one embodiment, there is provided a method for treating lung cancer in a subject in need thereof comprising administering a therapeutically effective amount of an aqueous pharmaceutical composition of anti-PD-1 antibody comprising:

I. Composition per 1 mL:

| Prolgolimab | 20.0 mg |
|---|---|
| Sodium acetate trihydrate | 1.742 mg |
| Trehalose dihydrate | 100 mg |
| Acetic acid glac. | to pH 5.0 |
| Water for injections | up to 1 ml. |

In one embodiment, there is provided a method for treating lung cancer in a subject in need thereof comprising administering a therapeutically effective amount of an aqueous pharmaceutical composition of anti-PD-1 antibody comprising:

II. Composition per 1 mL:

| Prolgolimab | 20.0 mg |
|---|---|
| Trehalose dihydrate | 100 mg |
| L-histidine | 0.92 mg |
| L-histidine hydrochloride | 2.96 mg |
| Water for injections | up to 1 ml. |

In one embodiment, there is provided a method for treating non-small cell lung cancer (NSCLC) in a subject in need thereof comprising administering a therapeutically effective amount of an aqueous pharmaceutical composition of anti-PD-1 antibody comprising:

I. Composition per 1 mL:

| Prolgolimab | 20.0 mg |
|---|---|
| Sodium acetate trihydrate | 1.742 mg |

(continued)

| Trehalose dihydrate | 100 mg |
| Acetic acid glac. | to pH 5.0 |
| Water for injections | up to 1 ml. |

In one embodiment, there is provided a method for treating non-small cell lung cancer (NSCLC) in a subject in need thereof comprising administering a therapeutically effective amount of an aqueous pharmaceutical composition of anti-PD-1 antibody comprising:

II. Composition per 1 mL:

| Prolgolimab | 20.0 mg |
| Trehalose dihydrate | 100 mg |
| L-histidine | 0.92 mg |
| L-histidine hydrochloride | 2.96 mg |
| Water for injections | up to 1 ml. |

In one embodiment, there is provided a method for treating inoperable or metastatic non-small cell lung cancer in a subject in need thereof comprising administering a therapeutically effective amount of an aqueous pharmaceutical composition of anti-PD-1 antibody comprising:

I. Composition per 1 mL:

| Prolgolimab | 20.0 mg |
| Sodium acetate trihydrate | 1.742 mg |
| Trehalose dihydrate | 100 mg |
| Acetic acid glac. | to pH 5.0 |
| Water for injections | up to 1 ml. |

In one embodiment, there is provided a method for treating inoperable or metastatic non-small cell lung cancer in a subject in need thereof comprising administering a therapeutically effective amount of an aqueous pharmaceutical composition of anti-PD-1 antibody comprising:

II. Composition per 1 mL:

| Prolgolimab | 20.0 mg |
| Trehalose dihydrate | 100 mg |
| L-histidine | 0.92 mg |
| L-histidine hydrochloride | 2.96 mg |
| Water for injections | up to 1 ml. |

In one of the embodiments, the invention relates to a method for treating melanoma, comprising administering to a subject in need thereof a therapeutically effective amount of anti-PD-1 antibody prolgolimab.

In one of the embodiments, the invention relates to a method for treating inoperable melanoma, comprising administering to a subject in need thereof a therapeutically effective amount of anti-PD-1 antibody prolgolimab.

In one of the embodiments, the invention relates to a method for treating metastatic melanoma, comprising administering to a subject in need thereof a therapeutically effective amount of anti-PD-1 antibody prolgolimab.

In one of the embodiments, the invention relates to a method for treating early stages of melanoma before and after definitive treatment, comprising administering to a subject in need thereof a therapeutically effective amount of anti-PD-1 antibody prolgolimab.

In one of the embodiments, the invention relates to a method for treating lung cancer, comprising administering to a subject in need thereof a therapeutically effective amount of anti-PD-1 antibody prolgolimab.

In one of the embodiments, the invention relates to a method for treating non-small cell lung cancer (NSCLC), comprising administering to a subject in need thereof a therapeutically effective amount of anti-PD-1 antibody prolgolimab.

In one of the embodiments, the invention relates to a method for treating inoperable or metastatic non-small cell lung cancer, comprising administering to a subject in need thereof a therapeutically effective amount of anti-PD-1 antibody prolgolimab.

The therapeutically effective amount of anti-PD-1 antibody of the invention, and of the aqueous compositions including the anti-PD-1 antibody prolgolimab of the invention, in the formulations provided depends on the condition being treated, the severity of the condition, the previous therapy and the patient's history and response to the therapeutic agent. A suitable dose can be adjusted by the decision of the attending physician so that it can be administered to the patient once or through several injections.

In one of the embodiments, the effective amount of anti-PD-1 antibody per dose for a patient is from about 0.01 to 10 mg per kilogram body weight, or about 1 to 10 mg per kilogram body weight, or about 0.05 mg per kilogram body weight, or about 0.25 mg per kilogram body weight, or about 0.5 mg per kilogram body weight, or about 1 mg per kilogram body weight, or about 2 mg per kilogram body weight, or about 3 mg per kilogram body weight, or about 4 mg per kilogram body weight, or about 5 mg per kilogram body weight, or about 6 mg per kilogram body weight, or about 7 mg per kilogram body weight, or about 8 mg per kilogram body weight, or about 9 mg per kilogram body weight, or about 10 mg per kilogram body weight.

The frequency of dosing may be normally about once per week, or about once every 2 weeks, or about once every 3 weeks.

In another embodiment, an acceptable dose for administration by infusion may contain 5-450 mg/dose, or may contain 40 mg, or 50 mg, or 60 mg per dose; or may contain 70 mg, or 80 mg, or 90 mg, or 100 mg per dose; or may contain 110 mg, or 120 mg, or 130 mg, or 140 mg per dose; or may contain 150 mg, or 160 mg, or 170 mg, or 180 mg per dose; or may contain 190 mg, or 200 mg, or 210 mg, or 220 mg per dose; or may contain 230 mg, or 240 mg, or 250 mg, or 260 mg per dose; or may contain 270 mg, or 280 mg, or 290 mg per dose, or may contain 300 mg, or 310 mg, or 320 mg, or 330 mg, or 340 mg, or 350 mg per dose; or may contain 360 mg, or 370 mg, or 380 mg, or 390 mg, or 400 mg, or 410 mg , or 420 mg, or 430 mg, or 440 mg, or 450 mg per dose.

In one embodiment of the invention, a dose may be delivered as one or more than one infusion. A dose may be delivered as one, two or three infusions. In some embodiments of the invention, the duration of treatment may be from one to several infusions. In some embodiments of the invention, improvement of the patient's condition can be achieved through treatment over an extended period. In some embodiments of the invention, the duration of treatment may be until progression of a disease or lifelong.

In another embodiment, the pharmaceutical compositions of the present invention may be prepared as a non-packaged formulation, and in essence, the components of the pharmaceutical composition are present in amounts higher than may be required for administration, and are diluted accordingly before administration.

Alternatively, a pharmaceutical composition may be frozen, spray-dried or lyophilized and reconstituted before application in an appropriate sterile carrier. Lyophilisation can be performed using techniques known in the art, which include the various steps, such as a freezing, annealing, primary and secondary drying.

The pharmaceutical compositions may be administered as a single therapeutic agent or in combination with additional therapeutic agents as needed. Thus, in one embodiment, the methods for treatment and/or prophylaxis provided are used in combination with administration of a therapeutically effective amount of another active agent. Another active agent can be administered before, during or after the administration of the pharmaceutical compositions of the present invention. The other active agent may be administered as part of the provided composition or, alternatively, as a separate formulation.

The administration of provided pharmaceutical compositions can be carried out by the various means, including a parenteral, oral, buccal, nasal, rectal and local administration. Parenteral administration may include, but not limited to transdermal, subcutaneous, intravenous, intra-arterial, intraperitoneal, intradermal, intracardiac, intraventricular, intracranial, intratracheal, intrathecal administration, intramuscular injection, intravitreal injection.

The pharmaceutical compositions of the present invention are particularly suitable for parenteral administration, i.e. subcutaneously, intramuscularly, intravenously, intraperitoneally, into the spinal cord, into the joints, intrasynovially and/or intrathecally. Parenteral administration may be by the bolus injection or continuous infusion. The pharmaceutical compositions for injection can be in the standard dosage form, for example, in the ampoules, the vials, the prefilled syringes or in the multi-dose containers with an added preservative, but not limited to this. In addition, a number of recent approaches to drug delivery have been developed, and the pharmaceutical compositions of the present invention are suitable for administration by these new methods, for example, BD Physioject™, Inject-ease®, Genject®, pen-injectors such as GenPen®, and needleless devices such as like MediJector® and BioJector®. The pharmaceutical composition of the present invention can also be adapted for not yet open methods of administration.

See also Langer, 1990, Science, 249: 1527-1533.

The provided pharmaceutical compositions can also be formulated as a depot preparation. Such long-acting formulations can be administered by the implantation (for example, subcutaneously or intramuscularly) or by the intramuscular injection. Thus, for example, the compositions can be modified using suitable polymeric or hydrophobic materials (for example, as an emulsion in an acceptable oil), or ion exchange resins, or as a moderately soluble derivatives, for example, as a moderately soluble salt.

The pharmaceutical compositions, if desired, may be provided in a vial, package, or in a dispenser device, which may contain one or more unit dosage forms containing the active ingredient. In one embodiment, the dispenser device may comprise a syringe containing a single dose of the liquid composition ready for injection. The syringe may be accompanied by instructions for administration.

In another embodiment, the present invention relates to a kit or container containing the aqueous pharmaceutical composition according to the invention. The concentration of the antibody in the aqueous pharmaceutical composition may vary over a wide range, but, as a rule, within the range of from about 1 to about 200 mg/ml. The kit may also be accompanied by instructions for use.

The method of obtaining the above compositions includes adding to the aqueous phase acetate buffer agents, followed by adding, in any sequence, the following components: trehalose, prolgolimab and/or a solubilizer selected from the group: polysorbate 20, polysorbate 80, Poloxamer 188 or a combination thereof.

The method of obtaining the above compositions includes adding to the aqueous phase histidine buffer agents, followed by adding, in any sequence, the following components: trehalose, prolgolimab and/or a solubilizer selected from the group: polysorbate 20, polysorbate 80, Poloxamer 188 or a combination thereof.

EXEMPLARY STUDY:

The following represents exemplary studies for determining reagents and concentrations of same for making the aqueous pharmaceutical composition of anti-PD-1 antibody prolgolimab of the present invention.

The examples and studies presented herein are for illustrative purposes, demonstrating the suitability of certain of the constituents used in aqueous pharmaceutical compositions of anti-PD-1 antibody prolgolimab. It will be understood that other methods and techniques may be used by a person of ordinary skill in the art without departing on the ideas of the present invention.

The suitability of the aqueous compositions of the present invention was tested using the exemplary methods described herein.

Example 1. Preparing stable anti-PD-1 antibody prolgolimab formulations

Antibody samples (5 mg/ml) were prepared in Stirred Cell (Millipore) under pressure. To this end, the initial antibody formulation was placed in a cell, the protein was concentrated under a compressed air stream to 10 mg/ml under continuous stirring, at least 10 times volume of an aqueous solution with the target formulation comprising the buffering agents, the osmotic agents and, if necessary, additional water soluble stabilizers was then added to the cell. After diafiltration, the antibody was concentrated to about 10 mg/ml, unloaded from the cell, and the exact protein concentration was measured by UV spectroscopy. The appropriate solution of excipients was then added to the sample to prepare a solution comprising protein at a target concentration of 5 ± 0.2 mg/ml.

The protein samples with a concentration of more than 50 mg/ml were prepared in Pellicon cassettes (Millipore) in a tangential flow mode. To this end, the initial antibody formulation was placed in a diafiltration tank, the protein was concentrated to 50-100 mg/ml, at least 10 times volume of the solution with the target formulation comprising buffering agents, osmotic agents and, if necessary, additional water soluble stabilizers was then supplied to the system. The concentrate of osmotic agents and water-soluble stabilizers may alternatively be added after diafiltration. After the diafiltration, the antibody was concentrated to a concentration exceeding the target one, unloaded from the system, and the exact protein concentration was measured. The appropriate solution of excipients was then added to the sample to prepare a solution with protein at the target concentration.

When obtaining the formulations comprising solubilizers, the surfactant concentrates were added to the antibody after diafiltering and concentrating with the final dilution of the antibody to the target concentration with a solution of excipients.

Before aseptic filling in the final container (for example, a glass/plastic vessel, vial or syringe), the antibody solution was filtered using a 0.22 $\mu$m membrane.

Example 2. Protein concentration assay in test samples

The protein concentration was measured by UV spectroscopy at a wavelength of 280 nm in UV transparent plates.

Each sample was diluted with the appropriate solution of excipients to ~0.5 mg/ml. 150 $\mu$l of the diluted sample was placed to UV spectroscopy plate well. An optical density of solutions in the plate wells was measured using a plate spectrophotometer at a wavelength of 280 nm. The appropriate solution of excipients was used as a reference solution.

The concentration (mg/ml) of the protein (C) was calculated using the following formula: $C = \dfrac{A(280) * b}{\varepsilon * l}$ , where

$A_{280}$ is a value of optical density at a wavelength of 280 nm;

$\varepsilon$ is an extinction coefficient of the test protein;

b is a total dilution factor for a sample;

l is layer thickness in a plate well; for 150 $\mu$l, l = 0.42 cm.

Example 3. Study of PEG aggregation

A solution of PEG 6000 with a mass concentration of 20-25% in a test excipient composition was prepared. The resulting solutions were filtered through a Durapore 0.45 $\mu$m filter.

The estimated amount of a sample, solution of excipients, and 20-25% PEG 6000 solution was transferred to 96 well UV plates so that the concentration of PEG 6000 in a number of the wells ranged from 0 to 18% and a protein concentration in each well was 1 mg/ml. All solutions obtained in wells were thoroughly mixed by a pipetting.

Turbidity the solutions was then evaluated visually, and optical density of the solutions at a wavelength of 400 nm was measured.

Protein precipitation in the presence of PEG is associated with the effect of volume substitution, i.e. protein is sterically excluded from regions of solvent by the polymer. This results in an increase in protein concentration until its solubility will be exceeded and it will be precipitated. The less stable is a sample, the lower is PEG 6000 concentration, at which the sample will form visible aggregates (opalescence).

Example 4. Evaluation of colloidal stability by shake test

The test samples were divided into 2 portions of 200 $\mu$l each and placed into glass vials, 1 vial per formulation was transferred to a refrigerator for aging at 2-8 °C, the rest vials were placed in a shaker and shaken at 800 rpm at 2-8 °C for the specified period. After the stress, the vials were vortexed and transferred for analysis.

Example 5. Evaluation of colloidal stability by cryoconcentration

The test samples were divided into 2 portions and placed into plastic vials: 1 vial per formulation was stored in a refrigerator at 2-8 °C; the rest vials were stored in a freezer at minus 16-20 °C for the specified period of time. After the stress, the vials were removed from the freezer, kept at room temperature until the content was completely thawed; the solutions were then vortexed and transferred for analysis.

Example 6. Evaluation of thermal stability by Thermostress

The test samples were divided into 2 portions and placed into separate glass vials: 1 vial per composition was stored in a refrigerator at 2-8 °C, the rest vials were incubated in a thermostat at a required temperature for the specified period of time. After heating, the vials were removed from the thermostat, kept at room temperature for about 15 minutes, and transferred for analysis.

Example 7. Determination of samples homogeneity by size-exclusion high-performance liquid chromatography (SEC HPLC)

Tosoh column TSK-GelG3000SW$_{XL}$ 7.8 mm ID $\times$ 30 cm, cat. No 08541.

Column temperature: 25 °C

Mobile phase flow rate: 0.7 ml/min.

Injection volume: 10 $\mu$l.

Sample concentration: 5 mg/ml.

Detector wavelength: 220 nm.

Elution time: 25 min.

Mobile phase: Disodium hydrogen phosphate anhydrous 7.1 mg/ml. Sodium chloride 17.54 mg/ml. The mobile phase pH was adjusted to 7.0 with orthophosphoric acid.

The change in purity after stress was calculated using the following formula:

```
    Δ = (fracture of main peak after stress - fracture of main peak
before stress)
```

Example 8. Determination of charge heterogeneity (charged form profile) of samples using Labchip GX II, Caliper.

Preparation of the test samples.

The samples were diluted to a concentration of 1 mg/ml. 2 $\mu$l of carboxypeptidase B (CpB) solution at 5 mg/ml was added to 200 $\mu$l of the resulting solution, the solution was mixed and incubated for 2 hours at 37°C. The test samples were dialyzed against 3 changes of water. For dialyzing, the test solutions were placed in 0.5 ml Amicon Ultra centrifuge tubes and centrifuged for 10 min at 10,000 rpm in Eppendorf Centrifuge 5417R. Intensity of solution absorption relative to water was measured using Cary 50 Bio spectrophotometer. Probes of test series with a concentration of 2 mg/ml were prepared. 3 $\mu$l/well of Labelling Buffer (from HT Protein Charge Variant Labeling Kit), 15 $\mu$l/well of test solutions and 3 $\mu$l/well of Dye Mixture (from HT Protein Charge Variant Labeling Kit) were introduced into a 96-well plate (Bio-Rad). The plate was placed in dark environment for 10 minutes, 36 $\mu$l/well of water was then added to each well and mixed by pipetting. The solutions were centrifuged at 1000 rpm in Eppendorf Centrifuge 5417R.

Preparation of working solutions and filling of chip

Working solutions and a chip were prepared according to the manufacturer protocol using HT Protein Charge Variant Labeling Kit. Launch of assay is a standard procedure. HT Protein Charge Variant 90s was used as a method of assay.

Example 9. Determination of sample purity using Caliper Labchip GXII under reducing and non-reducing conditions

Preparation of the test samples.

700 $\mu$l of HT Protein Express Sample Buffer was used to prepare each of denaturing and reducing solutions. For reducing samples, 24.5 $\mu$l of 1M dithiothreitol (DTT) was introduced. For non-reduced samples, 24.5 $\mu$l of 1M iodoacetamide (IAM) was introduced to the sample buffer as an alkylating agent.

For each sample, two microtubes were prepared; 35 $\mu$l of denaturing buffer was added to one of the two tubes, and 35 $\mu$l of reducing buffer was added to the other one. The samples were diluted to a concentration of 2 mg/ml. 5 $\mu$l of the sample was introduced into each pair of tubes. The samples were denatured at 100°C for 5 minutes. The tubes were vortexed, 70 $\mu$l/tube of water was then added, and the tubes were vortexed. 44 $\mu$l/well of each sample was transferred to wells of a 96-well plate.

Preparation of working solutions and filling of chip

Working solutions and a chip were prepared according to the manufacturer protocol using HT Protein Express Reagent Kit. Launch of assay is a standard procedure. HT Protein Express 200 was selected as a method of assay.

Example 10. Determination of charged form profile of samples by ion exchange (IE) high performance liquid chromatography (HPLC)

Column: ProPac WCX-10 Analytical, 4 $\times$ 250 mm.
Precolumn: Pro Pac WCX-10G, 4 $\times$ 50 mm.
Column temperature: 30 °C.
Mobile phase flow rate: 0.7 ml/min.
Injection volume: 50 $\mu$l.
Sample concentration: 1 mg/ml.
Detector wavelength: 220 nm.
Elution time: 60 min.
Mobile phase:

Eluent A: 0.03 M 2-(N-morpholino)ethanesulfonic acid (MES), pH 6.0
Eluent B: 0.03 M MES, 0.5 M NaCl, pH 6.0.
Eluent A gradient: 86 % - 0 % - 86 %.

Before the assay, the test sample was treated with carboxypeptidase B at +37°C $\pm$ 1°C for 2 h.

Absolute change in the charged form profile after stress was calculated by the following formula:

```
     Δ = |acid fraction content before stress — acid fraction content
after stress| + |dominant fraction content before stress — dominant
fraction content after stress| + |base fraction content before stress
— base fraction content after stress.
```

Example 11. Determination of low molecular weight impurities by reducing and non-reducing vertical gel electrophoresis (VEP) in polyacrylamide gel (PAG)

Polyacrylamide gel (PAAG) was prepared in glass plates in the presence of sodium dodecyl sulfate, said plates consisting of a concentrating layer of 4% PAAG and a separating layer of 12.5% PAG (under reducing conditions) / 8% PAG (under non-reducing conditions).

An electrophoresis chamber was assembled and installed in accordance with a vertical electrophoresis apparatus user manual. The probes were prepared by diluting samples with purified water to a final concentration of 1 mg/ml. A volume equivalent of 40 $\mu$g was taken, and the prepared probes of the test sample were mixed in a ratio of 3:1 (volume/volume) with a 4x sample buffer solution containing 2-mercaptoethanol (reducing conditions) and not containing 2-mercaptoethanol (non-reducing conditions), and stirred. The resulting solutions were incubated at (99 $\pm$ 1) °C for 3 min (samples containing 2-mercaptoethanol) and at (99 $\pm$ 1) °C for 1 min (samples without 2-mercaptoethanol). The solutions were cooled to room temperature, mixed, and transferred to PAG wells under an electrode buffer solution layer.

Electrophoresis was performed in constant current mode using a water cooling system. Parameters of power supply were set: the voltage was 110 V during passing of the dye front through the concentrating gel. After moving of the dye front to the lower separation gel at the level of 5-7 mm, the voltage was increased to 180 V. The power supply was turned off when the dye front reached the bottom line of the gel.

After electrophoresis, the gels were detached from the glasses, and the proteins were fixed in a fixing solution for 16-18 hours at room temperature. The gels were then stained (in an Acid Blue 83 solution) and washed to obtain a clear visualization of the bands. The gels were scanned. The purity and impurities in the test samples were evaluated using GelPro software.

Example 12. Relative specific activity determination

The relative specific activity of monoclonal anti-PD-1 antibody samples was evaluated by their ability to specifically bind to PD-1 protein on the surface of Jurkat PD-1 NFAT line cell membrane. The day before the analysis, PDL-1 was immobilized in the wells of culture plates. The following day the plates were washed, 50 $\mu$l/well of a solution of phytohaemagglutinin P (PanEco, Russia, Cat. No. M021) was added. Using a Freedom Evo robot, serial dilutions of the standard and test samples were prepared and introduced into culture plates at 10 $\mu$l/well. Jurkat PD-1 NFAT cell suspension was introduced into culture plates at 40 $\mu$l/well. The plates were incubated for 4-6 hours at 37 °C, 5% $CO_2$. All above procedures were performed under aseptic conditions.

Following incubation, 100 $\mu$l/well of BioGlo (Promega, USA, Cat. No. G7941) solution was added, and luminescence level was determined.

Using Magellan software, we constructed four-parameter curves for the dependence of the average luminescence value on the protein concentration for solutions of the standard sample and the test sample located on the same tablet.

The relative specific activity of the test sample as % (RP) was calculated by the following formula:

$$RP = \frac{ED_{50}st}{ED_{50}test} \cdot 100\%,$$

where

$ED_{50}st$ is the value of half effective dose of a standard sample, ng/ml;
$ED_{50}test$ is the value of half effective dose of a test sample, ng/ml.

The average value of relative specific activity was taken as the final result, said average value was calculated from three independent measurements (determined from 3 different culture plates).

Example 13. Determination of viscosity

Dynamic viscosity of test solutions was measured by rotational viscometry using CAP2000+L Brookfield viscometer.

Example 14. Study of the origin of buffer solution

Test formulations

4 buffer solutions were selected for the present study. Molarity and pH of the buffer were selected taking into account the restrictions for possible subcutaneous administration, as well as pI of antibody (pH of test solutions were selected at minimum possible physiological values).

Composition (per 1 mL):

| | | |
|---|---|---|
| Acetate, pH 5.0 | Prolgolimab | 5 mg |
| | Sodium acetate trihydrate (t/h) | 0.436 mg |
| | Acetic acid glacial (glac.) to | pH 5.0 |
| Citrate, pH 5.0 | Prolgolimab | 5 mg |
| | Sodium citrate dihydrate (d/h) | 0.932 mg |
| | Citric acid anhydrous (anhyd.) | 0.352 mg |
| Histidine, pH 5.5 | Prolgolimab | 5 mg |
| | L-histidine | 0.23 mg |
| | L-histidine hydrochloride monohydrate (m/h) | 0.74 mg |
| Phosphate, pH 6.0 | Prolgolimab | 5 mg |
| | Sodium dihydrogen phosphate monohydrate (m/h) | 1.21 mg |
| | Sodium hydrogen phosphate anhydrous (anhyd.) | 0.17 mg |

Determination of colloidal stability by PEG aggregation

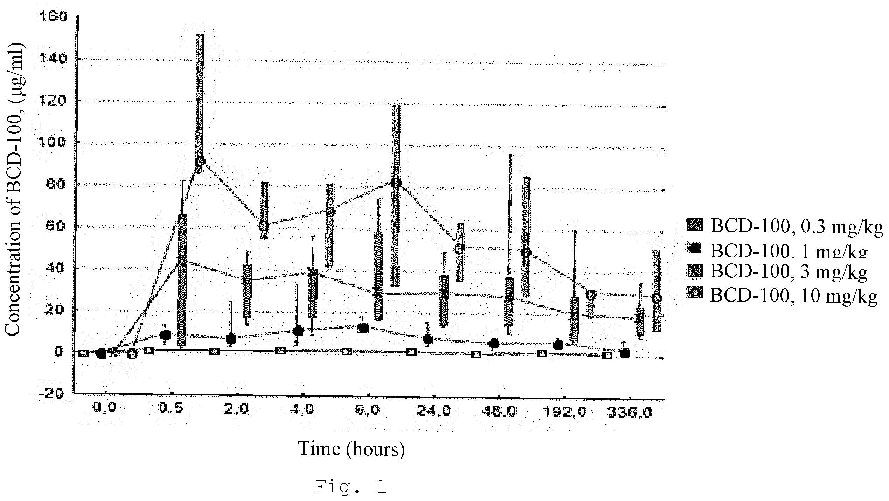

The study was performed in three replications for each sample. The results are shown in Table 1 and in Fig. 1.

[00404] Table 1. Solutions average optical density immediately after preparation at wavelength of 400 nm.

| PEG 6000, % | 0 | 2 | 4 | 6 | 8 | 10 | 12 | 14 | 16 | 18 |
|---|---|---|---|---|---|---|---|---|---|---|
| Acetate, pH 5.0 | 0.059 | 0.061 | 0.063 | 0.063 | 0.062 | 0.064 | 0.066 | 0.068 | 0.069 | 0.070 |
| Citrate, pH 5.0 | 0.056 | 0.058 | 0.059 | 0.301 | 0.932 | 1.118 | 1.393 | 1.398 | 1.412 | 1.541 |
| Histidine, pH 5.5 | 0.058 | 0.062 | 0.062 | 0.063 | 0.064 | 0.066 | 0.978 | 1.255 | 1.387 | 1.563 |
| Phosphate, pH 6.0 | 0.053 | 0.061 | 0.061 | 0.497 | 1.031 | 1.410 | 1.612 | 1.658 | 1.684 | 1.710 |

As a result, the samples in histidine and acetate buffer solutions showed the highest colloidal stability in the presence of PEG. The phosphate- and citrate-based compositions were excluded from further study, since aggregation at 6% PEG is an indicator of unsatisfying colloidal stability. Based on the results obtained, the acetate and histidine buffer solutions were selected for further pH/molarity selection.

Example 15. Selection of solution pH/buffer capacity

Test formulations (per ml)

| Name | pH 5.5 | pH 6.0 | pH 6.5 |
|---|---|---|---|
| 5 mM histidine buffer solution | Prolgolimab 5 mg L-histidine 0.23 mg L-histidine h/c m/h 0.74 mg | Prolgolimab 5 mg L-histidine 0.45 mg L-histidine h/c m/h 0.45 mg | Prolgolimab 5 mg L-histidine 0.58 mg L-histidine h/c m/h 0.27 mg |
| 10 mM histidine buffer solution | Prolgolimab 5 mg L-histidine 0.46 mg L-histidine h/c m/h 1.48 mg | Prolgolimab 5 mg L-histidine 0.9 mg L-histidine h/c m/h 0.9 mg | Prolgolimab 5 mg L-histidine 1.16 mg L-histidine h/c m/h 0.54 mg |
| 20 mM Histidine buffer solution | Prolgolimab 5 mg L-histidine 0.92 mg L-histidine h/c m/h 2.96 mg | Prolgolimab 5 mg L-histidine 1.8 mg L-histidine h/c m/h 1.8 mg | Prolgolimab 5 mg L-histidine 2.32 mg L-histidine h/c m/h 1.08 mg |

| Name | pH 4.5 | pH 5.0 | pH 5.5 |
|---|---|---|---|
| 5 mM acetate buffer solution | Prolgolimab 5 mg Sodium acetate t/h 0.24 mg Acetic acid to pH to 4.5 | Prolgolimab 5 mg Sodium acetate t/h 0.44 mg Acetic acid to pH to 5.0 | Prolgolimab 5 mg Sodium acetate t/h 0.58 mg Acetic acid to pH to 5.5 |
| 10 mM acetate buffer solution | Prolgolimab 5 mg Sodium acetate t/h 0.49 mg Acetic acid to pH to 4.5 | Prolgolimab 5 mg Sodium acetate t/h 0.87 mg Acetic acid to pH to 5.0 | Prolgolimab 5 mg Sodium acetate t/h 1.16 mg Acetic acid to pH to 5.5 |
| 20 mM acetate buffer solution | Prolgolimab 5 mg Sodium acetate t/h 0.98 mg Acetic acid to pH to 4.5 | Prolgolimab 5 mg Sodium acetate t/h mg 1.74 Acetic acid to pH to 5.0 | Prolgolimab 5 mg Sodium acetate t/h 2.31 mg Acetic acid to pH to 5.5 |

The study was conducted by thermostress at 50°C for 72 hours. The results are shown in Table 2. Samples homogeneity was determined by SE HPLC and electrophoresis using Labchip system. Charged form profile was analyzed using Labchip system.

Table 2. Results summary for quality indicators before and after thermostress

| | pH theor. | pH pract. | pH pract. after 50°C 72h | Initial control | | | Thermostress 72 h | | | | | | |
| | | | | SE HPLC | Monomer content, EP Lab-Chip | | SE HPLC | Change in content of frac-tions, LabChip* | | | Change in mono-mer content, EP LabChip | |
| | | | | Monomer content, % | Re-duc-ing EP,% | Non-reduc-ing EP,% | Δ monomer content, % | Δ acid frac-tions, % | Δ domi-nant frac-tions, % | Δ base frac-tions, % | Reduc-ing EP, % | Non-re-ducing EP, % |
|---|---|---|---|---|---|---|---|---|---|---|---|---|
| 5 mM Histidine pH 5.5 | | 5.71 | 5.72 | 98.46 | 98.70 | 94.50 | -0.07 | +23.80 | -14.13 | -9.68 | -0.09 | -5.69 |
| 10 mM Histidine pH 5.5 | 5.5 | 5.68 | 5.68 | 98.55 | 98.49 | 95.05 | -0.20 | +24.87 | -15.09 | -9.77 | 0.38 | -5.74 |
| 20 mM Histidine pH 5.5 | | 5.58 | 5.63 | 98.53 | 99.05 | 94.12 | -0.19 | +24.63 | -15.21 | -9.42 | -0.50 | -2.24 |
| 5 mM Histidine pH 6.0 | | 6.20 | 6.12 | 98.40 | 98.97 | 93.99 | -0.18 | +23.00 | -14.16 | -8.84 | -0.22 | -2.12 |
| 10 mM Histidine pH 6.0 | 6.0 | 6.18 | 6.20 | 98.57 | 98.94 | 94.18 | -0.23 | +24.37 | -14.92 | -9.45 | -0.25 | -5.01 |
| 20 mM Histidine pH 6.0 | | 6.12 | 6.17 | 98.61 | 98.95 | 93.99 | -0.16 | +25.77 | -16.16 | -9.61 | -0.27 | -2.36 |
| 5 mM Histidine pH 6.5 | | 6.64 | 6.67 | 98.70 | 98.94 | 94.09 | -0.43 | +23.11 | -13.90 | -9.20 | -0.39 | -2.15 |
| 10 mM Histidine pH 6.5 | 6.5 | 6.42 | 6.38 | 98.59 | 99.02 | 93.75 | -0.31 | +24.69 | -15.34 | -9.37 | -0.34 | -2.89 |
| 20 mM Histidine pH6.5 | | 6.36 | 6.34 | 98.74 | 99.07 | 93.82 | -0.40 | +27.20 | -17.41 | -9.80 | -0.45 | -2.43 |
| 5 mM Acetate pH 4.5 | | 4.91 | 5.03 | 98.79 | 98.93 | 90.95 | -0.46 | +22.45 | -14.06 | -8.40 | -0.37 | 0.39 |
| 10 mM Acetate pH 4.5 | 4.5 | 4.87 | 4.87 | 98.58 | 98.88 | 90.89 | -0.39 | +25.69 | -15.61 | -10.08 | -0.58 | 0.09 |
| 20 mM Acetate pH 4.5 | | 4.86 | 4.66 | 98.59 | 98.86 | 92.85 | -0.41 | +26.48 | -16.84 | -9.64 | -1.09 | -1.35 |

Table 2. Results summary for quality indicators before and after thermostress (continued)

| | pH. theor. | pH. pract. | pH. pract. after 50°C 72h | Initial control | | | Thermostress 72 h | | | | | | |
|---|---|---|---|---|---|---|---|---|---|---|---|---|
| | | | | SE HPLC | | | SE HPLC | Change in content of fractions, LabChip* | | | Change in monomer content, EP Lab-Chip | |
| | | | | Mono-mer con-tent, % | Re-duc-ing EP, % | Non-re-ducing EP, % | Δ monomer content, % | Δ acid fractions | Δ domi-nant frac-tions, % | Δ base frac-tions, % | Reducing EP, % | Non-re-ducing EP, % |
| 5 mM Acetate pH 5.0 | 5.0 | 5.44 | 5.43 | 98.55 | 99.20 | 93.31 | -0.33 | +24.28 | -14.70 | -9.59 | -0.62 | -0.99 |
| 10 mM Acetate pH 5.0 | | 5.33 | 5.31 | 98.65 | 99.23 | 92.79 | -0.42 | +25.35 | -15.71 | -9.65 | -0.63 | -0.87 |
| 20 mM Acetate pH 5.0 | | 5.22 | 5.20 | 98.60 | 98.85 | 94.67 | -0.37 | +24.30 | -15.75 | -9.54 | -0.38 | 1.65 |
| 5 mM Acetate pH 5.5 | 5.5 | 6.11 | 5.84 | 98.55 | 99.17 | 92.48 | -0.35 | +24.52 | -15.18 | -9.35 | -0.37 | 0.26 |
| 10 mM Acetate pH 5.5 | | 5.71 | 5.72 | 98.48 | 99.07 | 92.50 | -0.31 | +25.40 | -15.40 | -10.00 | -0.25 | 2.03 |
| 20 mM Acetate pH 5.5 | | 5.63 | 5.66 | 98.67 | 98.95 | 93.94 | -0.39 | +26.42 | -16.68 | -9.74 | -1.17 | -1.97 |

* The change was calculated by the following formula: Δ = fraction content after stress — fraction content before stress

▉ - the best result          ▒ - the worst result          ☐ - initial control or average result

The results of the study show that the presence of a monoclonal anti-PD-1 antibody (prolgolimab) in an aqueous solution results in increase in pH level. pH is best stabilized by 20 mM buffer solutions.

All test samples exhibited high aggregation stability in SE HPLC, because the increase in impurities after thermostress was not more than 0.5% for all samples. Histidine-based samples with pH 5.5 - 6.0 and acetate-based samples with pH 5.0 - 5.5 exhibited the best stability.

All formulations exhibited similar stability of charged form profile; quantitative difference of the change in fraction content between the formulations is within the accuracy limits of the method.

According to the results of reducing gel electrophoresis, all histidine-based formulations exhibited increased stability, whereas under non-reducing conditions, the acetate-based solutions showed the best results.

Based on the resulting data on stability in terms of pH, purity and charged form profile of monoclonal anti-PD-1 antibody, the following formulations of excipients are recommended for further study:

| 20 mM Acet 5.0/20 mM Acetate buffer solution pH 5.0 | Sodium acetate t/h | 1.74 mg/ml |
| | Acetic acid qlac. | to pH 5.0 |
| 20 mM His 5.5 /20 mM his- tidine buffer solution pH 5.5 | L-histidine | 0.92 mg/ml |
| | L-histidine hydrochloride m/h | 2.96 mg/ml |

Example 16. Selection of histidine-based pharmaceutical composition

Test formulations

For screening for a stable pharmaceutical composition based on 20 mM histidine buffer solution with pH 5.5, the following excipients were selected: mannitol, trehalose dihydrate, sucrose (osmotic agents). All test solutions are isotonic.

Test samples (mg to 1 ml)

| No. | Prolgolimab | Osmotic agents | | | Stabilizers | | Solubilizers | | 20 mM His pH 5.5 |
| | | Mannitol | Trehalose d/h | Sucrose | L-Proline | Glycine | Polysorbate 80 | Poloxamer 188 | |
|---|---|---|---|---|---|---|---|---|---|
| 1 | 5 | 50 | | | | | | | to 1 ml |
| 2 | 5 | 50 | | | | | 0.2 | | to 1 ml |
| 3 | 5 | 50 | | | | | 0.5 | | to 1 ml |
| 4 | 5 | 50 | | | | | 0.8 | | to 1 ml |
| 5 | 5 | 50 | | | | | | 0.2 | to 1 ml |
| 6 | 5 | 50 | | | | | | 0.5 | to 1 ml |
| 7 | 5 | 50 | | | | | | 0.8 | to 1 ml |
| 8 | 5 | 5 | | | 28.8 | | | | to 1 ml |
| 5 | 5 | 5 | | | 28.8 | | 0.2 | | to 1 ml |
| 10 | 5 | 5 | | | 28.8 | | 0.5 | | to 1 ml |
| 11 | 5 | 5 | | | 28.8 | | 0.8 | | to 1 ml |
| 12 | 5 | 5 | | | 28.8 | | | 0.2 | to 1 ml |
| 13 | 5 | 5 | | | 28.8 | | | 0.5 | to 1 ml |
| 14 | 5 | 5 | | | 28.8 | | | 0.8 | to 1 ml |
| 15 | 5 | 33.5 | | | | 7.5 | | | to 1 ml |
| 16 | 5 | 33.5 | | | | 7.5 | 0.2 | | to 1 ml |
| 17 | 5 | 33.5 | | | | 7.5 | 0.5 | | to 1 ml |
| 18 | 5 | 33.5 | | | | 7.5 | 0.8 | | to 1 ml |
| 19 | 5 | 33.5 | | | | 7.5 | | 0.2 | to 1 ml |
| 20 | 5 | 33.5 | | | | 7.5 | | 0.5 | to 1 ml |
| 21 | 5 | 33.5 | | | | 7.5 | | 0.8 | to 1 ml |
| 22 | 5 | | 100 | | | | | | to 1 ml |
| 23 | 5 | | 100 | | | | 0.2 | | to 1 ml |
| 24 | 5 | | 100 | | | | 0.5 | | to 1 ml |
| 25 | 5 | | 100 | | | | 0.8 | | to 1 ml |
| 26 | 5 | | 100 | | | | | 0.2 | to 1 ml |
| 27 | 5 | | 100 | | | | | 0.5 | to 1 ml |
| 23 | 5 | | 100 | | | | | 0.8 | to 1 ml |
| 29 | 5 | | 10 | | 28.8 | | | | to 1 ml |
| 30 | 5 | | 10 | | 28.8 | | 0.2 | | to 1 ml |
| 31 | 5 | | 10 | | 28.8 | | 0.5 | | to 1 ml |
| 32 | 5 | | 10 | | 28.8 | | 0.8 | | to 1 ml |
| 33 | 5 | | 10 | | 28.8 | | | 0.2 | to 1 ml |
| 34 | 5 | | 10 | | 28.8 | | | 0.5 | to 1 ml |

| No. | Prolgolimab | Osmotic agents | | | Stabilizers | | Solubilizers | | 20 mM His pH 5.5 |
| | | Mannitol | Trehalose d/h | Sucrose | L-Proline | Glycine | Polysorbate 80 | Poloxamer 188 | |
|---|---|---|---|---|---|---|---|---|---|
| 35 | 5 | | 10 | | 28.8 | | | 0.8 | to 1 ml |
| 36 | 5 | | 67 | | | 7.5 | | | to 1 ml |
| 37 | 5 | | 67 | | | 7.5 | 0.2 | | to 1 ml |
| 38 | 5 | | 67 | | | 7.5 | 0.5 | | to 1 ml |
| 39 | 5 | | 67 | | | 7.5 | 0.8 | | to 1 ml |
| 40 | 5 | | 67 | | | 7.5 | | 0.2 | to 1 ml |
| 41 | 5 | | 67 | | | 7.5 | | 0.5 | to 1 ml |
| 42 | 5 | | 67 | | | 7.5 | | 0.8 | to 1 ml |
| 43 | 5 | | | 100 | | | | | to 1 ml |
| 44 | 5 | | | 100 | | | 0.2 | | to 1 ml |
| 45 | 5 | | | 100 | | | 0.5 | | to 1 ml |
| 46 | 5 | | | 100 | | | 0.8 | | to 1 ml |
| 47 | 5 | | | 100 | | | | 0.2 | to 1 ml |
| 48 | 5 | | | 100 | | | | 0.5 | to 1 ml |
| 49 | 5 | | | 100 | | | | 0.8 | to 1 ml |
| 50 | 5 | | | 10 | 28.8 | | | | to 1 ml |
| 51 | 5 | | | 10 | 28.8 | | 0.2 | | to 1 ml |
| 52 | 5 | | | 10 | 28.8 | | 0.5 | | to 1 ml |
| 53 | 5 | | | 10 | 28.8 | | 0.8 | | to 1 ml |
| 54 | 5 | | | 10 | 28.8 | | | 0.2 | to 1 ml |
| 55 | 5 | | | 10 | 28.8 | | | 0.5 | to 1 ml |
| 56 | 5 | | | 10 | 28.8 | | | 0.8 | to 1 ml |
| 57 | 5 | | | 67 | | 7.5 | | | to 1 ml |
| 58 | 5 | | | 67 | | 7.5 | 0.2 | | to 1 ml |
| 59 | 5 | | | 67 | | 7.5 | 0.5 | | to 1 ml |
| 60 | 5 | | | 67 | | 7.5 | 0.8 | | to 1 ml |
| 61 | 5 | | | 67 | | 7.5 | | 0.2 | to 1 ml |
| 62 | 5 | | | 67 | | 7.5 | | 0.5 | to 1 ml |
| 63 | 5 | | | 67 | | 7.5 | | 0.8 | to 1 ml |

Sample stability was evaluated by: thermostress at 50°C for 72 hours, shake test for 120 h at a speed of 800 rpm, and one-time freezing at minus 16 - 20 °C and thawing at +25 ± 1°C. Turbidity of the solutions was evaluated by spectrophotometry at 400 nm. Samples homogeneity was determined by SE HPLC and electrophoresis using Labchip system. Charged form profile was analyzed using Labchip system.

EP 4 019 047 A1

**Table 3.** Results summary for quality indicators before and after stress obtained after analyzing the samples by gel filtration and UV spectrophotometry.

| No. | Composition of excipients, mg/ml | | | | | | | | Monomer content SE HPLC, % | Change in purity SE HPLC, % | | | Optic. density, 400 nm | | Selection for iso-form pro-file analysis |
| | Prolgolimab | Mannitol | Trehalose d/h | Sucrose | L-Proline | Glycine | Polysorbate 80 | Poloxamer 188 | | Shake test 120 h | Freezing -18°C, thawing +25°C | Ther-mostress, +50°C 96 h | Shake test 120 h | Freezing -18°C, thawing +25°C | |
|---|---|---|---|---|---|---|---|---|---|---|---|---|---|---|---|
| 1 | 5 | 50 | | | | | | | 97.54 | -0.30 | -1.05 | -1.37 | 0.1232 | 0.0700 | |
| 2 | 5 | 50 | | | | | 0.2 | | 97.52 | -0.26 | -1.33 | -1.77 | 0.1795 | 0.0735 | |
| 3 | 5 | 50 | | | | | 0.5 | | 97.54 | -0.33 | -1.72 | -1.54 | 0.1703 | 0.0731 | |
| 4 | 5 | 50 | | | | | 0.8 | | 97.48 | -0.31 | -1.90 | -28.94 | 0.1822 | 0.0764 | |
| 5 | 5 | 50 | | | | | | 0.2 | 97.14 | 0.12 | -1.16 | -0.80 | 0.1852 | 0.0752 | |
| 6 | 5 | 50 | | | | | | 0.5 | 97.53 | -0.32 | -3.22 | -1.29 | 0.1740 | 0.0729 | |
| 7 | 5 | 50 | | | | | | 0.8 | 97.48 | -0.72 | -4.45 | -1.24 | 0.2017 | 0.0730 | |
| 8 | 5 | 5 | | | 28.8 | | | | 97.50 | -0.22 | -1.11 | -1.50 | 0.0938 | 0.0747 | |
| 9 | 5 | 5 | | | 28.8 | | 0.2 | | 98.05 | -0.71 | -2.16 | -2.75 | 0.1541 | 0.0758 | |
| 10 | 5 | 5 | | | 28.8 | | 0.5 | | 98.12 | -0.80 | -2.40 | -3.07 | 0.1456 | 0.0779 | |
| 11 | 5 | 5 | | | 28.8 | | 0.8 | | 98.23 | -0.98 | -2.67 | -3.18 | 0.1477 | 0.0794 | |
| 12 | 5 | 5 | | | 28.8 | | | 0.2 | 97.93 | -0.58 | -1.80 | -2.88 | 0.1557 | 0.0767 | |
| 13 | 5 | 5 | | | 28.8 | | | 0.5 | 97.90 | -0.61 | -1.98 | -2.04 | 0.1548 | 0.0759 | |
| 14 | 5 | 5 | | | 28.8 | | | 0.8 | 97.95 | -0.68 | -2.12 | -2.05 | 0.1440 | 0.0761 | |

**Table 3.** Results summary for quality indicators before and after stress obtained after analyzing the samples by gel filtration and UV spectrophotometry. (continued)

| No. | Composition of excipients, mg/ml | | | | | | | | Monomer content SE HPLC, % | Change in purity SE HPLC, % | | | Optic. density, 400 nm | | Selection for iso-form pro-file analysis |
| | Prolgolimab | Mannitol | Trehalose d/h | Sucrose | L-Proline | Glycine | Polysorbate 80 | Poloxamer 181 | | Shake test 120 h | Freezing -18°C, thawing +25°C | Ther-mostress, +50°C 96 h | Shake test 120 h | Freezing - 18°C, thawing +25°C | |
|---|---|---|---|---|---|---|---|---|---|---|---|---|---|---|---|
| 15 | 5 | 33.5 | | | | 7.5 | | | 98.00 | -0.72 | -1.27 | -1.39 | 0.0859 | 0.0702 | |
| 16 | 5 | 33.5 | | | | 7.5 | 0.2 | | 97.94 | -0.79 | -1.42 | -2.39 | 0.1100 | 0.0746 | |
| 17 | 5 | 33.5 | | | | 7.5 | 0.5 | | 97.84 | -0.66 | -1.65 | -2.96 | 0.1035 | 0.0762 | |
| 18 | 5 | 33.5 | | | | 7.5 | 0.8 | | 97.87 | -0.71 | -2.08 | -2.90 | 0.1094 | 0.0763 | |
| 19 | 5 | 33.5 | | | | 7.5 | | 0.2 | 97.86 | -0.63 | -1.76 | -1.22 | 0.0827 | 0.0722 | |
| 20 | 5 | 33.5 | | | | 7.5 | | 0.5 | 97.82 | -0.63 | -2.44 | -1.35 | 0.0994 | 0.0740 | |
| 21 | 5 | 33.5 | | | | 7.5 | | 0.8 | 97.93 | -0.75 | -2.87 | -1.37 | 0.1115 | 0.0735 | |
| 22 | 5 | | 100 | | | | | | 97.96 | -0.69 | -0.68 | -0.56 | 0.0849 | 0.0716 | V |
| 23 | 5 | | 100 | | | | 0.2 | | 97.90 | -0.64 | -0.68 | -0.82 | 0.1135 | 0.0747 | V |
| 24 | 5 | | 100 | | | | 0.5 | | 97.86 | -0.65 | -0.06 | -0.89 | 0.1090 | 0.0763 | V |
| 25 | 5 | | 100 | | | | 0.8 | | 97.88 | -0.73 | -0.66 | -0.78 | 0.1110 | 0.0784 | V |
| 26 | 5 | | 100 | | | | | 0.2 | 97.90 | -0.66 | -0.65 | -0.82 | 0.1096 | 0.0730 | V |
| 27 | 5 | | 100 | | | | | 0.5 | 97.92 | -0.68 | -0.62 | -0.60 | 0.1124 | 0.0741 | V |

**Table 3.** Results summary for quality indicators before and after stress obtained after analyzing the samples by gel filtration and UV spectrophotometry. (continued)

| No. | Composition of excipients, mg/ml | | | | | | | | Monomer content SE HPLC, % | Change in purity SE HPLC, % | | | Optic. density, 400 nm | | Selection for isoform profile analysis |
|---|---|---|---|---|---|---|---|---|---|---|---|---|---|---|---|
| | Prolgolimab | Mannitol | Trehalose d/h | Sucrose | L-Proline | Glycine | Polysorbate 80 | Poloxamer 188 | | Shake test 120 h | Freezing −18°C, thawing +25°C | Thermostress, +50°C 96 h | Shake test 120 h | Freezing −18°C, thawing +25°C | |
| 28 | 5 | | 100 | | | | | 0.8 | 97.82 | −0.63 | −0.55 | −0.56 | 0.1147 | 0.0740 | V |
| 29 | 5 | | 10 | | 28.8 | | | | 97.74 | −0.46 | −0.91 | −1.31 | 0.0890 | 0.0725 | |
| 30 | 5 | | 10 | | 28.8 | | 0.2 | | 97.86 | −0.59 | −1.02 | −1.64 | 0.1399 | 0.0776 | |
| 31 | 5 | | 10 | | 28.8 | | 0.5 | | 97.79 | −0.52 | −1.13 | −1.72 | 0.1476 | 0.0782 | |
| 32 | 5 | | 10 | | 28.8 | | 0.8 | | 97.89 | −0.67 | −1.17 | −1.90 | 0.1375 | 0.0790 | |
| 33 | 5 | | 10 | | 28.8 | | | 0.2 | 97.80 | −0.39 | −1.14 | −1.35 | 0.1499 | 0.0756 | |
| 34 | 5 | | 10 | | 28.8 | | | 0.5 | 97.77 | −0.53 | −1.05 | −1.53 | 0.1460 | 0.0760 | |
| 35 | 5 | | 10 | | 28.8 | | | 0.8 | 97.73 | −0.52 | −1.03 | −1.41 | 0.1409 | 0.0751 | |
| 36 | 5 | | 67 | | | 7.5 | | | 97.89 | −0.61 | −0.62 | −0.33 | 0.0802 | 0.0705 | V |
| 37 | 5 | | 67 | | | 7.5 | 0.2 | | 97.98 | −0.86 | −0.81 | −0.89 | 0.1006 | 0.0748 | V |
| 38 | 5 | | 67 | | | 7.5 | 0.5 | | 97.84 | −0.79 | −0.67 | −1.22 | 0.1108 | 0.0762 | V |
| 39 | 5 | | 67 | | | 7.5 | 0.8 | | 97.94 | −0.86 | −0.82 | −1.95 | 0.1057 | 0.0779 | V |
| 40 | 5 | | 67 | | | 7.5 | | 0.2 | 97.95 | −0.72 | −0.73 | −0.50 | 0.0849 | 0.0739 | V |
| 41 | 5 | | 67 | | | 7.5 | | 0.5 | 97.81 | −0.61 | −0.57 | −0.30 | 0.0969 | 0.0744 | V |
| 42 | 5 | | 67 | | | 7.5 | | 0.8 | 98.27 | −1.14 | −1.04 | −0.72 | 0.0967 | 0.0733 | V |
| 43 | 5 | | | 100 | | | | | 97.80 | −0.36 | −0.31 | −0.50 | 0.0858 | 0.0737 | V |
| 44 | 5 | | | 100 | | | 0.2 | | 97.79 | −0.29 | −0.36 | −1.50 | 0.1235 | 0.0792 | V |

40

**Table 3.** Results summary for quality indicators before and after stress obtained after analyzing the samples by gel filtration and UV spectrophotometry. (continued)

| No. | Composition of excipients, mg/ml | | | | | | | | Monomer content SE HPLC, % | Change in purity SE HPLC, % | | | Optic. density, 400 nm | | Selection for isoform profile analysis |
|---|---|---|---|---|---|---|---|---|---|---|---|---|---|---|---|
| | Prolgolimab | Mannitol | Trehalose d/h | Sucrose | L-Proline | Glycine | Polysorbate 80 | Poloxamer 188 | | Shake test 120 h | Freezing −18°C, thawing +25°C | Thermostress, +50°C 96 h | Shake test 120 h | Freezing −18°C, thawing +25°C | |
| 45 | 5 | | | 100 | | | 0.5 | | 97.77 | −0.55 | −0.29 | −1.02 | 0.1327 | 0.0820 | V |
| 46 | 5 | | | 100 | | | 0.8 | | 97.85 | −0.66 | −0.43 | −0.71 | 0.1230 | 0.0832 | V |
| 47 | 5 | | | 100 | | | | 0.2 | 97.80 | −0.41 | −0.30 | −0.44 | 0.1227 | 0.0778 | V |
| 43 | 5 | | | 100 | | | | 0.5 | 97.84 | −0.57 | −0.46 | −0.42 | 0.1281 | 0.0792 | V |
| 43 | 5 | | | 100 | | | | 0.8 | 97.81 | −0.38 | −0.32 | −0.69 | 0.1171 | 0.0779 | V |
| 50 | 5 | | | 10 | 28.8 | | | | 97.80 | −0.61 | −0.96 | −1.46 | 0.0885 | 0.0741 | V |
| 51 | 5 | | | 10 | 28.8 | | 0.2 | | 97.72 | −0.54 | −0.96 | −1.88 | 0.1364 | 0.0778 | V |
| 52 | 5 | | | 10 | 28.8 | | 0.5 | | 97.70 | −0.63 | −1.08 | −1.58 | 0.1601 | 0.0798 | V |
| 53 | 5 | | | 10 | 28.8 | | 0.8 | | 97.84 | −0.77 | −0.62 | −1.97 | 0.1454 | 0.0829 | V |
| 54 | 5 | | | 10 | 28.8 | | | 0.2 | 98.04 | −0.89 | −1.35 | −1.73 | 0.1391 | 0.0768 | V |
| 55 | 5 | | | 10 | 28.8 | | | 0.5 | 97.88 | −0.82 | −1.33 | −1.67 | 0.1321 | 0.0782 | V |
| 56 | 5 | | | 10 | 28.8 | | | 0.8 | 97.82 | −0.73 | −1.27 | −1.49 | 0.1302 | 0.0766 | V |
| 57 | 5 | | | 67 | | 7.5 | | | 97.87 | −0.61 | −0.48 | −0.15 | 0.0848 | 0.0738 | V |
| 58 | 5 | | | 67 | | 7.5 | 0.2 | | 97.87 | −0.84 | −0.44 | −0.89 | 0.1009 | 0.0778 | V |
| 53 | 5 | | | 67 | | 7.5 | 0.5 | | 97.65 | −0.59 | −0.29 | −0.29 | 0.1129 | 0.0804 | V |
| 60 | 5 | | | 67 | | 7.5 | 0.8 | | 97.74 | −0.61 | −0.35 | −0.76 | 0.1121 | 0.0830 | V |

EP 4 019 047 A1

41

Table 3. Results summary for quality indicators before and after stress obtained after analyzing the samples by gel filtration and UV spectrophotometry. (continued)

| No. | Composition of excipients, mg/ml | | | | | | | | Monomer content SE HPLC, % | Change in purity SE HPLC, % | | | Optic. density, 400 nm | | Selection for iso-form pro-file analysis |
|---|---|---|---|---|---|---|---|---|---|---|---|---|---|---|---|
| | Prolgolimab | Mannitol | Trehalose d/h | Sucrose | L-Proline | Glycine | Polysorbate 80 | Poloxamer 188 | | Shake test 120 h | Freezing -18°C, thawing +25°C | Ther-mostress, +50°C 96 h | Shake test 120 h | Freezing - 18°C, thawing +25°C | |
| 61 | 5 | | | 67 | | 7.5 | | 0.2 | 97.63 | -0.42 | -0.23 | 0.16 | 0.0982 | 0.0753 | V |
| 62 | 5 | | | 67 | | 7.5 | | 0.5 | 97.72 | -0.52 | -0.22 | -0.13 | 0.0945 | 0.0776 | V |
| 63 | 5 | | | 67 | | 7.5 | | 0.8 | 97.75 | -0.54 | -0.21 | 0.02 | 0.0899 | 0.0763 | V |

- the best result          - the worst result          - Initial control or average result

Table 4. Results summary for study of acid-base profile of samples before and after stress

| No. | Composition of excipients in 20 mM histidine buffer solution with pH 5.5, mg/ml | | | | | | | | Total change in acid-base profile in absolute value**, % | | | Selection for lyophilisation |
| | Prolgolimab | Mannitol | Trehalose d/h | Sucrose | L-Proline | Glycine | Polysorbate 80 | Poloxamer 188 | Shake test 120 h | Freezing –18°C, thawing +25°C | Thermostress, +50°C 96 h | |
|---|---|---|---|---|---|---|---|---|---|---|---|---|
| 22 | 5 | | 100 | | | | | | 5.64 | 0.68 | 33.34 | V |
| 23 | 5 | | 100 | | | | 0.2 | | 8.81 | 6.43 | 36.39 | |
| 24 | 5 | | 100 | | | | 0.5 | | 9.40 | 1.02 | 34.67 | |
| 25 | 5 | | 100 | | | | 0.8 | | 9.36 | 4.92 | 36.32 | |
| 26 | 5 | | 100 | | | | | 0.2 | 9.95 | 7.82 | 36.61 | |
| 27 | 5 | | 100 | | | | | 0.5 | 4.88 | 0.96 | 33.36 | V |
| 28 | 5 | | 100 | | | | | 0.8 | 8.98 | 6.94 | 36.14 | |
| 36 | 5 | | 67 | | | 7.5 | | | 6.50 | 1.38 | 32.62 | V |
| 37 | 5 | | 67 | | | 7.5 | 0.2 | | 5.34 | 1.60 | 32.65 | |
| 38 | 5 | | 67 | | | 7.5 | 0.5 | | 7.12 | 3.29 | 36.07 | |
| 39 | 5 | | 67 | | | 7.5 | 0.5 | | 9.00 | 1.90 | 36.83 | |
| 40 | 5 | | 67 | | | 7.5 | | 0.2 | 6.72 | 1.15 | 33.10 | |
| 41 | 5 | | 67 | | | 7.5 | | 0.5 | 6.96 | 1.71 | 34.43 | V |
| 42 | 5 | | 67 | | | 7.5 | | 0.8 | 10.61 | 2.95 | 35.86 | |
| 43 | 5 | | | 100 | | | | | 6.87 | 0.40 | 36.38 | V |
| 44 | 5 | | | 100 | | | 0.2 | | 10.22 | 2.67 | 41.24 | |
| 45 | 5 | | | 100 | | | 0.5 | | 8.54 | 1.72 | 41.14 | |
| 46 | 5 | | | 100 | | | 0.5 | | 6.73 | 0.38 | 33.02 | |
| 47 | 5 | | | 100 | | | | 0.2 | 9.20 | 2.84 | 37.24 | |
| 48 | 5 | | | 100 | | | | 0.5 | 7.11 | 0.40 | 34.88 | |
| 49 | 5 | | | 100 | | | | 0.8 | 9.69 | 3.48 | 36.52 | |
| 50 | 5 | | | 10 | 28.8 | | | | 6.66 | 1.33 | 35.62 | |
| 57 | 5 | | | 67 | | 7.5 | | | 5.45 | 0.98 | 33.56 | V |
| 58 | 5 | | | 67 | | 7.5 | 0.2 | | 10.78 | 1.68 | 34.48 | |
| 59 | 5 | | | 67 | | 7.5 | 0.5 | | 6.69 | 0.78 | 34.70 | |
| 60 | 5 | | | 67 | | 7.5 | 0.8 | | 11.31 | 1.68 | 33.52 | |
| 61 | 5 | | | 67 | | 7.5 | | 0.2 | 12.06 | 1.57 | 34.34 | |
| 62 | 5 | | | 67 | | 7.5 | | 0.5 | 6.00 | 0.95 | 34.74 | V |
| 63 | 5 | | | 67 | | 7.5 | | 0.8 | 12.06 | 3.24 | 34.92 | |

\* The change was calculated by the following formula: Δ = (fraction content after stress — fraction content before stress)
\*\* The absolute change was calculated by the following formula: Δ = acid fraction content before stress — acid fraction content after stress|+|base fraction content before stress — base fraction content after stress|+|dominant fraction content before stress — dominant fraction content after stress|

▮ – the best result ▦ – the worst result ☐ – Initial control or average result

Table 3 shows the negative effect of mannitol on thermal and colloidal stability of monoclonal anti-PD-1 antibody in 20 mM histidine buffer solution with pH 5.5: during thermostress for 96 h, an increase in impurities by SE HPLC was from 1.24 to 3.18 %; during shake test for 120 h, the solutions exhibited visible aggregation. Formulations containing mannitol also showed negative results of stability during cryoconcentration: after one cycle of freezing-thawing, an increase in impurities by SE HPLC was from 1.05 to 4.45 %, which is much higher than that of other formulations.

Formulations containing L-proline also showed low colloidal stability as a result of visible aggregation during shake test. After testing by freezing-thawing and thermostress, an increase in impurities in formulations containing L-proline was in average more than 1% by SE HPLC.

In the course of experiment, we detected high thermal and colloidal stability of prolgolimab (absence of significant changes in quality indicators for all stresses) in formulations containing trehalose dihydrate without solubilizers, such as polysorbate 80 and Poloxamer 188.

The best stabilizing effect for monoclonal anti-PD-1 antibody in 20 mM histidine buffer solution was shown by: trehalose dihydrate, sucrose, as well as combinations thereof with glycine. These formulations can be used for a lyophilic dosage form of monoclonal antibody.

Evaluation of stability of candidate histidine-based formulations following lyophilisation

Samples which showed the best stability at the previous stage of screening were tested for possibility of production of lyophilisate to prepare an infusion solution.

To this end, solutions containing 20 mg/ml or 100 mg/ml of monoclonal anti-PD-1 antibody prolgolimab were placed in glass vials of hydrolytic class I; the vials were loosely capped with rubber slotted stoppers. The vials containing the solution were placed into a lyophilisation chamber. Lyophilisation was performed in automatic mode. The stage of freezing was performed at minus 40°C, primary drying was at $(0.10 \pm 0.03)$ mbar, during secondary drying the temperature was increased, the pressure was $(0.05 \pm 0.02)$ mbar. After the drying procedure, the pressure was dropped to a level required for vacuum - $0.76 \pm 0.03$ mbar, the vials were capped with rubber stoppers, and vacuum was then released to atmospheric pressure. The capped vials were transferred for further study. The lyophilized samples of monoclonal anti-PD-1 antibody prolgolimab were stored at 2 - 8 °C.

To prove protein stability, the samples were reconstituted after lyophilisation. Monomer content was evaluated by SE HPLC; charged form profile was analyzed using Labchip system. Also, we measured the pH value before and after lyophilisation. The results are shown in Table 5.

During lyophilisation of selected histidine-based formulations and after lyophilisate reconstitution, we observed a slight shift in pH, which is within the accuracy limits of the method. All formulations exhibited high stability in purity by SE HPLC and in charged form profile after the reconstitution. However, a significant difference in purity between the samples was demonstrated by gel electrophoresis in LabChip Caliper system. Samples which are most stable in terms of this parameter are formulations No. 22, 27, 36 containing trehalose dihydrate. Formulations containing L-proline also exhibited a significant change in charged form profile; therefore, they are not recommended for use. The possibility of lyophilisation of formulations No. 22 and 27 with a protein concentration of 100 mg/ml was demonstrated.

**Table 5.** Results summary for quality indicators of formulations based on 20 mM histidine buffer solution with pH 5.5 before and after lyophilisation

| No. | Composition of excipients, mg/ml | | | | | | | | Before lyoph. | | After lyoph. | | Δ, (absolute change) after lyophilisation | | | | | |
|---|---|---|---|---|---|---|---|---|---|---|---|---|---|---|---|---|---|---|
| | | | | | | | | | | SE HPLC | | SE HPLC | | SE HPLC | Change in fraction content, LabChip | | | |
| | Prolgolimab | Mannitol | Trehalose d/h | Sucrose | L-Proline | Glycine | Polysorbate 80 | Poloxamer 188 | pH | purity, % | pH | purity, % | pH | purity, % | acid fractions*, % | dominant fractions*, % | base fractions*, % | Total change in acid-base profile in absolute value**, % |
| Protein concentration in solution 20 mg/ml | | | | | | | | | | | | | | | | | | |
| 22 | 20 | | 100 | | | | | | 5.40 | 98.87 | 5.66 | 99.00 | −0.06 | 0.13 | +0.24 | −0.23 | −0.03 | 0.53 |
| 27 | 20 | | 100 | | | | | 0.5 | 5.42 | 98.86 | 5.64 | 98.97 | −0.02 | 0.10 | +2.55 | −0.47 | −2.07 | 1.09 |
| 36 | 20 | | 67 | | | 7.5 | | | 5.60 | 98.89 | 5.65 | 98.96 | −0.05 | 0.07 | +1.32 | −1.19 | −0.14 | 2.65 |
| 41 | 20 | | 67 | | | 7.5 | | 0.5 | 5.45 | 98.89 | 5.64 | 98.98 | −0.19 | 0.09 | +1.28 | +0.30 | −0.85 | 2.43 |
| 43 | 20 | | | 100 | | | | | 5.38 | 98.87 | 5.65 | 99.02 | −0.27 | 0.15 | +2.03 | −1.71 | −0.31 | 4.06 |
| 57 | 20 | | | 67 | | 7.5 | | | 5.42 | 98.88 | 5.62 | 99.02 | −0.20 | 0.14 | −4.11 | +3.06 | +1.05 | 8.22 |
| 62 | 20 | | | 67 | | 7.5 | | 0.5 | 5.40 | 98.87 | 5.73 | 98.99 | −0.33 | 0.12 | +2.39 | −2.38 | 0 | 4.77 |
| 8 | 20 | 5 | | | 28.8 | | | | 5.71 | 98.87 | 5.70 | 98.48 | 0.01 | −0.39 | +5.12 | −3.88 | −1.23 | 10.23 |
| 29 | 20 | | 10 | | 28.8 | | | | 5.43 | 98.88 | 5.90 | 98.95 | −0.47 | 0.07 | +6.67 | −2.75 | −3.92 | 13.34 |
| 50 | 20 | | | 10 | 28.8 | | | | 5.50 | 98.89 | 5.76 | 98.94 | −0.26 | 0.05 | +1.74 | +0.38 | −2.12 | 4.24 |
| Protein concentration in solution 100 mg/ml | | | | | | | | | | | | | | | | | | |
| 22 | 100 | | 100 | | | | | | 5.44 | 99.13 | 5.61 | 98.81 | −0.17 | −0.22 | +1.46 | −0.45 | −0.03 | 1.94 |
| 27 | 100 | | 100 | | | | | 0.5 | 5.44 | 99.05 | 5.75 | 98.76 | −0.31 | −0.16 | +1.47 | −0.80 | −0.68 | 2.95 |

\* The change was calculated by the following formula Δ = (fraction content after stress − fraction content before stress)
\*\* The absolute change was calculated by the following formula: Δ = acid fraction content before stress − acid fraction content after stress|+|base fraction content before stress − base fraction content after stress|+|dominant fraction content before stress − dominant fraction content after stress|

▮ - the best result ▨ - the worst result ▯ - Initial control/average result

EP 4 019 047 A1

Preparation of a highly concentrated form and confirmation of stability under accelerated aging

Based on the results of screening in liquid and lyophilic dosage forms, the following formulations were selected for accelerated study of stability:

| Prolgolimab | 20 - 150 mg/ml | Prolgolimab | 20 - 150 mg/ml |
|---|---|---|---|
| L-histidine | 0.92 mg/ml | L-histidine | 0.92 mg/ml |
| L-histidine h/c m/h | 2.96 mg/ml | L-histidine h/c m/h | 2.96 mg/ml |
| Trehalose dihydrate | 100 mg/ml | Trehalose dihydrate | 80 mg/ml |
| Water for injections | up to 1 ml | Water for injections | up to 1 ml |

Trehalose dihydrate content was reduced to equilibrate the level of osmolality of solutions containing monoclonal anti-PD-1 antibody at increased concentration to physiological level (about 300 mOsm/kg), as well as to reduce the viscosity of solutions to a level less than 100 cP. Samples stability was confirmed by accelerated aging at +37°C; the test was performed by SE HPLC, IE HPLC and VEP; also, the relative specific activity of prolgolimab was determined. The results are shown in Tables 6 and 7.

**Table 6.** Results of stability analysis at 37 °C.

| Composition | Protein concentration | Parameter | Initial control | 2 weeks | 4 weeks |
|---|---|---|---|---|---|
| Prolgolimab 20 - 150 mg/ml L-histidine 0.92 mg/ml L-histidine h/c m/h 2.96 mg/ml Trehalose dihydrate 100 mg/ml | 20 mg/ml | Total protein, mg/ml | 19.9 | 20.1 | 20.0 |
| | 100 mg/ml | | 101.1 | 100.6 | 101.9 |
| | 150 mg/ml | | 149.8 | 151.3 | 150.4 |
| | 20 mg/ml | pH | 5.5 | 5.5 | 5.5 |
| | 100 mg/ml | | 5.6 | 5.6 | 5.6 |
| | 150 mg/ml | | 5.6 | 5.6 | 5.7 |
| | 20 mg/ml | Viscosity, cP | 8 | - | - |
| | 100 mg/ml | | 40 | - | - |
| | 150 mg/ml | | 122 | - | - |
| | Purity SE HPLC | | | | |
| | 20 mg/ml | Aggregate content, % | 0.40 | 0.67 | 0.81 |
| | | Monomer content, % | 99.11 | 98.65 | 98.32 |
| | 100 mg/ml | Aggregate content, % | 0.57 | 0.97 | 1.23 |
| | | Monomer content, % | 99.22 | 98.39 | 97.81 |
| | 150 mg/ml | Aggregate content, % | 0.51 | 1.11 | 1.75 |
| | | Monomer content, % | 99.19 | 97.92 | 97.09 |
| | 20 mg/ml | VEP under red. conditions Monomer content, % | 95.33 | 94.87 | 94.06 |
| | 100 mg/ml | | 95.27 | 94.79 | 93.93 |
| | 150 mg/ml | | 95.40 | 94.61 | 93.90 |
| | 20 mg/ml | VEP under non-red. conditions Monomer content, % | 93.36 | 91.96 | 91.14 |
| | 100 mg/ml | | 93.28 | 91.79 | 90.89 |
| | 150 mg/ml | | 93.21 | 91.64 | 90.52 |
| | Charged form profile (IE HPLC) | | | | |
| | 20 mg/ml | Acid fraction, % | 16.72 | 25.22 | 31.03 |
| | | Dominant fraction, % | 50.87 | 54.07 | 56.11 |
| | | Base fraction, % | 32.41 | 20.71 | 12.86 |
| | 100 mg/ml | Acid fraction, % | 17.00 | 25.95 | 32.04 |
| | | Dominant fraction, % | 50.66 | 53.91 | 55.86 |
| | | Base fraction, % | 32.34 | 20.14 | 12.10 |
| | 150 mg/ml | Acid fraction, % | 16.95 | 26.09 | 32.19 |
| | | Dominant fraction, % | 49.87 | 54.36 | 56.11 |
| | | Base fraction, % | 33.18 | 19.55 | 11.70 |
| | 20 mg/ml | Relative specific activity, % | 105 | 97 | 110 |
| | 100 mg/ml | | 101 | 104 | 97 |
| | 150 mg/ml | | 97 | 100 | 105 |

Table 7. Results of stability analysis at 37 °C.

| Composition | Protein concentration | Parameter | Initial control | 2 weeks | 4 weeks |
|---|---|---|---|---|---|
| Prolgolimab 20 - 150 mg/ml L-histidine 0.92 mg/ml L-histidine h/c m/h 2.96 mg/ml Trehalose dihydrate 80 mg/ml | 20 mg/ml | Total protein, mg/ml | 20.4 | 20.2 | 20.8 |
| | 100 mg/ml | | 100.3 | 102.8 | 101.2 |
| | 150 mg/ml | | 152.2 | 150.7 | 153.0 |
| | 20 mg/ml | pH | 5.4 | 5.5 | 5.6 |
| | 100 mg/ml | | 5.5 | 5.6 | 5.6 |
| | 150 mg/ml | | 5.6 | 5.6 | 5.7 |
| | 20 mg/ml | Viscosity, cP | 5 | - | - |
| | 100 mg/ml | | 28 | - | - |
| | 150 mg/ml | | 96 | - | - |
| | Purity SE HPLC | | | | |
| | 20 mg/ml | Aggregate content, % | 0.41 | 0.46 | 0.73 |
| | | Monomer content, % | 99.09 | 98.72 | 98.30 |
| | 100 mg/ml | Aggregate content, % | 0.55 | 0.98 | 1.10 |
| | | Monomer content, % | 99.26 | 98.60 | 97.79 |
| | 150 mg/ml | Aggregate content, % | 0.60 | 0.92 | 1.26 |
| | | Monomer content, % | 99.27 | 98.16 | 97.55 |
| | 20 mg/ml | VEP under red. conditions Monomer content, % | 95.48 | 94.79 | 94.15 |
| | 100 mg/ml | | 95.59 | 94.52 | 93.88 |
| | 150 mg/ml | | 95.26 | 94.40 | 93.76 |
| | 20 mg/ml | VEP under non-red. conditions Monomer content, % | 93.18 | 91.82 | 91.10 |
| | 100 mg/ml | | 93.04 | 91.62 | 90.77 |
| | 150 mg/ml | | 93.00 | 91.25 | 90.28 |
| | Charged form profile (IE HPLC) | | | | |
| | 20 mg/ml | Acid fraction, % | 15.38 | 24.11 | 30.66 |
| | | Dominant fraction, % | 51.81 | 55.09 | 56.31 |
| | | Base fraction, % | 32.81 | 20.80 | 13.03 |
| | 100 mg/ml | Acid fraction, % | 16.66 | 24.91 | 31.64 |
| | | Dominant fraction, % | 50.65 | 53.05 | 56.17 |
| | | Base fraction, % | 32.69 | 22.04 | 12.19 |

(continued)

| Composition | Protein concentration | Parameter | Initial control | 2 weeks | 4 weeks |
|---|---|---|---|---|---|
| | 150 mg/ml | Acid fraction, % | 17.17 | 25.36 | 32.52 |
| | | Dominant fraction, % | 50.34 | 54.06 | 57.81 |
| | | Base fraction, % | 32.49 | 20.58 | 9.67 |
| | 20 mg/ml | Relative specific activity, % | 100 | 103 | 94 |
| | 100 mg/ml | | 89 | 94 | 105 |
| | 150 mg/ml | | 91 | 99 | 108 |

Example 17. Selection of acetate-based pharmaceutical composition

Test formulations

For screening for a stable pharmaceutical composition based on 20 mM acetate buffer solution with pH 5.0, the following excipients were selected: mannitol, trehalose dihydrate, sucrose (osmotic agents), L-proline (osmotic agent and stabilizer), glycine (osmotic agent and stabilizer), polysorbate 80 and Poloxamer P188 (solubilizers). All test solutions are isotonic.

Test formulations (per ml) .

| No. | Prolgolimab | Osmotic agents | | | | | Stabilizers / Solubilizers | | 20 mM Acet pH 5.5 |
| | | Mannitol | Trehalose d/h | Sucrose | L-Proline | Glycine | Polysorbate 80 | Poloxamer 188 | |
|---|---|---|---|---|---|---|---|---|---|
| 1 | 5 | 50 | | | | | | | to 1 ml |
| 2 | 5 | 50 | | | | | 0.5 | | to 1 ml |
| 3 | 5 | 50 | | | | | | 0.5 | to 1 ml |
| 4 | 5 | 5 | | | 28.8 | | | | to 1 ml |
| 5 | 5 | 5 | | | 28.8 | | 0.5 | | to 1 ml |
| 6 | 5 | 5 | | | 28.8 | | | 0.5 | to 1 ml |
| 7 | 5 | 33.5 | | | | 7.5 | | | to 1 ml |
| 8 | 5 | 33.5 | | | | 7.5 | 0.5 | | to 1 ml |
| 9 | 5 | 33.5 | | | | 7.5 | | 0.5 | to 1 ml |
| 10 | 5 | | 100 | | | | | | to 1 ml |
| 11 | 5 | | 100 | | | | 0.5 | | to 1 ml |
| 12 | 5 | | 100 | | | | | 0.5 | to 1 ml |
| 13 | 5 | | 10 | | 28.8 | | | | to 1 ml |
| 14 | 5 | | 10 | | 28.8 | | 0.5 | | to 1 ml |
| 15 | 5 | | 10 | | 28.8 | | | 0.5 | to 1 ml |
| 16 | 5 | | 67 | | | 7.5 | | | to 1 ml |
| 17 | 5 | | 67 | | | 7.5 | 0.5 | | to 1 ml |
| 18 | 5 | | 67 | | | 7.5 | | 0.5 | to 1 ml |
| 19 | 5 | | | 100 | | | | | to 1 ml |
| 20 | 5 | | | 100 | | | 0.5 | | to 1 ml |
| 21 | 5 | | | 100 | | | | 0.5 | to 1 ml |
| 22 | 5 | | | 67 | | 7.5 | | | to 1 ml |
| 23 | 5 | | | 67 | | 7.5 | 0.5 | | to 1 ml |
| 24 | 5 | | | 67 | | 7.5 | | 0.5 | to 1 ml |

Sample stability was evaluated by: thermostress at 50°C for 72 hours, shake test for 120 h at 800 rpm, and one-time freezing at minus 16 - 20 °C and thawing at +25 ± 1°C. Turbidity of solutions was evaluated by spectrophotometry at 400 nm. Samples homogeneity was determined by SE HPLC and electrophoresis using Labchip system. Charged form profile was analyzed using Labchip system.

Table 8. Sample gel filtration/UV spectrophotometry results

| No. | Composition of excipients, mg/ml | | | | | | | | Monomer content SE HPLC, % | Change in purity SE HPLC, % | | | Optic. density, 400 nm | | Selection for acid/base profile analysis |
|---|---|---|---|---|---|---|---|---|---|---|---|---|---|---|---|
| | Prolgolimab | Mannitol | Trehalose d/h | Sucrose | L-Proline | Glycine | Polysorbate 80 | Kolliphor 188 | | Shake test 120 h | Freezing -18°C, Thawing +25°C | Thermostress +50°C, 96 h | Shake test 120 h | Freezing -18°C, Thawing +25°C | |
| 1 | 5 | 50 | | | | | | | 97.71 | -0.23 | -1.63 | -0.48 | 0.0749 | 0.0778 | |
| 2 | 5 | 50 | | | | | 0.5 | | 97.66 | -0.35 | -3.19 | -1.66 | 0.0749 | 0.0751 | |
| 3 | 5 | 50 | | | | | | 0.5 | 97.54 | -0.07 | -9.12 | -1.25 | 0.0688 | 0.0721 | |
| 4 | 5 | 5 | | | 28.8 | | | | 97.56 | -0.03 | -0.69 | -2.19 | 0.0809 | 0.0797 | |
| 5 | 5 | 5 | | | 28.8 | | 0.5 | | 97.74 | -0.36 | -1.16 | -2.59 | 0.0820 | 0.0804 | |
| 6 | 5 | 5 | | | 28.8 | | | 0.5 | 97.66 | -0.28 | -1.70 | -2.53 | 0.0773 | 0.0758 | |
| 7 | 5 | 33.5 | | | | 7.5 | | | 97.58 | -0.05 | -1.15 | -1.54 | 0.0745 | 0.0777 | |
| 8 | 5 | 33.5 | | | | 7.5 | 0.5 | | 97.52 | -0.21 | -2.84 | -2.08 | 0.0796 | 0.0776 | |
| 9 | 5 | 33.5 | | | | 7.5 | | 0.5 | 97.58 | -0.09 | -9.44 | -1.69 | 0.0733 | 0.0831 | |
| 10 | 5 | | 100 | | | | | | 97.63 | -0.19 | -0.44 | -0.17 | 0.0744 | 0.0767 | V |
| 11 | 5 | | 100 | | | | 0.5 | | 97.60 | -0.19 | -0.48 | -0.30 | 0.0778 | 0.0801 | V |
| 12 | 5 | | 100 | | | | | 0.5 | 97.52 | -0.10 | -0.22 | -0.10 | 0.0725 | 0.0733 | V |
| 13 | 5 | | 10 | | 28.8 | | | | 97.48 | -0.17 | -0.38 | -1.64 | 0.0781 | 0.0802 | |
| 14 | 5 | | 10 | | 28.8 | | 0.5 | | 97.55 | -0.12 | -0.71 | -1.93 | 0.0797 | 0.0819 | |
| 15 | 5 | | 10 | | 28.8 | | | 0.5 | 97.64 | -0.26 | -0.98 | -1.91 | 0.0756 | 0.0772 | |
| 16 | 5 | | 67 | | | 7.5 | | | 97.71 | -0.36 | -0.46 | -0.32 | 0.0754 | 0.0765 | V |
| 17 | 5 | | 67 | | | 7.5 | 0.5 | | 97.57 | -0.31 | -0.45 | -0.63 | 0.0776 | 0.0785 | V |
| 18 | 5 | | 67 | | | 7.5 | | 0.5 | 97.68 | -0.36 | -0.85 | -0.38 | 0.0726 | 0.0732 | V |

**Table 8.** Sample gel filtration/UV spectrophotometry results (continued).

| No. | Composition of excipients, mg/ml | | | | | | | | Monomer content SE HPLC, % | Change in purity SE HPLC, % | | | Optic. density, 400 nm | | Selection for acid/base profile analysis |
|---|---|---|---|---|---|---|---|---|---|---|---|---|---|---|---|
| | Prolgolimab | Mannitol | Trehalose d/h | Sucrose | L-Proline | Glycine | Polysorbate 80 | Kolliphor 188 | | Shake test 120 h | Freezing −18°C, Thawing +25°C | Thermostress, +50°C 96h | Shake test 120 h | Freezing −18°C, Thawing +25°C | |
| 19 | 5 | | | 100 | | | | | 97.56 | −0.26 | −0.34 | −0.01 | 0.0792 | 0.0819 | V |
| 20 | 5 | | | 100 | | | 0.5 | | 97.53 | −0.27 | −0.32 | −0.05 | 0.0813 | 0.0821 | V |
| 21 | 5 | | | 100 | | | | 0.5 | 97.50 | −0.29 | −0.38 | −0.04 | 0.0783 | 0.0767 | V |
| 22 | 5 | | | 67 | | 7.5 | | | 97.50 | −0.27 | −0.35 | −0.06 | 0.0787 | 0.0807 | V |
| 23 | 5 | | | 67 | | 7.5 | 0.5 | | 97.43 | −0.18 | −0.48 | −0.22 | 0.0812 | 0.0807 | V |
| 24 | 5 | | | 67 | | 7.5 | | 0.5 | 97.50 | −0.10 | −0.27 | −0.01 | 0.0761 | 0.0761 | V |

■ – the best result    ▨ – the worst result    ☐ – initial control or average result

**Table 9.** Sample acid/base profile results.

| No. | Composition of excipients, mg/ml | | | | | | | | Total change in acid-base profile in absolute value**, % | | | Selection for lyophilisation |
|---|---|---|---|---|---|---|---|---|---|---|---|---|
| | Prolgolimab | Mannitol | Trehalose d/h | Sucrose | L-Proline | Glycine | Polysorbate 80 | Kolliphor 100 | Shake test 120 h | Freezing -18°C, thawing +25°C | Thermostress, +50°C, 96 h | |
| 10 | 5 | | 100 | | | | | | 6.16 | 1.48 | 29.28 | V |
| 11 | 5 | | 100 | | | | 0.5 | | 6.53 | 1.64 | 33.76 | V |
| 12 | 5 | | 100 | | | | | 0.5 | 5.26 | 1.91 | 33.89 | V |
| 16 | 5 | | 67 | | 7.5 | | | | 6.59 | 1.09 | 33.47 | |
| 17 | 5 | | 67 | | 7.5 | | 0.5 | | 6.81 | 0.73 | 34.77 | |
| 18 | 5 | | 67 | | 7.5 | | | 0.5 | 7.15 | 0.76 | 34.95 | |
| 19 | 5 | | | 100 | | | | | 6.38 | 1.08 | 33.48 | V |
| 20 | 5 | | | 100 | | | 0.5 | | 6.92 | 0.93 | 34.58 | |
| 21 | 5 | | | 100 | | | | 0.5 | 6.3 | 1.05 | 34.06 | V |
| 22 | 5 | | | 67 | 7.5 | | | | 5.94 | 2.46 | 34.38 | |
| 23 | 5 | | | 67 | 7.5 | | 0.5 | | 5.77 | 1.74 | 33.63 | |
| 24 | 5 | | | 67 | 7.5 | | | 0.5 | 5.96 | 1.37 | 33.60 | V |

\* The change was calculated by the following formula: Δ = (fraction content after stress — fraction content before stress)

\*\* The absolute change was calculated by the following formula: Δ = acid fraction content before stress — acid fraction content after stress|+|base fraction content before stress — base fraction content after stress|+|dominant fraction content before stress — dominant fraction content after stress|

■ - the best result    ▨ - the worst result    □ - initial control or average result

The study showed negative effect of mannitol on thermal and colloidal stability of monoclonal anti-PD-1 antibody in 20 mM acetate buffer solution with pH 5.0: during thermostress for 96 h, an increase in impurities by SE HPLC was from 0.48 to 2.59 %; during shake test for 120 h, all test solutions did not exhibit visible aggregation (see UV spectrophotometry results). Formulations containing mannitol also showed negative results of stability during cryoconcentration: after one cycle of freezing-thawing, an increase in impurities by SE HPLC was from 0.69 to 9.44 %, which is much higher than that of other formulations.

The formulations containing L-proline also showed low thermal stability in terms of purity in SE HPLC analysis: an increase in impurities after 96 h of stress was 1.64 - 1.93 %. After freezing-thawing, an increase in impurities in formulations containing L-proline was not more than the average value of the most stable formulations in the group. In the course of the experiment, solubilizers did not show any effect of on thermal and colloidal stability of protein. In formulations containing trehalose dihydrate, prolgolimab exhibited high stability in all stress experiments in the absence of surfactants, such as polysorbate 80 or Poloxamer 188.

The best stabilizing effect for monoclonal anti-PD-1 antibody in 20 mM acetate buffer solution was shown by: trehalose dihydrate, sucrose, as well as combinations thereof with glycine. These formulations can be used to prepare lyophilic dosage forms of monoclonal antibody.

Determination of stability of candidate acetate-based formulations following lyophilisation

Samples which showed the best stability at the previous stage of screening were tested for possibility of production of lyophilisate to prepare an infusion solution.

To this end, solutions containing 20 mg/ml or 100 mg/ml of monoclonal anti-PD-1 antibody were placed in glass vials of hydrolytic class I; the vials were loosely capped with rubber slotted stoppers. The vials containing the solution were placed into a lyophilisation chamber. Lyophilisation was performed in automatic mode. The stage of freezing was performed at minus 40°C, primary drying was at (0.10 ± 0.03) mbar, during secondary drying the temperature was increased, the pressure was (0.05 ± 0.02) mbar. After the drying procedure, the pressure was dropped to a level required for vacuum - 0.76 ± 0.03 mbar, the vials were capped with rubber stoppers, and vacuum was then released to atmospheric pressure. The capped vials were transferred for further study. The lyophilized samples of monoclonal anti-PD-1 antibody were stored at 2 - 8 °C.

To prove protein stability, the samples were reconstituted after lyophilisation. Monomer content was evaluated by SE HPLC; charged form profile was analyzed using Labchip system. Also, we measured the pH value before and after lyophilisation. The results are shown in Table 8.

During lyophilisation of selected acetate-based formulations and after lyophilisate reconstitution, we detected a significant shift in pH within the range of 0.26 - 0.45. Such a shift may be caused by loss of acetate ions during lyophilisation. Thus, acetate-based formulations may be recommended to lyophilisation provided the above event is observed.

All formulations exhibited high stability in purity by SE HPLC and in charged form profile after the reconstitution. The study demonstrated the possibility of lyophilisation of formulations No. 10 and 12 with a protein concentration of 100 mg/ml.

**Table 10.** Results of stability of formulations based on 20 mM acetate buffer solution with pH 5.0 after lyophilisation.

| No. | Composition of excipients, mg/ml | | | | | | | | Before lyoph. | | After lyoph. | | Δ, (absolute change) after lyophilisation | | | | | |
|---|---|---|---|---|---|---|---|---|---|---|---|---|---|---|---|---|---|---|
| | | | | | | | | | | SE HPLC | | SE HPLC | | SE HPLC | Change in fraction content, LabChip | | | |
| | Prolgolimab | Mannitol | Trehalose d/h | Sucrose | L-Proline | Glycine | Polysorbate 80 | Poloxamer 188 | pH | Purity, % | pH | Purity, % | pH | Δ purity, % | Δ acid fractions *, % | Δ dominant fractions*, % | Δ base fractions*, % | Total change in acid-base profile in absolute value**, % |
| Protein concentration in solution 20 mg/ml | | | | | | | | | | | | | | | | | | |
| 10 | 20 | | 100 | | | | | | 4.99 | 98.91 | 5.44 | 99.06 | -0.45 | 0.15 | 1.66 | 0.90 | -2.56 | 4.12 |
| 10' | 20 | | 100 | | | | 0.2 | | 5.02 | 98.91 | 5.38 | 98.97 | -0.36 | 0.06 | 2.07 | -1.59 | -0.48 | 4.14 |
| 11 | 20 | | 100 | | | | 0.5 | | 5.00 | 98.91 | 5.35 | 98.97 | -0.35 | 0.06 | 3.04 | -2.68 | -0.35 | 4.07 |
| 10" | 20 | | 100 | | | | 0.8 | | 5.02 | 98.91 | 5.39 | 98.92 | -0.37 | 0.02 | 1.91 | -1.73 | -0.18 | 6.07 |
| 12' | 20 | | 100 | | | | | 0.2 | 5.05 | 98.93 | 5.34 | 98.96 | -0.29 | 0.03 | 1.44 | -0.42 | -1.01 | 2.87 |
| 12 | 20 | | 100 | | | | | 0.5 | 5.02 | 99.01 | 5.28 | 98.94 | -0.26 | -0.07 | 1.80 | -0.86 | -0.94 | 3.60 |
| 12" | 20 | | 100 | | | | | 0.8 | 5.01 | 99.19 | 5.43 | 98.96 | -0.42 | -0.23 | 1.91 | -1.02 | -0.88 | 3.81 |
| 19 | 20 | | | 100 | | | | | 5.00 | 99.19 | 5.37 | 98.98 | -0.37 | -0.21 | 2.37 | -1.90 | -0.47 | 4.74 |
| 21 | 20 | | | 100 | | | | 0.5 | 5.01 | 99.20 | 5.33 | 98.95 | -0.32 | -0.25 | 2.45 | -1.56 | -0.89 | 4.90 |
| 24 | 20 | | | 67 | | 7.5 | | 0.5 | 5.04 | 99.22 | 5.35 | 99.02 | -0.31 | -0.20 | 3.09 | -2.14 | -0.95 | 6.18 |
| Protein concentration in solution 100 mg/ml | | | | | | | | | | | | | | | | | | |
| 10 | 100 | | 100 | | | | | | 5.10 | 99.10 | 5.55 | 98.76 | -0.45 | -0.34 | 2.17 | -1.44 | -0.73 | 4.34 |
| 12 | 100 | | 100 | | | | | 0.2 | 5.06 | 99.05 | 5.41 | 98.76 | -0.35 | -0.35 | 1.04 | -1.68 | -1.36 | 4.08 |

\* The change was calculated by the following formula: Δ = (fraction content after stress — fraction content before stress)
\*\* The absolute change was calculated by the following formula: Δ = acid fraction content before stress — acid fraction content after stress|+|base fraction content before stress — base fraction content after stress|+|dominant fraction content before stress — dominant fraction content after stress|

■ - the best result          ▨ - the worst result          □ - initial control or average result

EP 4 019 047 A1

Preparation of a highly concentrated form and confirmation of stability under accelerated aging

Based on the results of screening in liquid and lyophilic dosage forms, the following formulations were selected for accelerated study of stability:

| Prolgolimab | 20 - 150 mg/ml | Prolgolimab | 20 - 150 mg/ml |
|---|---|---|---|
| Sodium acetate t/h | 1.742 mg/ml | Sodium acetate t/h | 1.742 mg/ml |
| Acetic acid qlac. | to pH 5. 0 | Acetic acid qlac. | to pH 5.0 |
| Trehalose dihydrate | 100 mg/ml | Trehalose dihydrate | 80 mg/ml |
| Water for injections | up to 1 ml | Water for injections | up to 1 ml |

Trehalose dihydrate content was reduced to equilibrate the level of osmolality of solutions containing monoclonal anti-PD-1 antibody at increased concentration to physiological level (about 300 mOsm/kg), as well as to reduce the viscosity of solutions to a level less than 100 cP. Samples were subjected to aging at +37°C and analyzed by SE HPLC, IE HPLC and VEP; also, the relative specific activity of protein was determined. The results are shown in Tables 9 and 10.

Table 11. Results of stability analysis at 37 °C.

| Composition | Protein concentration | Parameter | Initial control | 2 weeks | 4 weeks |
|---|---|---|---|---|---|
| Prolgolimab 20 - 150 mg/ml Sodium acetate t/h 1.742 mg/ml Acetic acid glac. to pH 5.0 Trehalose dihydrate 100 mg/ml | 20 mg/ml | Total protein, mg/ml | 21.0 | 20.85 | 20.89 |
| | 100 mg/ml | | 102.2 | 102.9 | 100.3 |
| | 150 mg/ml | | 152.1 | 150.0 | 151.6 |
| | 20 mg/ml | pH | 5.2 | 5.1 | 5.2 |
| | 100 mg/ml | | 5.3 | 5.3 | 5.3 |
| | 150 mg/ml | | 5.4 | 5.4 | 5.4 |
| | 20 mg/ml | Viscosity, cP | 6 | - | - |
| | 100 mg/ml | | 33 | - | - |
| | 150 mg/ml | | 110 | - | - |
| | Purity SE HPLC | | | | |
| | 20 mg/ml | Aggregate content, % | 0.34 | 0.41 | 0.68 |
| | | Monomer content, % | 99.17 | 98.85 | 98.39 |
| | 100 mg/ml | Aggregate content, % | 0.57 | 0.89 | 1.03 |
| | | Monomer content, % | 99.25 | 98.64 | 97.88 |
| | 150 mg/ml | Aggregate content, % | 0.64 | 0.98 | 1.35 |
| | | Monomer content, % | 99.20 | 98.08 | 97.64 |
| | 20 mg/ml | VEP under red. condtions Monomer content, % | 95.45 | 94.23 | 94.01 |
| | 100 mg/ml | | 95.29 | 94.60 | 94.12 |
| | 150 mg/ml | | 95.60 | 94.15 | 93.79 |
| | 20 mg/ml | VEP under non-red. conditions Monomer content, % | 93.05 | 91.40 | 91.14 |
| | 100 mg/ml | | 93.22 | 91.05 | 90.95 |
| | 150 mg/ml | | 93.79 | 90.87 | 90.22 |
| | Charged form profile (IE HPLC) | | | | |

(continued)

| Composition | Protein concentration | Parameter | Initial control | 2 weeks | 4 weeks |
|---|---|---|---|---|---|
| | 20 mg/ml | Acid fraction, % | 15.63 | 24.15 | 29.32 |
| | | Dominant fraction, % | 50.81 | 55.96 | 58.03 |
| | | Base fraction, % | 33.56 | 19.89 | 12.65 |
| | 100 mg/ml | Acid fraction, % | 16.02 | 23.76 | 30.44 |
| | | Dominant fraction, % | 51.01 | 55.33 | 57.57 |
| | | Base fraction, % | 32.97 | 20.91 | 11.99 |
| | 150 mg/ml | Acid fraction, % | 16.31 | 24.48 | 33.23 |
| | | Dominant fraction, % | 50.99 | 54.07 | 57.51 |
| | | Base fraction, % | 32.70 | 21.45 | 9.26 |
| | 20 mg/ml | Relative specific activity, % | 89 | 93 | 99 |
| | 100 mg/ml | | 107 | 112 | 97 |
| | 150 mg/ml | | 92 | 106 | 103 |

**Table 12.** Results of stability analysis at 37 °C.

| Composition | Protein concentration | Parameter | Initial control | 2 weeks | 4 weeks |
|---|---|---|---|---|---|
| Prolgolimab 20 - 150 mg/ml Sodium acetate t/h 1.742 mg/ml Acetic acid glac. to pH 5.0 Trehalose dihydrate 80 mg/ml | 20 mg/ml | Total protein, mg/ml | 19.9 | 20.4 | 20.5 |
| | 100 mg/ml | | 103.5 | 102.1 | 102.0 |
| | 150 mg/ml | | 150.2 | 153.8 | 153.0 |
| | 20 mg/ml | pH | 5.1 | 5.2 | 5.2 |
| | 100 mg/ml | | 5.3 | 5.2 | 5.3 |
| | 150 mg/ml | | 5.4 | 5.4 | 5.4 |
| | 20 mg/ml | Viscosity, cP | 5 | - | - |
| | 100 mg/ml | | 26 | - | - |
| | 150 mg/ml | | 93 | - | - |
| | Purity SE HPLC | | | | |
| | 20 mg/ml | Aggregate content, % | 0.49 | 0.79 | 0.98 |
| | | Monomer content, % | 99.22 | 98.85 | 98.47 |
| | 100 mg/ml | Aggregate content, % | 0.52 | 1.09 | 1.23 |
| | | Monomer content, % | 99.04 | 98.36 | 97.72 |
| | 150 mg/ml | Aggregate content, % | 0.64 | 1.15 | 1.55 |
| | | Monomer content, % | 99.00 | 97.88 | 97.14 |
| | 20 mg/ml | VEP under red. conditions Monomer content, % | 95.47 | 94.70 | 94.16 |
| | 100 mg/ml | | 95.47 | 94.81 | 93.81 |
| | 150 mg/ml | | 95.47 | 94.63 | 93.94 |
| | 20 mg/ml | VEP under non-red. conditions Monomer content, % | 93.20 | 91.89 | 91.14 |
| | 100 mg/ml | | 93.20 | 91.35 | 90.16 |
| | 150 mg/ml | | 93.20 | 91.80 | 90.97 |
| | Charged form profile (IE HPLC) | | | | |
| | 20 mg/ml | Acid fraction, % | 17.70 | 23.75 | 28.29 |
| | | Dominant fraction, % | 49.87 | 54.86 | 57.31 |
| | | Base fraction, % | 32.43 | 21.39 | 14.40 |
| | 100 mg/ml | Acid fraction, % | 16.66 | 24.91 | 29.38 |
| | | Dominant fraction, % | 50.65 | 53.05 | 57.61 |
| | | Base fraction, % | 32.69 | 22.04 | 13.01 |
| | 150 mg/ml | Acid fraction, % | 16.81 | 24.11 | 31.52 |
| | | Dominant fraction, % | 50.34 | 54.09 | 57.81 |
| | | Base fraction, % | 32.85 | 21.8 | 10.67 |
| | 20 mg/ml | Relative specific activity, % | 100 | 103 | 94 |
| | 100 mg/ml | | 89 | 94 | 105 |
| | 150 mg/ml | | 91 | 99 | 108 |

Although the invention has been described with reference to preferred embodiments, it is to be understood that modifications may be resorted to as will be apparent to those skilled in the art. Such modifications and variations are to be considered within the purview and scope of the present invention.

Representative, non-limiting examples of the present invention were described above in detail. This detailed description is merely intended to teach a person of skill in the art further details for practicing preferred aspects of the present teachings and is not intended to limit the scope of the invention. Furthermore, each of the additional features and teachings disclosed above and below may be utilized separately or in conjunction with other features and teachings.

Moreover, combinations of features and steps disclosed in the above detailed description, as well as in the experimental examples, may not be necessary to practice the invention in the broadest sense, and are instead taught merely to particularly describe specific examples of the present invention. Furthermore, features of the above-described specific examples, as well as the corresponding independent and dependent claims below, may be combined in ways that are not specifically and explicitly enumerated in order to provide additional useful embodiments of the present invention.

Provided are further examples of studies using various aqueous pharmaceutical compositions comprising anti-PD-1 antibody prolgolimab for treating malignant neoplasms, such as melanoma, including inoperable or metastatic melanoma, early stages of melanoma before and after definitive treatment; lung cancer, non-small cell lung cancer (NSCLC), including inoperable or metastatic non-small cell lung cancer. The exemplary pharmaceutical compositions used for these studies are characterized by the following compositions below (Table 12.1).

Table 12.1

| I. Composition per 1 ml: | |
| --- | --- |
| Prolgolimab | 20.0 mg |
| Sodium acetate trihydrate | 1.742 mg |
| Trehalose dihydrate | 100 mg |
| Acetic acid glac. | to pH 5.0 |
| Water for injections | up to 1 ml. |
| II. Composition per 1 ml: | |
| Prolgolimab | 20.0 mg |
| Trehalose dihydrate | 100 mg |
| L-histidine | 0.92 mg |
| L-histidine hydrochloride | 2.96 mg |
| Water for injections | up to 1 ml. |

Main information on clinical development of test product

The pharmacokinetics, pharmacodynamics, safety and immunogenicity of BCD-100 (prolgolimab) as monotherapy following intravenous administration were studied in phase I dose escalation clinical trial. The study demonstrated safety and advantages of the test product in patients with common forms of malignant neoplasms of various localizations (melanoma, NSCLC); based on the results of the study, two BCD-100 administration regimens were selected for further clinical development: 3 mg/kg i/v once in 3 weeks and 1 mg/kg i/v once in 2 weeks.

The aim of trial No. BCD-100-2/MIRACULUM of the use of BCD-100 in patients with unresectable or metastatic melanoma was to evaluate the pharmacokinetics, efficacy, safety and immunogenicity of two dosage regimens as monotherapy: 3 mg/kg i/v once in 3 weeks and 1 mg/kg i/v once in 2 weeks. The primary endpoint (ORR) was assessed based on the results of an interim analysis after 6 months. BCD-100 demonstrated a favorable safety profile in all dosage regimens. The both dosage regimens demonstrated efficacy that is about 60% DC and 30% ORR in patients with unresectable or metastatic melanoma.

Example 18. Use of prolgolimab (BCD-100) for treating patients with common forms of malignant neoplasms of various localizations, phase I trial, BCD-100-1

Trial design

Trial No. BCD-100-1 investigated pharmacokinetics and tolerance of BCD-100 (prolgolimab). Trial No. BCD-100-1 (NCT03050047) was a phase I multicenter open-label trial in patients with solid tumors, which was conducted within the territory of the Russian Federation. The primary task of the trial was to evaluate pharmacokinetics and clinical pharmacological parameters of BCD-100. The main characteristics of BCD-100-1 trial are given in Table 13.

**Table 13.** Characteristics of BCD-100-1 trial

| Name Trial code (country) | Trial objec-tives | Patients | Design / duration | Therapy | Endpoints |
|---|---|---|---|---|---|
| Multicenter open-label single-arm multi-cohort trial of pharmacokinetic and pharmacodynamic properties, safety and immunogenicity of BCD-100 product (JSC BIOCAD, Russia) (phase I) in patients with common forms of malignant neoplasms of various localizations BCD-100-1 Phase I (Russian Federation) | PK, PD, tolerance, safety, pilot efficacy and immunogenicity Study of pharmacokinetic, pharmacodynamic properties, safety and immunogenicity of BCD-100 product (prolgolimab) following intravenous administration of increasing doses as monotherapy | 15 patients with various types of inoperable malignant neoplasms: 6 patients in 1 mg/kg cohort, 6 patients in 3 mg/kg cohort, and 3 patients in 10 mg/kg cohort | Multicenter open-label multi-cohort dose escalation trial (phase I) 3 months | a BCD-100$_{AT}$ dose of 0.3 mg/kg $_{I/V}$ every 2 weeks, after 4 weeks (two administrations) dose escalation to 1 mg/kg BCD-100 at dose of 1 mg/kg i/v every 2 weeks (Q2W) for 85 days (main period of trial, 6 administrations) BCD-100 at a dose of 3 mg/kg i/v every 2 weeks (Q2W) for 85 days (main period of study, 6 administrations) BCD-100 at a dose of 10 mg/kg i/v every 2 weeks (Q2W) for 85 days (main period of study, 6 administrations) | Pharmacokinetics: AUC(D-336), AUC(D-∞), $C_{max}$, $T_{max}$, $T_\%$, $K_{el}$, Cl, $V_d$ following one-time use of the study product; $C_{min}$ during 6 uses of the study product Pharmacodynamics: % occupancy of PD-1 receptors with BCD-100 prod-uct 4 h following the first administration, 336 h following the first administration (but prior to the next dose), and before the 6th administration Immunogenicity: Detection rate: BAB, NAB Safety: Occurrence rate and severity of adverse events (AEs) associated with administration of the study product based on the assessment by the Data Monitoring and Safety Committee Efficacy: Pilot assessment of antitumour activity of BCD-100 based on CT results (RECIST 1.1 and irRC criteria) |

60

The first patient was given a starting dose of BCD-100 product (0.3 mg/kg) every two weeks. In case of absence of dose-limiting toxicity for 4 weeks, the patient was subjected to dose escalation to 1 mg/kg once in two weeks. After the first patient, the trial was continued under traditional dose escalation 3+3 design, i.e. if no DLT, a cohort of 3 patients was sequentially enrolled at the next dose level every 4 weeks. At each dose level, BCD-100 was assigned either for 85 days (about 3 months), or until DLT signs/disease progression was observed.

If DLT events at a certain dose level were observed in one patient, the next cohort of 3 patients received the product at the same level; thus, total of 6 patients started receiving this dose of BCD-100. If DLT events at a certain dose level were observed in 2 or more patients, dose escalation and product assignment to new patients were stopped.

The duration of patient follow-up following therapy with BCD-100 product was 126 days. Within this period, we monitored adverse events, performed medical examinations and blood analyses (once in 2 weeks for the first 28 days, and then once in 28 days).

Trial results

Summary of characteristics of patient population

The study enrolled 15 patients with common forms of malignant solid neoplasms of various localizations (melanoma, including choroidal melanoma, NSCLC, renal cell cancer, mesothelioma), aged 18 and older, of both genders. To qualify for enrollment, the patients must have had 0-2 ECOG score and at least one measurable target lesion according to RECIST 1.1 (excluding bone metastases). Disease characteristics in patients and distribution of patients are shown in Table 14, Table 15.

**Table 14.** Disease characteristics in patient population (BCD-100-1 trial)

| Parameter | All patients (n = 15) | | BCD-100 1 mg/kg (n = 6) | | BCD-100 3 mg/kg (n = 6) | | BCD-100 10 mg/kg (n = 3) | | p-value[1] |
|---|---|---|---|---|---|---|---|---|---|
| | n | % | n | % | n | % | n | % | |
| Diagnosis | | | | | | | | | |
| Melanoma | 9 | 60.00 | 4 | 66.67 | 3 | 50 | 2 | 66.67 | 1 |
| NSCLC | 4 | 26.67 | 1 | 16.67 | 2 | 33.33 | 1 | 33.33 | 1 |
| Renal cell cancer | 1 | 6.67 | 0 | 0 | 1 | 16.67 | 0 | 0.00 | 1 |
| Mesothelioma | 1 | 6.67 | 1 | 16.67 | 0 | 0 | 0 | 0.00 | 1 |
| Stage | | | | | | | | | |
| III | 4 | 26.67 | 2 | 33.33 | 1 | 16.67 | 1 | 33.33 | 1 |
| IV | 11 | 73.33 | 4 | 66.67 | 5 | 83.33 | 2 | 66.67 | 1 |
| Previous treatment | | | | | | | | | |
| Surgery | 10 | 66.67 | 4 | 66.67 | 4 | 66.67 | 2 | 66.67 | 1 |
| Radiotherapy | 2 | 13.33 | 1 | 16.67 | 1 | 16.67 | 0 | 0.00 | 1 |
| Other treatment[2] | 7 | 46.67 | 3 | 50.00 | 2 | 33.33 | 2 | 66.67 | 0.814 |
| Radiotherapy of inoperable condition | 10 | 66.67 | 5 | 83.33 | 3 | 50.00 | 2 | 66.67 | 0.775 |
| One line | 6 | 40.00 | 2 | 33.33 | 2 | 33.33 | 2 | 66.67 | 0.640 |
| Two or more lines | 4 | 26.67 | 3 | 50.00 | 1 | 16.67 | 0 | 0 | 0.440 |
| Duration of disease (months) | | | | | | | | | |
| Mean value | 26.01 | | 25.13 | | 20.69 | | 38.38 | | |
| Median | 17.07 | | 11.50 | | 6.23 | | 20.13 | | |
| Minimum | 0.60 | | 2.07 | | 0.60 | | 17.07 | | 0.689 |

(continued)

| Parameter | All patients | | BCD-100 1 mg/kg | | BCD-100 3 mg/kg | | BCD-100 10 mg/kg | | p-value[1] |
|---|---|---|---|---|---|---|---|---|---|
| | (n = 15) | | (n = 6) | | (n = 6) | | (n = 3) | | |
| | n | % | n | % | n | % | n | % | |
| Maximum | 91.20 | | 72.77 | | 91.20 | | 77.93 | | |
| SD | 31.01 | | 29.26 | | 35.21 | | 34.29 | | |
| Note:<br>[1] all cohorts were compared using two-tailed Fisher's exact test<br>[2] "other treatment" refers to the use of medicine such as interferon, targeted drugs, cancer vaccines | | | | | | | | | |

**Table 15.** Groups of patients (BCD-100-1 trial)

| Patient | Cohort | BCD-100 dose (mg/kg) | Qty of administrations | Further participation |
|---|---|---|---|---|
| 10-01 | 1 | 0.3→1 | 6 | According to protocol |
| 08-02 | 1 | 1 | 6 | According to protocol |
| 10-03 | 1 | 1 | 5 | Discontinued due to disease progression |
| 08-04 | 1 | 1 | 6 | According to protocol |
| 12-05 | 1 | 1 | 5 | Discontinued due to disease progression |
| 12-06 | 1 | 1 | 6 | According to protocol |
| 12-07 | 2 | 3 | 6 | According to protocol |
| 12-08 | 2 | 3 | 6 | According to protocol |
| 13-09 | 2 | 3 | 6 | According to protocol |
| 13-10 | 2 | 3 | 3 | Discontinued due to SAE |
| 13-11 | 2 | 3 | 6 | According to protocol |
| 01-12 | 2 | 3 | 6 | According to protocol |
| 13-13 | 3 | 10 | 6 | According to protocol |
| 13-14 | 3 | 10 | 6 | According to protocol |
| 08-15 | 3 | 10 | 5 | Discontinued due to disease progression |

The final analysis includes data on 15 patients (6 patients from BCD-100 1 mg/kg cohort, including 1 patient after dose escalation, 6 patients from BCD-100 3 mg/kg cohort and 3 patients from BCD-100 10 mg/kg cohort). All patients received treatment within the framework of the main stage of the trial (85 days).

All patient cohorts were balanced with respect to main demographic characteristics. Patient gender distribution was even with 53.33% females (8 patients), 46.67% males (7 patients). The median patient age was 56 years (minimum age was 35 years, maximum age was 77 years).

In respect to characteristics of the main disease, patients with melanoma (9/15 patients) prevailed in the population. The population also included 4 patients with NSCLC, 1 patient with pleural mesothelioma and 1 patient with renal cell cancer. The duration of disease at the moment of screening in the entire population was (median) 17.07 months, minimum duration was 0.6 months, maximum duration was 91.2 months.

Clinical safety results summary from phase I trial

Safety was studied in a population including all patients who received at least one dose of the study product (n=15). Each of 15 patients exhibited AEs and/or SAEs, total of events was 247. Statistical analysis did not show any difference between the cohorts in respect to AEs (p=0.567, Kruskal-Wallis test). Summary Table 16 with product safety data is shown below.

AEs of severity grade 3 by CTCAE 4.03 were detected in 40.00% (6 out of 15) patients, i.e. in 2 patients in each cohort. The investigator considered AEs of severity grade 3 and above in 4 out 6 of these patients as treatment related: in a patient who received BCD-100 at a dose of 1 mg/kg, in a patient who received BCD-100 at a dose of 3 mg/kg, and in 2 patients who received BCD-100 at a dose of 10 mg/kg. Many of these adverse events were hematological.

SAEs were observed in 3 patients who received BCD-100 at a dose of 1 mg/kg, and in 1 patient who received 3 mg/kg of BCD-100.

During the trial, one case of dose-limiting toxicity (DLT) was detected. Patient 13-09 who received 2 doses of BCD-100 at 3 mg/kg showed abnormal endocrine profile (TSH reduction) and developed autoimmune thyroid disease (immune-mediated grade 2 adverse event by CTCAE 4.03 criteria). The Data Monitoring and Safety Committee regarded this case as a DLT event. Patient 13-09 continued receiving the study therapy, the course of the patient's disease was classified as stable by RECIST 1.1 and irRC criteria.

Apart from autoimmune thyroid disease, immune-mediated AEs were observed in 33.33% (5 out of 15) of patients: in 33.33% (2 out of 6) of patients who received 1 mg/kg of BCD-100, in 50.00 (3 out of 6) of patients who received 3 mg/kg of BCD-100, but in none of patients who received 10 mg/kg of BCD-100. All those AEs were severity grade 1 events by CTCAE 4.03, and were not regarded as DLT events.

AEs/SAEs resulted in a temporary discontinuation of the study therapy in 3 patients: in 16.67% (1 out of 6) of patients who received 1 mg/kg of BCD-100 and in 33.33% (2 out of 6) of patients who received 3 mg/kg of BCD-100. All AEs that caused temporary treatment discontinuation were immune-related (severity grade 1 hyperthyroidism in patient 10-03 who received 1 mg/kg of BCD-100; severity grade 1 autoimmune thyroid disease in patient 13-10, and severity grade 2 autoimmune thyroid disease in patient 13-09 (the both patients received 3 mg/kg of BCD-100)). Patient 10-03 stopped receiving the study therapy. Patients 13-09 and 13-10 received glucocorticoids for the disease, the treatment was then resumed. None of the treatment interruptions were above the norms (2-4 weeks) as established in the trial protocol.

The study product was canceled in 3 patients due to disease progression (in 2 of them, following 5 administrations at a dose of 1 mg/kg, and in one of them, following 5 administrations at a dose of 10 mg/kg). The reason for discontinuation in all cases was disease progression, but not the administration of BCD-100.

SAE resulted in treatment discontinuation followed by death of one of the study patients. Patient 13-10 who received 3 administrations of product at a dose of 3 mg/kg experienced severity grade 5 right hemispheric stroke. The investigator regarded this AE to have possibly associated with the study therapy.

No difference in terms of change in laboratory and physiological parameters over time between the groups of patients was observed. Although the analysis of these parameters revealed isolated examples of statistically significant differences, these differences were regarded as ones which reflect normal natural variability. Deviations detected after biochemical blood assay and blood coagulation test (increased level of transaminases, bilirubin, deviations in blood coagulation indices, etc.) were mostly typical for the study population (patients with inoperable neoplasms), expected and transient; the parameters normalized without consequences, no further treatment was necessary.

The described results demonstrate a favorable safety profile of BCD-100 product in any of the doses studied following intravenous administration.

**Table 16.** Safety data (BCD-100-1 trial)

| Patient proportion | All patients (n = 15) | Cohorts | | |
|---|---|---|---|---|
| | | BCD-100, 1 mg/kg (n = 6) | BCD-100, 3 mg/kg (n = 6) | BCD-100, 10 mg/kg (n = 3) |
| Any AE | 15 (100%) | 6 (100%) | 6 (100%) | 3 (100%) |
| - treatment related | 11 (73%) | 4 (67%) | 5 (83%) | 2 (67%) |
| Grade 3-5 AE | 6 (40%) | 2 (33%) | 2 (33%) | 2 (67%) |
| - treatment related | 4 (27%) | 1 (17%) | 1 (17%) | 2 (67%) |
| SAE | 3 (20%) | 2 (33%) | 1 (17%) | 0 (0%) |
| - treatment related | 1 (7%) | 0 (0%) | 1 (17%) | 0 (0%) |
| Dose-limiting toxicity | 1 (7%) | 0 (0%) | 1 (17%) | 0 (0%) |

(continued)

| Patient proportion | All patients (n = 15) | Cohorts | | |
|---|---|---|---|---|
| | | BCD-100, 1 mg/kg (n = 6) | BCD-100, 3 mg/kg (n = 6) | BCD-100, 10 mg/kg (n = 3) |
| Immune-mediated AEs | 5 (33%) | 2 (33%) | 3 (50%) | 0 (0%) |
| Premature discontinuation due to AE | 1 (7%) | 0 (0%) | 1 (17%) | 0 (0%) |
| Treatment cycle was postponed due to AE | 3 (25%) | 1 (17%) | 2 (33%) | 0 (0%) |
| Immunogenicity (formation of antibodies to product) | 0 (0%) | 0 (0%) | 0 (0%) | 0 (0%) |

Immunogenicity

Immunogenicity was evaluated at screening, on day 28 of the study therapy, at the end of the main period (on day 85); whereas among patients who continued receiving therapy with BCD-100 product, immunogenicity was also evaluated every 42 days thereafter. Immunogenicity was evaluated in 2 steps as follows: in step 1, we conducted screening for the presence of binding antibodies (BABs) to the product in serum samples from the patients; in step 2, we conducted screening of samples with positive BAB titer for the presence of neutralizing antibodies (NABs).

Immunogenicity was evaluated in all patients with serum samples available for analysis at the moment of screening and in subsequent days of analyses (n=15). No sample containing BABs was detected. Since there were no samples containing BABs, the analysis for the presence of NATs was not performed.

Pharmacokinetics/metabolism summary of phase I trials.

Pharmacokinetics (PK) assay includes data on patients with not more than 3 omitted/lost/spoiled blood serum samples for the determination of BCD-100 concentration following the first administration of the product (n=15).

Pharmacokinetics (both following the first administration of the product and through all administrations) was studied in the following groups:

- in the first patient (BCD-100 dose of 0.3 mg/kg) (n=1);
- in the first cohort of patients (BCD-100 dose of 1 mg/kg) (n=5);
- in the second cohort of patients (BCD-100 dose of 3 mg/kg) (n=6);
- in the third cohort of patients (BCD-100 dose of 10 mg/kg) (n=3);

The pharmacokinetic assay included evaluation of standard PK properties (distribution and excretion) of the study product following multiple intravenous administration. The results of the assay are shown in Table 17.

Blood samples for the pharmacokinetic assay (for the content of BCD-100 in blood serum) was taken in all patients enrolled, at each dose level. The following time points for sample collection were used: prior to the first administration of the product; 30 minutes, 2 h ($\pm$15 min), 4 h ($\pm$15 min), 6 h ($\pm$15 min), 24 h ($\pm$1 h), 48 h ($\pm$2 h), 192 h ($\pm$8 h), and 336 h ($\pm$8 h) following the first administration of the product (prior to the second administration), and prior to each subsequent administration of the product once in 2 weeks. Blood samples for the determination of concentration of BCD-100 in blood serum were collected simultaneously with the collection of samples for the immunogenicity analysis (to evaluate the possible effect of antibodies to the product on pharmacokinetic properties of BCD-100). The concentration of BCD-100 in blood serum was determined using a validated ELISA procedure.

**Table 17.** Pharmacokinetic parameters (BCD-100-1 trial)

| Mean (median [upper quartile; lower quartile]) | 1 mg/kg (n=5) | 3 mg/kg (n=6) | 10 mg/kg (n=3) | p-value[1] |
|---|---|---|---|---|
| $C_{max}$ ($\mu$g/ml) | 17.36 (13.9[13.1; 15.4]) | 50.65 (49.4[17.4; 74.7]) | 110.467 (93.100[85.80; 152.50]) | 0.0103 |
| $T_{max}$(h) | 8.1 (6[4;6]) | 12.333 (6[4;6]) | 0.5 | |

(continued)

| Mean (median [upper quartile; lower quartile]) | 1 mg/kg (n=5) | 3 mg/kg (n=6) | 10 mg/kg (n=3) | p-value[1] |
|---|---|---|---|---|
| $AUC_{(0-336 h)}$ (($\mu$g/ml)$\times$h) | 1934.815 (2201.575 [1576.75; 2360.925]) | 8972.413 (8482.938 [3397.3; 14254.5]) | 12927.308 (13441.075 [7382.850; 17958.000]) | 0.0088 |
| $AUC_{(0-\infty)}$ (($\mu$g/ml)$\times$h) | 3664.334 (3491.898 [2348.363; 3510.258]) | 18071.061 (15978.296 [14630.459; 21616.064]) | 27943.841 (27373.114 [11989.993; 44468.417]) | 0.0123 |
| T½(h) | 305.511 (288.811 [248.440; 319.423]) | 470.936 (447.192 [305.351; 597.541]) | 315.015 (336.479 [247.553; 361.014]) | |
| $C_{min}$ ($\mu$g/ml) | 3.260 (2.600 [1.800; 3.600]) | 15.817 (14.100 [10.300; 21.800]) | 30.833 (28.700 [12.900; 50.900]) | |
| [1] Kruskal-Wallis test | | | | |

All parameters were measured following single administration of the product, with exception of the $C_{min}$ parameter which was calculated for 6 administrations of the product (12 weeks).

Statistically significant differences between the three cohorts that received different doses were detected in the following parameter: $AUC_{(0-336 h)}$ (p=0.0088, Kruskal-Wallis test); $AUMC_{(0-336 h)}$ (p=0.0088, Kruskal-Wallis test); $AUC_{(0-\infty)}$ (p=0.0123, Kruskal-Wallis test); $C_{max}$ (p=0.0103, Kruskal-Wallis test). More favorable pharmacokinetic profiles were observed at the doses of 3 mg/kg and 10 mg/kg as compared to the dose of 1 mg/kg; however, the PK, efficacy and safety parameters did not exhibit association with dosage.

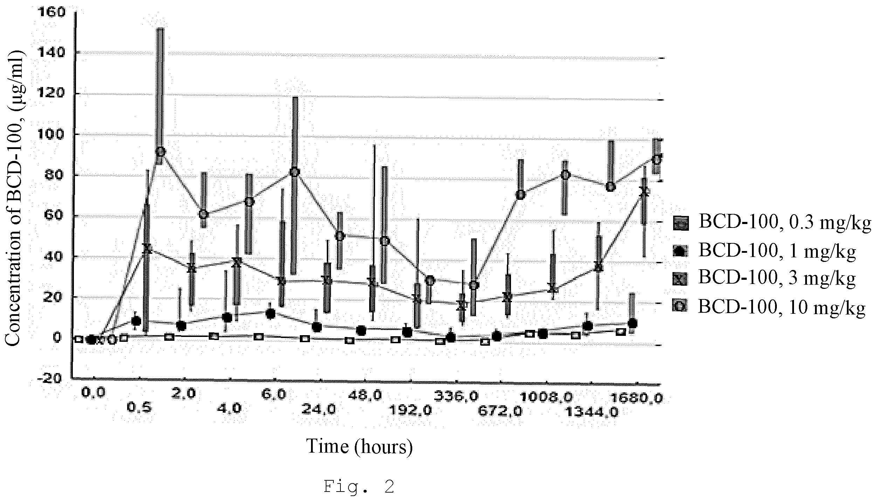

Figure 1 and Figure 2 show BCD-100 product concentration profiles for the period of the first administration to week 10 in all dose cohorts (1 mg/kg cohort [n=5], 3 mg/kg cohort [n=6] and 10 mg/kg cohort [n=3]).

The calculation of PK parameters showed that BCD-100 concentration is directly proportional to the dose administered, reaching the peak between 30 min and 6 h following the administration and gradually decreasing afterwards. We detected no dependence between half-life and the amount of product administered. The value of the $T_{½}$ parameter was typical for monoclonal antibodies (12-18 days).

Pharmacodynamics results summary from phase I trial

Pharmacodynamics was evaluated in all patients with serum samples available at the moment of screening and in subsequent days of analyses (n=15).

Pharmacodynamics was studied in the following groups:

- in the first patient (BCD-100 dose of 0.3 mg/kg) (n=1);
- in the first cohort of patients (BCD-100 dose of 1 mg/kg) (n=5);
- in the second cohort of patients (BCD-100 dose of 3 mg/kg) (n=6);
- in the third cohort of patients (BCD-100 dose of 10 mg/kg) (n=3);

The level of PD-1 receptor saturation by BCD-100 product helps to evaluate the interaction between the study product and a target thereof and is the most important pharmacodynamic marker of activity of monoclonal anti-PD-1 antibody, as PD-1 receptor blockade activates an anti-tumor immune response. Blood sampling for pharmacodynamics evaluation (% of PD-1 receptor saturation by BCD-100 product) was conducted in all patients. Sample collection for PD evaluation was performed prior to administration of the product, 4 h and 336 h following the administration (but prior to the second administration), and prior to the sixth administration of the product.

The results demonstrate high saturation of the PD-1 receptor by BCD-100 product (from 95% to 100%) at all dose levels and in all cell populations (Table 18).

**Table 18.** PD-1 receptor saturation (BCD-100-1 trial).

| Cohort | Parameter | CD4+ | | | $p^2$ | CD8+ | | | $p^2$ | CD4+ HLA-DR+ | | | $p^2$ | CD8+ HLA-DR+ | | | $p^2$ |
|---|---|---|---|---|---|---|---|---|---|---|---|---|---|---|---|---|---|
| | | Visit 1 | Visit 5 | Visit 10 | | Visit 1 | Visit 5 | Visit 10 | | Visit 1 | Visit | Visit 10 | | Visit 1 | Visit 5 | Visit 10 | |
| Cohort BCD-100 1 mg/kg (n = 5) | Mean | 95.60 | 95.64 | 91.65 | 0.7165 | 96.42 | 95.55 | 94.65 | 0.7165 | 97.83 | 98.17 | 96.86 | 0.7165 | 97.19 | 97.43 | 97.01 | 0.7165 |
| | Median | 95.21 | 97.72 | 95.44 | | 96.62 | 98.35 | 97.08 | | 98.45 | 99.10 | 99.15 | | 97.17 | 98.81 | 97.89 | |
| | Minimum | 89.89 | 86.24 | 83.41 | | 89.48 | 83.09 | 88.13 | | 94.00 | 94.87 | 91.42 | | 93.70 | 90.71 | 93.15 | |
| | Maximum | 99.36 | 99.03 | 96.10 | | 100.00 | 100.00 | 98.75 | | 100.00 | 100.00 | 100.00 | | 100.00 | 100.00 | 100.00 | |
| | L. quartile | 94.32 | 97.01 | 83.41 | | 96.23 | 97.81 | 88.13 | | 97.83 | 97.58 | 91.42 | | 95.24 | 98.14 | 93.15 | |
| | U. quartile | 99.21 | 98.20 | 96.10 | | 99.75 | 98.50 | 98.75 | | 98.86 | 99.31 | 100.00 | | 99.85 | 99.51 | 100.00 | |
| | ST. DEV. | 3.92 | 5.31 | 7.14 | | 4.25 | 7.01 | 5.71 | | 2.28 | 2.05 | 4.73 | | 2.78 | 3.82 | 3.51 | |
| | CV, % | 4.10 | 5.55 | 7.80 | | 4.41 | 7.34 | 6.03 | | 2.33 | 2.09 | 4.88 | | 2.86 | 3.93 | 3.62 | |
| Cohort BCD-100 3 mg/kg (n = 6) | Mean | 98.14 | 96.24 | 99.19 | 0.0695 | 99.48 | 95.72 | 99.58 | 0.0147 | 97.46 | 99.04 | 99.24 | 0.5134 | 98.75 | 97.88 | 99.05 | 0.9355 |
| | Median | 99.94 | 95.84 | 100.00 | | 100.00 | 96.27 | 100.00 | | 100.00 | 99.53 | 99.54 | | 100.00 | 98.63 | 99.90 | |
| | Minimum | 93.84 | 92.83 | 96.29 | | 97.39 | 90.80 | 98.08 | | 87.30 | 97.26 | 98.00 | | 95.31 | 91.75 | 95.95 | |
| | Maximum | 100.00 | 99.99 | 100.00 | | 100.00 | 98.26 | 100.00 | | 100.00 | 100.00 | 99.95 | | 100.00 | 100.00 | 100.00 | |
| | L. quartile | 96.93 | 93.85 | 99.65 | | 100.00 | 94.52 | 99.83 | | 100.00 | 97.93 | 98.88 | | 98.43 | 98.25 | 99.40 | |
| | U. quartile | 100.00 | 99.08 | 100.00 | | 100.00 | 98.18 | 100.00 | | 100.00 | 100.00 | 99.84 | | 100.00 | 100.00 | 100.00 | |
| | ST. DEV. | 2.74 | 2.95 | 1.63 | | 1.17 | 2.81 | 0.84 | | 5.68 | 1.16 | 0.81 | | 2.04 | 3.10 | 1.75 | |
| | CV, % | 2.80 | 3.07 | 1.64 | | 1.17 | 2.94 | 0.85 | | 5.83 | 1.17 | 0.82 | | 2.06 | 3.17 | 1.77 | |

**Table 18.** PD-1 receptor saturation (BCD-100-1 trial) (continued)

| Cohort | Parameter | CD4+ | | | p[2] | CD8+ | | | p[2] | CD4+ HLA-DR+ | | | p[2] | CD8+ HLA-DR+ | | | p[2] |
|---|---|---|---|---|---|---|---|---|---|---|---|---|---|---|---|---|---|
| | | Visit 1 | Visit 5 | Visit 10 | | Visit 1 | Visit 5 | Visit 10 | | Visit 1 | Visit 5 | Visit 10 | | Visit 1 | Visit 5 | Visit 10 | |
| Cohort BCD-100 10 mg/kg (n = 3) | Mean | 97.00 | 98.61 | 98.71 | 0.6065 | 96.86 | 100.00 | 97.82 | 0.1561 | 98.89 | 98.72 | 100.00 | 0.3679 | 99.37 | 100.00 | 100.00 | - |
| | Median | 97.45 | 99.25 | 98.71 | | 98.06 | 100.00 | 97.82 | | 98.82 | 99.02 | 100.00 | | 100.00 | 100.00 | 100.00 | |
| | Minimum | 93.66 | 96.58 | 97.41 | | 92.58 | 100.00 | 95.64 | | 98.18 | 97.13 | 100.00 | | 98.10 | 100.00 | 100.00 | |
| | Maximum | 99.89 | 100.00 | 100.00 | | 99.94 | 100.00 | 100.00 | | 99.66 | 100.00 | 100.00 | | 100.00 | 100.00 | 100.00 | |
| | L. quartile | 93.66 | 96.58 | 97.41 | | 92.58 | 100.00 | 95.64 | | 98.18 | 97.13 | 100.00 | | 98.10 | 100.00 | 100.00 | |
| | U. quartile | 99.89 | 100.00 | 100.00 | | 99.94 | 100.00 | 100.00 | | 99.66 | 100.00 | 100.00 | | 100.00 | 100.00 | 100.00 | |
| | ST. DEV. | 3.14 | 1. 80 | 1.83 | | 3.82 | 0.00 | 3. 08 | | 0.74 | 1.46 | 0.00 | | 1. 10 | 0.00 | 0.00 | |
| | CV, % | 3.24 | 1.82 | 1.86 | | 3.95 | 0.00 | 3.15 | | 0.75 | 1.48 | 0.00 | | 1. 10 | 0.00 | 0.00 | |
| p-value[1] | | 0.3236 | 0.416 | 0.047 | | 0.1083 | 0.032 | 0.1589 | | 0.3185 | 0.6547 | 0.1804 | | 0.2648 | 0.1402 | 0.3018 | |

Clinical efficacy results summary from phase I trial 14 out of 15 patients who received BCD-100 were included in a population for efficacy evaluation. One of the patients discontinued therapy due to SAE (death); no evaluation of tumor response to therapy using CT was performed by the time of patient's death.

Overall response rate (partial response rate + complete response rate) and disease control (stable disease + partial response + complete response) were determined 85 days following the therapy with BCD-100 product. Efficacy evaluation carried out 85 days following therapy with BCD-100 product was based on the analysis of CT scanning results. Tumor response was evaluated by RECIST 1.1 and Immune-Related RECIST (irRECIST) criteria.

According to irRECIST criteria developed for Immunotherapy, disease control was reached in 28.57% (4 out of 14) of patients. Overall response rate was 7.14% (1 out of 14). No significant differences in response rate between different patient cohorts were observed (Table 19).

Table 19. Tumor response (BCD-100-1 trial)

| Patient | BCD-100 dose mg/kg | Diagnosis | Mutations | Response by RECIST 1.1 | Response by irRECIST | BCD-100 therapy |
|---|---|---|---|---|---|---|
| 10-01 | 0.3→1 | Metastatic melanoma | BRAF V600E + | Partial response | Partial response (irPR) | Lasted 9 months (PR was confirmed after 6 and 9 months) |
| 08-02 | 1 | Locally advanced NSCLC | Not tested for EGFR and ALK | Disease progression | Stable disease (irSD) | Lasted 8 months (SD was confirmed after 6 months) |
| 10-03 | 1 | Metastatic melanoma | BRAF V600E + | Disease progression | Disease progression (irDP) | Discontinued |
| 08-04 | 1 | Metastatic pleural mesothelioma | | Disease progression | Stable disease (irSD) | Discontinued (due to clinical symptoms) |
| 12-05 | 1 | Unresectable melanoma | BRAF V600E + | Disease progression | Disease progression (irDP) | Discontinued |
| 12-06 | 1 | Metastatic melanoma | BRAF V600E + | Disease progression | Disease progression (irDP) | Discontinued |
| 12-07 | 3 | Metastatic NSCLC | EGFR+ | Disease progression | Disease progression (irDP) | Discontinued |
| 12-08 | 3 | Metastatic NSCLC | EGFR +, ALK was not tested | Disease progression | Disease progression (irDP) | Discontinued |
| 13-09 | 3 | Metastatic melanoma | BRAF- | Stable disease | Stable disease (irSD) | Lasted 2 months (lymphadenectomy) |
| 13-11 | 3 | Unresectable melanoma | BRAF- | Disease progression | Disease progression (irDP) | Discontinued |
| 01-12 | 3 | Metastatic renal cell cancer | | Disease progression | Disease progression (irDP) | Discontinued |
| 13-13 | 10 | Metastatic NSCLC | EGFR-, ALK- | Stable disease | Stable disease (irSD) | Lasted 1 month |

(continued)

| Patient | BCD-100dose mg/kg | Diagnosis | Mutations | Response by RECIST 1.1 | Response by irRECIST | BCD-100 therapy |
|---------|-------------------|-----------|-----------|------------------------|----------------------|-----------------|
| 13-14 | 10 | Metastatic melanoma | BRAF- | Disease progression | Disease progression (irDP) | Discontinued |
| 08-15 | 10 | Metastatic melanoma | BRAF- | Disease progression | Disease progression (irDP) | Discontinued |

Conclusion

The analysis of data obtained clearly demonstrates a favourable safety profile of BCD-100 product used at a wide range of doses ranging from 0.03 mg/kg to 10.0 mg/kg. Choosing of 2 administration regimens of BCD-100 product seems to be optimal for further clinical study: 1 mg/kg once in 2 weeks and 3 mg/kg once in 3 weeks.

Example 19. Use of prolgolimab (BCD-100) for treating patients with unresectable/metastatic melanoma, phase II trial, BCD-100-2/MIRACULUM

Trial design

Trial No. BCD-100-2/MIRACULUM (MIRACULUM, NCT03269565) is a multi-center open-label randomized trial of the pharmacokinetics, efficacy, safety, and immunogenicity of BCD-100 product (prolgolimab) as monotherapy in previously untreated or treated patients with unresectable/metastatic melanoma. BCD-100 product administered intravenously at a dose of 3 mg/kg once in 3 weeks was compared to BCD-100 administered intravenously at a dose of 1 mg/kg once in 2 weeks. The study is in progress now in the Russian Federation and Belarus. Data after 1 year of treatment were obtained and analyzed.

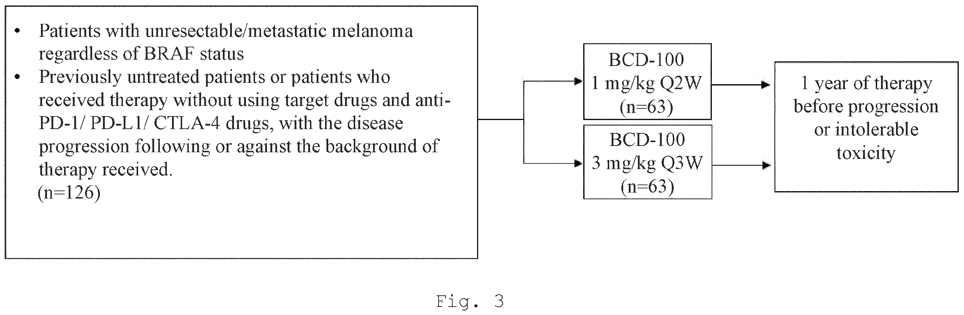

Each efficacy parameter was evaluated separately for each group. The primary endpoint for efficacy evaluation was overall response rate (partial response rate + complete response rate) by irRECIST in the study participants against the background of therapy with BCD-100 product. The best response to treatment for the above period was considered for the calculation of ORR (overall response rate) and disease control. The secondary endpoints for efficacy evaluation were progression-free and overall survival in patients 12 months after the treatment initiation, disease control rate (disease stabilization rate + partial response + complete response), time to treatment response, treatment response duration (Figure 3, Table 20).

**Table 20.** BCD-100-2/MIRACULUM (NCT03269565) trial characteristics

| Name Trial code (country) | Trial objectives | Patients | Design / duration | Therapy | Endpoints |
|---|---|---|---|---|---|
| International multi-center open-label randomized trial of the efficacy, pharmacokinetic properties, safety, and immunogenicity of BCD-100 product (JSC BIOCAD, Russia) as monotherapy in patients with unresectable/meta-static melanoma BCD-100-2/MIRACULUM (NCT03269565) Phase III (Russian Federation, Belarus) | PK, safety, efficacy and immunogenicity To study efficacy, pharmacokinetics, safety, and immunogenicity of two dose administration regimens of BCD-100 (JSC BIOCAD, Russia) as monotherapy in patients with unresetale/ metastatic melanoma. | 126 patients with melanoma: (n=63 in each of the groups) | Randomized, open-label international multi-center clinical trial in subjects with unresectable/ metastatic melanoma Main period: 52 weeks; follow-up: 12 weeks (total of 64 weeks) | BCD-100, intravenously, 1 mg/kg once in two weeks BCD100, intravenously, 3 mg/kg once in three weeks | Primary (PK) : AUCo-t, $C_{MAX}$, $T_{MAX}$ T½, $K_{EL}$, Cl, etc. following multiple intravenous administration using two dose regimens Immunogenicity: BAB/NAB detection rate following multiple i/v administration of the product using two dose regimens |

Trial results

Summary of characteristics of patient population

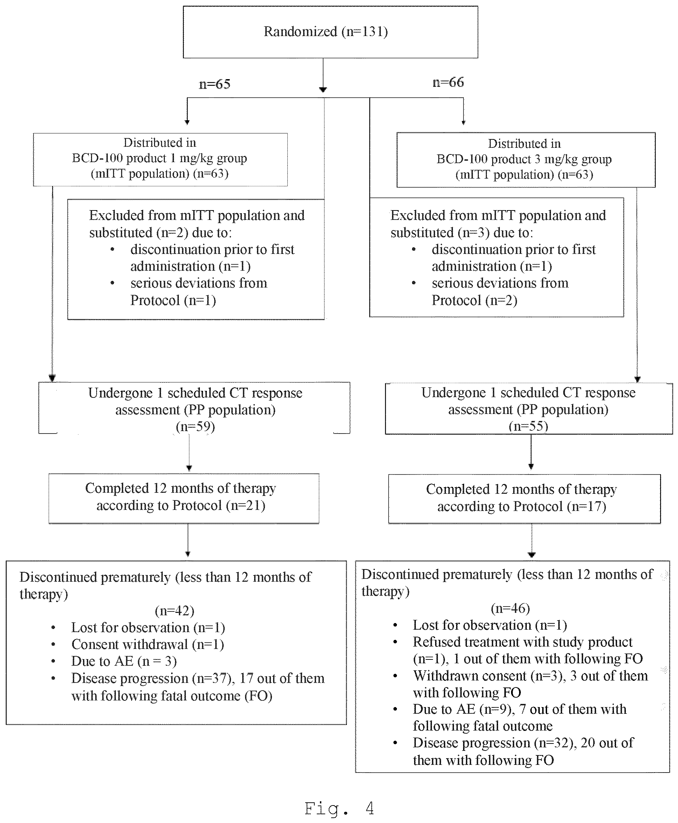

131 patients were enrolled aged 18 and older. The patient population included both males and females suffering from common melanoma, including choroidal melanoma. This population included patients who took the place of those who discontinued prior to first administration of BCD-100, or of those who discontinued as a result of serious deviations from the protocol. The resulting modified ITT population of patients who received at least one dose of BCD-100 included 126 patients. To qualify for enrollment, the patients must have had 0-1 ECOG score and at least one measurable target lesion according to RECIST 1.1 (excluding bone metastases) (FIgure 4, Table 21) .

Table 21. Disease characteristics in patient population (BCD-100-2/MIRACULUM trial)

| Parameter | BCD-100, 1 mg/kg n = 63 | | BCD-100, 3 mg/kg n = 63 | | p-value |
|---|---|---|---|---|---|
| | n | % | n | % | |
| **Diagnosis (at the moment of screening)** | | | | | |
| Unresectable stage II melanoma | 4 | 6.35 | 1 | 1.59 | 0.365[2] |
| Unresectable stage III melanoma | 0 | 0.00 | 1 | 1.59 | 1.000[2] |
| Metastatic stage IV melanoma | 59 | 93.65 | 61 | 96.83 | 0.680[2] |
| **Tumor histologic type** | | | | | |
| Mixed cell melanoma | 3 | 4.76 | 2 | 3.17 | 1.000[1] |
| Epithelioid cell melanoma | 23 | 36.51 | 26 | 41.27 | 0.715[1] |
| Spindle cell melanoma | 5 | 7.94 | 5 | 7.94 | 1.000[2] |
| Nevocellular melanoma | 1 | 1.59 | 0 | 0.00 | 1.000[2] |
| NA | 31 | 49.21 | 30 | 47.62 | 1.000[1] |

(continued)

| Tumors with non-typical localizations | | | | | |
|---|---|---|---|---|---|
| Melanoma with non-typical localization | 4 | 6.35 | 5 | 7.94 | 1.000[2] |
| Choroidal melanoma | 3 | 4.76 | 5 | 7.94 | 0.718[2] |
| Intestinal mucosal melanoma | 1 | 1.59 | 0 | 0.00 | 1.000[2] |
| **Degree of metastasis** | | | | | |
| **AJCC 7 classification** | | | | | |
| M0 | 0 | 0.00 | 0 | 0.00 | 1.000[2] |
| M1a | 6 | 9.52 | 8 | 12.70 | 0.777[1] |
| M1b | 13 | 20.64 | 8 | 12.70 | 0.339[1] |
| M1c | 44 | 69.84 | 47 | 74.60 | 0.691[1] |
| **AJCC8 classification** | | | | | |
| M1d | 17 | 26.98 | 9 | 14.29 | 0.123[1] |
| **CNS metastasis** | | | | | |
| Present | 17 | 26.98 | 9 | 14.29 | 0.123[1] |
| Not present | 46 | 73.02 | 54 | 85.71 | 0.123[1] |
| **PD-L1 status** | | | | | |
| Positive | 33 | 52.38 | 31 | 49.21 | 0.859[1] |
| Negative | 12 | 19.05 | 14 | 22.22 | 0.826[1] |
| Not studied | 18 | 28.57 | 18 | 28.57 | 1.000[1] |
| **BRAF status** | | | | | |
| BRAF mutation detected | 21 | 33.33 | 24 | 38.1 | 0.710[1] |
| BRAF mutation not detected | 25 | 39.68 | 22 | 34.92 | 0.713[1] |
| Not studied | 17 | 26.98 | 17 | 26.98 | 1.000[2] |
| **Median BTS (tumor volume at screening)** | | | | | |
| Median BTS | | 71 mm | | 97 mm | 0.041[3] |
| **LDH level at screening** | | | | | |
| Norm | 42 | 66.67 | 41 | 65.08 | 1.000[1] |
| Above norm | 21 | 33.33 | 22 | 34.92 | 1.000[1] |
| **Prior therapy** | | | | | |
| **Therapy of early stages** | | | | | |
| Neoadjuvant therapy | 0 | 0.00 | 0 | 0.00 | 1.000[2] |
| Surgical treatment | 59 | 93.65 | 49 | 77.78 | 0.02[2] |
| Adjuvant therapy | 23 | 36.51 | 24 | 38.1 | 1.000[1] |
| **Therapy of late stages** | | | | | |
| Chemotherapy of common disease | | | | | |
| Not conducted (BCD-100 product is used in first line) | 46 | 73.02 | 47 | 74.60 | 1.000[1] |
| Conducted | 17 | 26.98 | 16 | 25.40 | 1.000[1] |
| 1 line of prior therapy (BCD-100 product is used in second line) | 15 | 23.81 | 8 | 12.7 | 0.166[1] |

(continued)

| Therapy of late stages | | | | | |
| --- | --- | --- | --- | --- | --- |
| 2 lines of prior therapy (BCD-100 product is used in third line) | 2 | 3.17 | 8 | 12.7 | 0.095[2] |
| Other (immunotherapy, vaccinotherapy) | 6 | 9.52 | 5 | 7.94 | 1.000[2] |
| Radiotherapy | 4 | 6.35 | 7 | 11.11 | 0.53[2] |
| Duration of the disease (months) | | | | | |
| Mean | 33.73 | | 31.02 | | |
| Median | 19.55 | | 15.44 | | |
| Minimum | 0.53 | | 0.13 | | 0.174[3] |
| Maximum | 315.83 | | 227.09 | | |
| ST. DEV. | 46.12 | | 45.86 | | |
| Note: [1]Yates-corrected Pierson's $\chi^2$ test, [2]two-tailed Fisher's exact test, [3]Mann-Whitney test, NA - no data available. | | | | | |

Safety was analyzed in all patients who received at least one administration of the study product ("modified Intent-to-treat" (mITT) population, n=126).

Efficacy was analyzed in two patient populations:

- all patients who received at least one administration of the study product (mITT population, n=126);
- patients who received at least one administration of the study product and had at least 1 scheduled dynamic CT examination to evaluate response ("per protocol" (PP) population, n=114).

The pharmacokinetics assay included data on all patients who received at least one administration of BCD-100 product with not more than 3 omitted/lost/spoiled samples necessary according to the active protocol version as of the moment of determination (n=125).

Clinical efficacy results summary from trial No. BCD-100-2/MIRACULUM

Results of evaluation of efficacy primary endpoint (ORR) indicate sufficient efficacy of BCD-100 product at the both dose regimens, in all patient populations studied. Accordingly, ORR and disease control in the PP population was 40.68% and 67.80% in the 1st group and 32.73% and 52.73% in the 2nd therapy group. Similar efficacy results were observed in the mITT population. Thus, an expected target response rate (28%) and critical value r (11 responses) were reached in the both therapy groups.

Subgroup analysis of therapy lines demonstrated high efficacy of BCD-100 product at a minimal dose of 1 mg/kg in previously untreated patient population (ORR was 50.00%).

Results of analysis of secondary efficacy endpoints confirmed the conclusion about sufficient efficacy of BCD-100 product at the both doses. Rates of PFS, OS, response duration were comparable to those of best-in-class PD-1 inhibitors.

12-month PFS was 41.27% in patients of the BCD-100 1 mg/kg group, and 34.92% in patients of the BCD-100 3 mg/kg group. Median PFS was 5.78 months (95% CI 3.52 - -) in patients of the BCD-100 1 mg/kg group, and 2.33 months (95% CI 2.07-10.25) in patients of the BCD-100 3 mg/kg group (p=0.400, log-rank test). No statistically significant differences were observed after detailed assessment of progression-free survival in each of the groups, according to therapy lines (by irRECIST criteria, in the mITT population) . BCD-100 product was equally effective both when used in previously untreated patients and when used in previously treated patients.

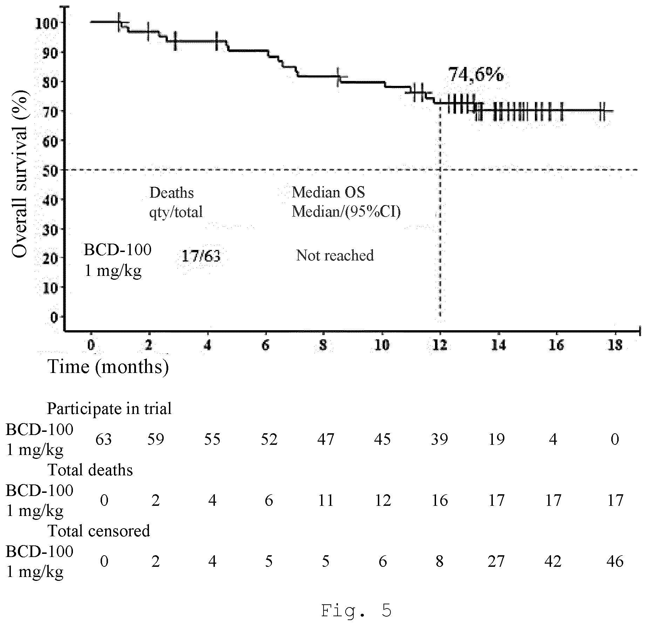

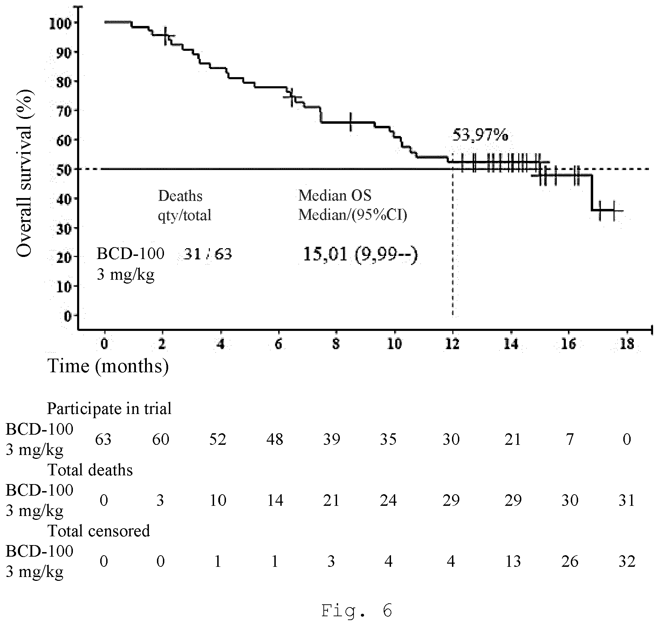

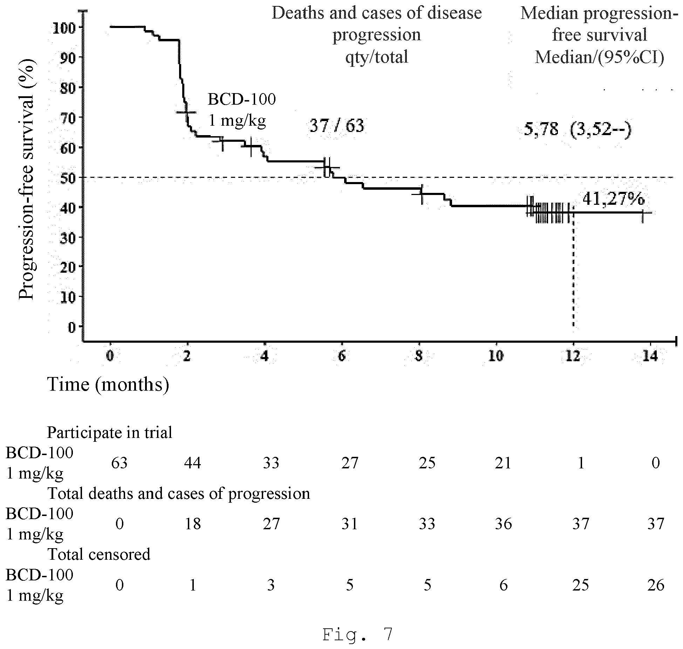

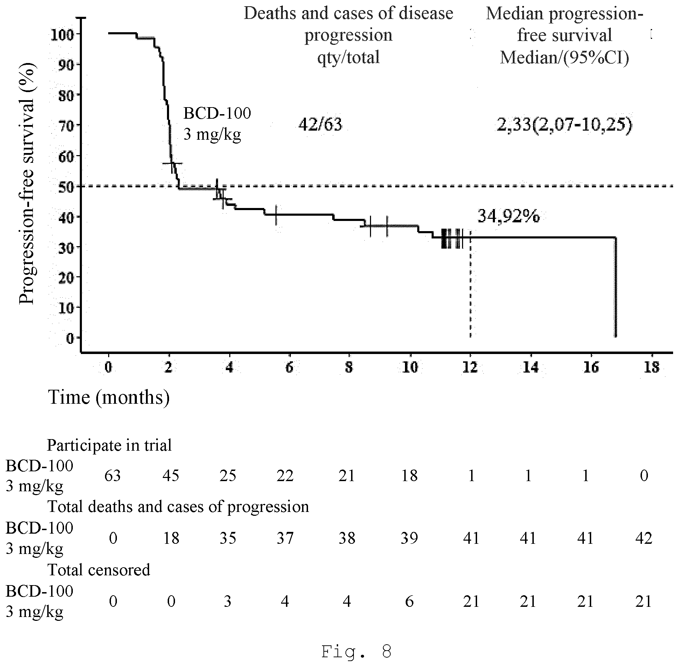

With median observation of 13.8 months (95% CI 13.2-14.7), median overall survival in patients of the BCD-100 1 mg/kg group was not reached (95% CI -[1] - -). 12-month OS was 74.60% in patients of the BCD-100 1 mg/kg group. Median OS in patients of the BCD-100 3 mg/kg group was 15 months (95% CI 9.99 - -) with median observation of 14.5 months (95% CI 13.9-15.2). 12-month OS was 53.97% in patients of the BCD-100 3 mg/kg group. No statistically significant differences were observed after detailed assessment of overall survival in each of the groups, according to therapy lines. BCD-100 product was equally effective both when used in previously untreated patients and when used in previously treated patients. Median OSs were reached by none of subgroups of patients who received BCD-100 at a dose of 1 mg/kg (p=0.800; log-rank test). Median OS was 16.8 months (95% CI 9.33 - -) in previously untreated patients and 15 months (95% CI 7.46 - -) in previously treated patients, in the BCD-100 product 3 mg/kg group (p=0.900; log-rank test) (Table 22, Table 23, Figure 5, Figure 6, Figure 7, Figure 8).

Table 22. Primary endpoint reflecting product efficacy in the PP population (population of patients who received treatment according to the protocol) based on the results of BCD-100-2/MIRACULUM trial

| Efficacy parameters | irRECIST | | | | |
| | BCD-100, 1 mg/kg (n=59) | | BCD-100, 3 mg/kg (n=55) | | p-value |
| | n | % | n | % | |
| Partial response (PR) | 19 | 32.20 | 16 | 29.09 | 0.875[1] |
| Complete response (CR) | 5 | 8.47 | 2 | 3.64 | 0.440[2] |
| Stabilization (ST) | 16 | 27.12 | 11 | 20.00 | 0.501[1] |
| Progression (PROG)[2] | 19 | 32.20 | 26 | 47.27 | 0.146[1] |
| **Disease control (DC)** | **40** | **67.80** | **29** | **52.73** | **0.146[1]** |
| **Overall response rate (ORR)** | **24** | **40.68** | **18** | **32.73** | **0.493[1]** |
| Note: [1]Yates-corrected Pierson's $\chi^2$ test, [2]two-tailed Fisher's exact test | | | | | |

Clinical safety results summary from trial No. BCD-100-2/MIRACULUM

BCD-100 demonstrated a favorable safety profile in the both dose regimens (Table 23).

Table 23. BCD-100 product safety profile according to the results of BCD-100-2/MIRACULUM trial [1] "-" presently and herein means "not reached" [2] Confirmed by data from 1 scheduled CT examination at 8 visit

| Percentage of patients with: | BCD-100, 1 mg/kg n=63 n(%) | BCD-100, 3 mg/kg n=63 n(%) | P-value |
| --- | --- | --- | --- |
| AEs | 54 (85.71%) | 54 (85.71%) | 1.000[1] |
| AEs associated, in investigators' opinion, with the therapy received | 35 (55.56%) | 34 (53.97%) | 1.000[1] |
| Severity grade 3 and above AEs by CTCAE 4.03 | 18 (28.57%) | 21 (33.33%) | 0.699[1] |
| Severity grade 3 and above AEs by CTCAE 4.03 associated, in investigators' opinion, with the therapy received | 8 (12.70%) | 2 (3.17%) | 0.095[2] |
| SAEs | 5 (7.94%) | 10 (15.87%) | 0.271[2] |
| SAEs associated, in investigators' opinion, with the therapy received | 2 (3.17%) | 0 (0.00%) | 0.496[2] |
| imAEs | 23 (36.51%) | 22 (34.92%) | 1.000[1] |
| Severity grade 3 and above imAEs by CTCAE 4.03 | 5 (7.94%) | 1 (1.59%) | 0.207[2] |
| Therapy discontinuation due to AE development | 3 (4.76%) | 2 (3.17%) | 1.000[2] |
| Therapy discontinuation due to imAE development | 1 (1.59%) | 1 (1.59%) | 1.000[2] |
| Note: [1]Yates-corrected Pierson's $\chi^2$ test [2]two-tailed Fisher's exact test | | | |

Based on the data above, one may conclude that the results as obtained in the present study do not contradict the known data on the safety profile of monoclonal anti-PD-1 receptor antibody products. No statistically significant differences in product safety profile between the doses studied were observed, with the exception of the amount of SAEs resulting in death (a significantly larger amount was observed in patients of the BCD-100 3 mg/kg group).

Immunogenicity

Immunogenicity was evaluated in all patients with serum samples available for analysis at the moment of screening and in subsequent days of analyses (n=121). BABs to BCD-100 were detected in none of the patients.

Pharmacokinetics and metabolism summary results from trial No. BCD-100-2/MIRACULUM

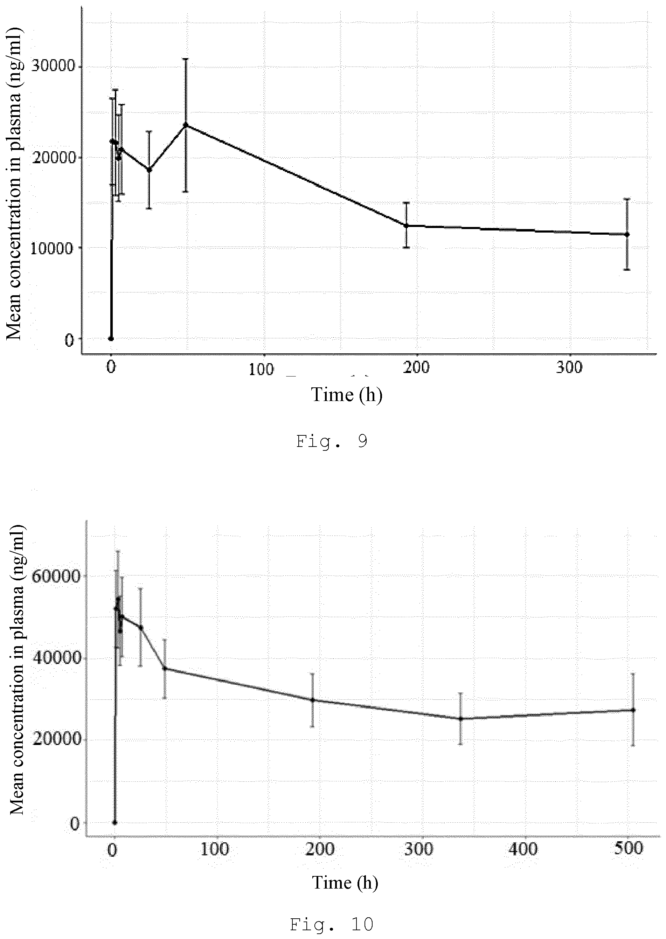

The analysis showed that a single intravenous administration of BCD-100 product at a dose from 1 to 3 mg/kg is characterized by linearly increasing concentration of the study substance in blood plasma, followed by a uniform decrease, where the half-life of the product, regardless of a dose administered into an organism, is characterized by the time from 11.5 to 17 days that is typical for that of IgG immunoglobulins (Figure 9, Figure 10).

The tendency of dose-dependent increase in study product concentration in blood plasma (according to $C_{max}$ and $AUC_{0-t,SS}$ parameters) remains following multiple administration of BCD-100 product as well, which corresponds to the literature data on PK profile of other anti-PD-1 antibody products.

Persistent maintenance of $C_{min}$ at a sufficient level against the background of a treatment course, as demonstrated in clinical trial No. BCD-100-2/MIRACULUM, indicates that the therapeutic concentrations of the product are maintained both when the BCD-100 product is used at a dose of 1 mg/kg once in 2 weeks and when the BCD-100 product is used at a dose of 3 mg/kg in a regimen of once in 3 weeks. Thus, the use of BCD-100 in the both dose regimens is justified from the perspective of pharmacokinetic properties.

The assessment of the $T_{1/2}$ parameter, including comparative assessment between the treatment arms, was difficult due to the fact that a number of patients lacked the typical terminal product elimination phase following a single administration.

Overall, BCD-100 product both at a dose of 1 mg/kg once in 2 weeks and at a dose of 3 mg/kg once in 3 weeks was characterized by pharmacokinetic properties that ensure a long retention time of the product in the organism, which is sufficient for maintaining a stable therapeutic concentration against the background of multiple administration. No dose-dependent efficacy/safety was observed.

Pharmacodynamics results summary from phase I trial

The percentage of PD-1 receptor saturation by BCD-100 product in all white blood cell subpopulations did not differ between the therapy arms. PD-1 saturation of >99% in the population of activated helper T cells and cytotoxic white blood cells was observed in 33.33% of patients who were analyzed for this parameter (14 out of 42). No statistically significant difference was observed between the therapy arms: 8 patients received BCD-100 at a dose of 1 mg/kg, 6 patients received BCD-100 at a dose 3 mg/kg.

Ki-67 is a universal marker of cell proliferation due to the fact that it is found only in dividing cells and degraded for 1.5 to 2 h after mitosis. The Ki-67 marker is detected in the regions of telomeres, centromeres, and within nucleoli. During the therapy, the percentage of Ki-67-positive cytotoxic T cells increased in the both groups, no significant differences were, however, observed. The analysis of the portion of Ki-67-positive cytotoxic T cells also did not reveal any statistically significant differences between the groups.

During the therapy, the percentage of Th9 in the overall population of helper T cells increased mainly in group 2 (BCD-100, 3 mg/kg Q3W), no significant differences were, however, observed. Also, no significant differences were found after cross-group comparison.

According to the literature data available, early increase in Th9 level is associated with improved response to treatment with nivolumab for melanoma.[3] It should be noted that these results were obtained for a limited number of patients (n=42) who were heterogeneous in gender and characteristics of the main disease.

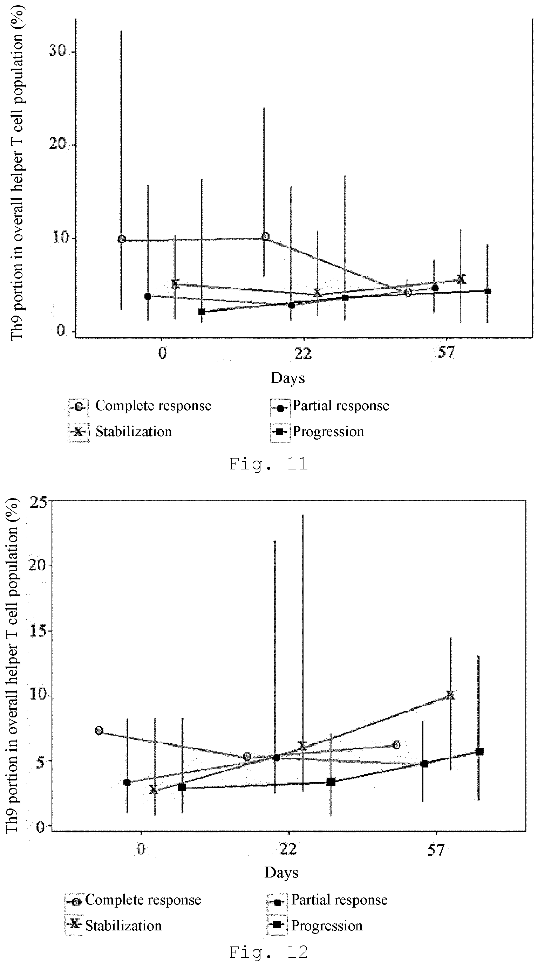

No statistically significant differences were revealed in the both cohorts after a comparative analysis of Th9 portion in the overall population of helper T cells in subgroups exhibiting different types of responses to therapy with BCD-100 product. However, the provided graphs show a tendency towards an improved response to the therapy in patients with initially increased level of Th9 (Figure 11, Figure 12).

[3] Nonomura Y, Otsuka A, Hakashima C, Seidel JA, Kitoh A, Dainichi T, Hakajima S, Sawada Y, Matsushita S, Aoki M, Takenouchi T, Fujimura T, Hatta N, Koreeda S, Fukushima S, Honda T, Kabashima K, Peripheral blood Th9 cells are a possible pharmacodynamic biomarker of nivolumab treatment efficacy in metastatic melanoma patients. Oncoimmunology_2016 Oct 18;5(12):e1248327.

Conclusion

The resulting data on efficacy, safety and pharmacokinetic properties of BCD-100 are sufficient to justify the use of the product both in a dose regimen of 1 mg/kg once in 2 weeks and in a dose regimen of 3 mg/kg once in 3 weeks.

The international multi-center open-label randomized phase II trial No. BCD-100-2/MIRACULUM in a patient population with unresectable/metastatic melanoma demonstrated significant therapeutic advantages as compared to the known data on chemotherapy efficacy, and results that are comparable to those of the best existing treatment methods. Taking into account the efficacy data with a favorable safety profile, as obtained in the trial, the use of BCD-100 product in routine clinical practice meets the benefit-risk balance in the given patient population.

**Claims**

1. An aqueous pharmaceutical composition of anti-PD-1 antibody comprising:

    (a) prolgolimab at a concentration from 15 mg/ml to 40 mg/ml as an antibody;
    (b) trehalose dihydrate at a concentration from 80 mg/ml to 110 mg/ml;
    (c) sodium acetate trihydrate at a concentration from 0.2 mg/ml to 2.5 mg/ml; and
    (d) acetic acid to pH 4.5-5.5.

2. The aqueous pharmaceutical composition according to Claim 1, wherein said prolgolimab is present at a concentration of 20 mg/ml.

3. The aqueous pharmaceutical composition according to any of Claims 1-2, wherein said trehalose dihydrate is present at a concentration from 95 mg/ml to 105 mg/ml.

4. The aqueous pharmaceutical composition according to Claim 3, wherein said trehalose dihydrate is present at a concentration of 100 mg/ml.

5. The aqueous pharmaceutical composition according to Claim 4, wherein said sodium acetate trihydrate is present at a concentration from 1.6 mg/ml to 1.9 mg/ml.

6. The aqueous pharmaceutical composition according to Claim 5, wherein said sodium acetate trihydrate is present at a concentration from 1.7 mg/ml to 1.8 mg/ml.

7. The aqueous pharmaceutical composition according to Claim 6, wherein said sodium acetate trihydrate is present at a concentration of 1.742 mg/ml.

8. The aqueous pharmaceutical composition according to any of Claims 1-2, wherein said acetic acid is added to pH 5.0.

9. The aqueous pharmaceutical composition of anti-PD-1 antibody comprising:

    (a) prolgolimab at a concentration from 90 mg/ml to 150 mg/ml as an antibody;
    (b) trehalose dihydrate at a concentration from 50 mg/ml to 110 mg/ml;
    (c) sodium acetate trihydrate at a concentration from 0.2 mg/ml to 2.5 mg/ml; and
    (d) acetic acid to pH from 4.5 to 5.5.

10. The aqueous pharmaceutical composition according to Claim 9, wherein said prolgolimab is present at a concentration from 90 mg/ml to 110 mg/ml.

11. The aqueous pharmaceutical composition according to any of Claims 9-10, wherein said prolgolimab is present at a concentration of 100 mg/ml.

12. The aqueous pharmaceutical composition according to Claim 9, wherein said trehalose dihydrate is present at a concentration from 75 mg/ml to 85 mg/ml.

13. The aqueous pharmaceutical composition according to Claim 12, wherein said trehalose dihydrate is present at a concentration of 80 mg/ml.

**14.** The aqueous pharmaceutical composition according to Claim 9, wherein said sodium acetate trihydrate is present at a concentration from 1.6 mg/ml to 1.9 mg/ml.

**15.** The aqueous pharmaceutical composition according to Claim 14, wherein said sodium acetate trihydrate is present at a concentration from 1.7 mg/ml to 1.8 mg/ml.

**16.** The aqueous pharmaceutical composition according to Claim 15, wherein said sodium acetate trihydrate is present at a concentration of 1.742 mg/ml.

**17.** The aqueous pharmaceutical composition according to Claim 8, wherein said acetic acid is added to pH from 5.0 to 5.5.

**18.** The aqueous pharmaceutical composition of anti-PD-1 antibody comprising:

(a) prolgolimab at a concentration from 5 mg/ml to 150 mg/ml as an antibody;
(b) trehalose dihydrate at a concentration from 70 mg/ml to 110 mg/ml;
(c) L-histidine at a concentration from 0.2 to 2.5 mg/ml; and
(d) L-histidine hydrochloride at a concentration from 0.2 to 3.5 mg/ml.

**19.** The aqueous pharmaceutical composition according to Claim 18, wherein said prolgolimab is present at a concentration from 15 mg/ml to 40 mg/ml.

**20.** The aqueous pharmaceutical composition according to Claim 19, wherein said prolgolimab is present at a concentration from 15 mg/ml to 25 mg/ml.

**21.** The aqueous pharmaceutical composition according to Claim 20, wherein said prolgolimab is present at a concentration of 20 mg/ml.

**22.** The aqueous pharmaceutical composition according to any of Claims 18-21, wherein said trehalose dihydrate is present at a concentration from 95 mg/ml to 105 mg/ml.

**23.** The aqueous pharmaceutical composition according to Claim 22, wherein said trehalose dihydrate is present at a concentration of 100 mg/ml.

**24.** The aqueous pharmaceutical composition according to Claim 18, wherein said prolgolimab is present at a concentration from 90 mg/ml to 110 mg/ml.

**25.** The aqueous pharmaceutical composition according to Claim 24, wherein said prolgolimab is present at a concentration of 100 mg/ml.

**26.** The aqueous pharmaceutical composition according to Claims 18, 24-25, wherein said trehalose dihydrate is present at a concentration from 75 mg/ml to 85 mg/ml.

**27.** The aqueous pharmaceutical composition according to Claim 26, wherein said trehalose dihydrate is present at a concentration of 80 mg/ml.

**28.** The aqueous pharmaceutical composition according to Claim 18, wherein said L-histidine is present at a concentration from 0.7 mg/ml to 1.0 mg/ml.

**29.** The aqueous pharmaceutical composition according to Claim 28, wherein said L-histidine is present at a concentration of 0.92 mg/ml.

**30.** The aqueous pharmaceutical composition according to Claim 18, wherein said L-histidine hydrochloride is present at a concentration from 2.8 mg/ml to 3.3 mg/ml.

**31.** The aqueous pharmaceutical composition according to Claim 30, wherein said L-histidine hydrochloride is present at a concentration of 2.96 mg/ml.

32. The aqueous pharmaceutical composition according to Claim 18, wherein said composition has pH from 5.5 to 6.5.

33. The aqueous pharmaceutical composition according to Claim 32, wherein said composition has pH from 5.5 to 6.0.

34. The aqueous pharmaceutical composition according to any of Claims 1, 18, further comprising a suitable solubilizer.

35. The aqueous pharmaceutical composition according to Claim 34, wherein said solubilizer is Poloxamer 188.

36. The aqueous pharmaceutical composition according to Claim 35, wherein said Poloxamer 188 is in an amount greater than 0 mg/ml but equal to or less than 1 mg/ml.

37. The aqueous pharmaceutical composition of anti-PD-1 antibody comprising:

   (a) prolgolimab at a concentration of 20 mg/ml as an antibody;
   (b) trehalose dihydrate at a concentration of 100 mg/ml;
   (c) sodium acetate trihydrate at a concentration from 0.2 to 2.5 mg/ml; and
   (d) acetic acid to pH from 4.5 to 5.5.

38. The aqueous pharmaceutical composition of anti-PD-1 antibody according to Claim 37 comprising:

   (a) prolgolimab at a concentration of 20 mg/ml as an antibody;
   (b) trehalose dihydrate at a concentration of 100 mg/ml;
   (c) sodium acetate trihydrate at a concentration from 1.7 mg/ml to 1.8 mg/ml; and
   (d) acetic acid to pH 5.0.

39. The aqueous pharmaceutical composition of anti-PD-1 antibody according to Claim 38 comprising:

   (a) prolgolimab at a concentration of 20 mg/ml as an antibody;
   (b) trehalose dihydrate at a concentration of 100 mg/ml;
   (c) sodium acetate trihydrate at a concentration of 1.742 mg/ml; and
   (d) acetic acid to pH 5.0.

40. The aqueous pharmaceutical composition of anti-PD-1 antibody comprising:

   (a) prolgolimab at a concentration of 100 mg/ml as an antibody;
   (b) trehalose dihydrate at a concentration of 80 mg/ml;
   (c) sodium acetate trihydrate at a concentration from 0.2 to 2.5 mg/ml; and
   (d) acetic acid to pH from 4.5 to 5.5.

41. The aqueous pharmaceutical composition of anti-PD-1 antibody according to Claim 40 comprising:

   (a) prolgolimab at a concentration of 100 mg/ml as an antibody;
   (b) trehalose dihydrate at a concentration of 80 mg/ml;
   (c) sodium acetate trihydrate at a concentration from 1.7 mg/ml to 1.8 mg/ml; and
   (d) acetic acid to pH from 5.0 to 5.5.

42. The aqueous pharmaceutical composition of anti-PD-1 antibody according to Claim 41 comprising:

   (a) prolgolimab at a concentration of 100 mg/ml as an antibody;
   (b) trehalose dihydrate at a concentration of 80 mg/ml;
   (c) sodium acetate trihydrate at a concentration of 1.742 mg/ml; and
   (d) acetic acid to pH from 5.0 to 5.5.

43. The aqueous pharmaceutical composition of anti-PD-1 antibody comprising:

   (a) prolgolimab at a concentration of 20 mg/ml as an antibody;
   (b) trehalose dihydrate at a concentration of 100 mg/ml;
   (c) L-histidine at a concentration from 0.2 to 2.5 mg/ml; and

(d) L-histidine hydrochloride at a concentration from 0.2 to 3.5 mg/ml;
(e) wherein said composition has pH from 5.5 to 6.5.

44. The aqueous pharmaceutical composition of anti-PD-1 antibody according to Claim 43 comprising:

(a) prolgolimab at a concentration of 20 mg/ml as an antibody;
(b) trehalose dihydrate at a concentration of 100 mg/ml;
(c) L-histidine at a concentration of 0.92 mg/ml; and
(d) L-histidine hydrochloride at a concentration of 2.96 mg/ml;
(e) wherein said composition has pH 5.5.

45. The aqueous pharmaceutical composition of anti-PD-1 antibody comprising:

(a) prolgolimab at a concentration of 100 mg/ml as an antibody;
(b) trehalose dihydrate at a concentration of 80 mg/ml;
(c) L-histidine at a concentration from 0.2 to 2.5 mg/ml; and
(d) L-histidine hydrochloride at a concentration from 0.2 to 3.5 mg/ml;
(e) wherein said composition has pH from 5.5 to 6.5.

46. The aqueous pharmaceutical composition of anti-PD-1 antibody according to Claim 45 comprising:

(a) prolgolimab at a concentration of 100 mg/ml as an antibody;
(b) trehalose dihydrate at a concentration of 80 mg/ml;
(c) L-histidine at a concentration of 0.92 mg/ml; and
(d) L-histidine hydrochloride at a concentration of 2.96 mg/ml;
(e) wherein said composition has pH from 5.5 to 6.0.

47. The aqueous pharmaceutical composition of anti-PD-1 antibody comprising:

(a) prolgolimab at a concentration of 20 mg/ml as an antibody;
(b) trehalose dihydrate at a concentration of 100 mg/ml;
(c) sodium acetate trihydrate at a concentration of 1.742 mg/ml;
(d) acetic acid to pH 5.0.

48. The aqueous pharmaceutical composition of anti-PD-1 antibody comprising:

(a) prolgolimab at a concentration of 20 mg as an antibody;
(b) trehalose dihydrate at a concentration of 100 mg;
(c) sodium acetate trihydrate at a concentration of 1.742 mg;
(d) acetic acid to pH 5.0, and
(e) water for injections up to 1.0 ml.

49. The aqueous pharmaceutical composition according to any of Claims 37-48, further comprising a suitable solubilizer.

50. The aqueous pharmaceutical composition according to Claim 49, wherein said solubilizer is Poloxamer 188.

51. The aqueous pharmaceutical composition according to Claim 50, wherein said Poloxamer 188 is present in an amount greater than 0 mg/ml but equal to or less than 1 mg/ml.

52. The aqueous pharmaceutical composition according to any of Claims 1-51, wherein said composition is intended for parenteral administration.

53. The aqueous pharmaceutical composition according to Claim 52, wherein said composition is intended for intravenous, subcutaneous or intramuscular administration.

54. The aqueous pharmaceutical composition according to any of Claims 1-51, wherein said composition is present in a vial.

55. The aqueous pharmaceutical composition according to Claim 54, wherein said vial is a glass vial.

56. The aqueous pharmaceutical composition according to Claim 54, wherein said vial has a volume from 1 ml to 50 ml.

57. The aqueous pharmaceutical composition according to Claim 54, wherein said vial has a volume of 5 ml, 10 ml, 15 ml or 20 ml.

58. The aqueous pharmaceutical composition according to any of Claims 1-51, wherein said composition is in a syringe.

59. The aqueous pharmaceutical composition according to Claim 58, wherein said syringe has a capacity of 1 ml.

60. The aqueous pharmaceutical composition according to Claim 58, wherein said syringe has a capacity of 2 ml.

61. The aqueous pharmaceutical composition according to any of Claims 1-51, wherein said composition is in a pre-filled syringe.

62. The aqueous pharmaceutical composition according to Claim 61, wherein said pre-filled syringe has a capacity of 1 ml.

63. The aqueous pharmaceutical composition according to Claim 61, wherein said pre-filled syringe has a capacity of 2 ml.

64. A use of an aqueous pharmaceutical composition of anti-PD-1 antibody prolgolimab according to any of Claims 1-63 for treating a malignant neoplasm in a subject in need thereof.

65. The use according to Claim 64, wherein the malignant neoplasm is selected from the group comprising melanoma, including inoperable or metastatic melanoma, early stages of melanoma before and after definitive treatment; lung cancer, non-small cell lung cancer (NSCLC), including inoperable or metastatic non-small cell lung cancer; non-squamous non-small cell lung cancer, squamous cell lung cancer; small cell lung cancer, including inoperable or metastatic small cell lung cancer; early stages of lung cancer before and after definitive treatment; cervical cancer, including metastatic cervical cancer, early stages of cervical cancer before and after definitive treatment; head and neck tumors, including head and neck squamous cell cancer; Hodgkin's lymphoma; stomach and bowel tumors, metastatic squamous cell esophageal cancer; bladder cancer, including metastatic urothelial carcinoma, kidney cancer; endometrial cancer, including metastatic endometrial cancer, early stages of endometrial cancer before and after definitive treatment; breast cancer, including metastatic breast cancer, early stages of endometrial cancer before and after definitive treatment; liver cancer, including metastatic or inoperable liver cancer, early stages of liver cancer before and after definitive treatment; inoperable or metastatic solid tumor, including inoperable or metastatic solid tumor with signs of microsatellite instability.

66. The use of an aqueous pharmaceutical composition of anti-PD-1 antibody prolgolimab according to any of Claims 1-8, 19-22, 37-39, 43-44 for treating a malignant neoplasm in a subject in need thereof.

67. The use according to Claim 66, wherein said aqueous pharmaceutical composition is administered at a dose of anti-PD-1 antibody prolgolimab of 1 mg/kg of body weight.

68. The use according to Claim 66, wherein said aqueous pharmaceutical composition is administered at a dose of anti-PD-1 antibody prolgolimab of 3 mg/kg of body weight.

69. The use according to Claim 66, wherein said aqueous pharmaceutical composition is administered every 2 weeks.

70. The use according to Claim 66, wherein said aqueous pharmaceutical composition is administered every 3 weeks.

71. The use according to Claim 66, wherein said aqueous pharmaceutical composition is administered at a dose of anti-PD-1 antibody prolgolimab of 1 mg/kg of body weight every 2 weeks.

72. The use according to Claim 66, wherein said aqueous pharmaceutical composition is administered at a dose of anti-PD-1 antibody prolgolimab of 3 mg/kg of body weight every 3 weeks.

73. The use according to Claim 66, wherein said aqueous pharmaceutical composition is administered parenterally.

74. The use according to Claim 73, wherein said parenteral administration is intravenous, subcutaneous or intramuscular administration.

75. The use according to Claim 73, wherein said aqueous pharmaceutical composition is administered intravenously as an infusion.

76. The use according to Claim 66, wherein the malignant neoplasm is melanoma, including inoperable or metastatic melanoma, early stages of melanoma before and after definitive treatment; lung cancer, non-small cell lung cancer (NSCLC), including inoperable or metastatic non-small cell lung cancer.

77. A method for treating a malignant neoplasm in a subject in need thereof comprising administering a therapeutically effective amount of an aqueous pharmaceutical composition according to any of Claims 1-8, 19-22, 37-39, 43-44.

78. The method according to Claim 77, wherein said aqueous pharmaceutical composition is administered at a dose of anti-PD-1 antibody prolgolimab of 1 mg/kg of body weight.

79. The method according to Claim 77, wherein said aqueous pharmaceutical composition is administered at a dose of anti-PD-1 antibody prolgolimab of 3 mg/kg of body weight.

80. The method according to Claim 77, wherein said aqueous pharmaceutical composition is administered every 2 weeks.

81. The method according to Claim 77, wherein said aqueous pharmaceutical composition is administered every 3 weeks.

82. The method according to Claim 77, wherein said aqueous pharmaceutical composition is administered at a dose of anti-PD-1 antibody prolgolimab of 1 mg/kg of body weight every 2 weeks.

83. The method according to Claim 77, wherein said aqueous pharmaceutical composition is administered at a dose of anti-PD-1 antibody prolgolimab of 3 mg/kg of body weight every 3 weeks.

84. The method according to Claim 77, wherein said aqueous pharmaceutical composition is administered parenterally.

85. The method according to Claim 84, wherein said parenteral administration is intravenous, subcutaneous or intramuscular administration.

86. The method according to Claim 84, wherein said aqueous pharmaceutical composition is administered intravenously as an infusion.

87. The method according to Claim 77, wherein the malignant neoplasm is melanoma, including inoperable or metastatic melanoma, early stages of melanoma before and after definitive treatment; lung cancer, non-small cell lung cancer (NSCLC), including inoperable or metastatic non-small cell lung cancer.

88. The method for treating a malignant neoplasm in a subject in need thereof comprising administering a therapeutically effective amount of prolgolimab.

89. The method according to Claim 88, wherein said prolgolimab is administered at a dose of 1 mg/kg.

90. The method according to Claim 88, wherein said prolgolimab is administered at a dose of 3 mg/kg.

91. The method according to Claim 88, wherein said prolgolimab is administered every 2 weeks.

92. The method according to Claim 88, wherein said prolgolimab is administered every 3 weeks.

93. The method according to Claim 88, wherein said prolgolimab is administered at a dose of 1 mg/kg every 2 weeks.

94. The method according to Claim 88, wherein said prolgolimab is administered at a dose of 3 mg/kg every 3 weeks.

95. The method according to Claim 88, wherein said prolgolimab is administered parenterally.

**96.** The method according to Claim 95, wherein said parenteral administration is intravenous, subcutaneous or intramuscular administration.

**97.** The method according to Claim 95, wherein said prolgolimab is administered intravenously as an infusion.

**98.** The method according to Claim 88, wherein the malignant neoplasm is melanoma, including inoperable or metastatic melanoma, early stages of melanoma before and after definitive treatment; lung cancer, non-small cell lung cancer (NSCLC), including inoperable or metastatic non-small cell lung cancer.

Fig. 1

Fig. 2

- Patients with unresectable/metastatic melanoma regardless of BRAF status
- Previously untreated patients or patients who received therapy without using target drugs and anti-PD-1/ PD-L1/ CTLA-4 drugs, with the disease progression following or against the background of therapy received.
(n=126)

BCD-100
1 mg/kg Q2W
(n=63)

BCD-100
3 mg/kg Q3W
(n=63)

1 year of therapy before progression or intolerable toxicity

Fig. 3

Randomized (n=131)

n=65

n=66

Distributed in
BCD-100 product 1 mg/kg group
(mITT population) (n=63)

Distributed in
BCD-100 product 3 mg/kg group
(mITT population) (n=63)

Excluded from mITT population and
substituted (n=2) due to:
  • discontinuation prior to first
    administration (n=1)
  • serious deviations from
    Protocol (n=1)

Excluded from mITT population and
substituted (n=3) due to:
  • discontinuation prior to first
    administration (n=1)
  • serious deviations from
    Protocol (n=2)

Undergone 1 scheduled CT response
assessment (PP population)
(n=59)

Undergone 1 scheduled CT response
assessment (PP population)
(n=55)

Completed 12 months of therapy
according to Protocol (n=21)

Completed 12 months of therapy
according to Protocol (n=17)

Discontinued prematurely (less than 12 months of
therapy)
                              (n=42)
  • Lost for observation (n=1)
  • Consent withdrawal (n=1)
  • Due to AE (n = 3)
  • Disease progression (n=37), 17 out of them
    with following fatal outcome (FO)

Discontinued prematurely (less than 12 months of
therapy)
                              (n=46)
  • Lost for observation (n=1)
  • Refused treatment with study product
    (n=1), 1 out of them with following FO
  • Withdrawn consent (n=3), 3 out of them
    with following FO
  • Due to AE (n=9), 7 out of them with
    following fatal outcome
  • Disease progression (n=32), 20 out of
    them with following FO

Fig. 4

Fig. 5

Fig. 6

| | | | | | | | | | | |
|---|---|---|---|---|---|---|---|---|---|---|
| Participate in trial | | | | | | | | | | |
| BCD-100 3 mg/kg | 63 | 60 | 52 | 48 | 39 | 35 | 30 | 21 | 7 | 0 |
| Total deaths | | | | | | | | | | |
| BCD-100 3 mg/kg | 0 | 3 | 10 | 14 | 21 | 24 | 29 | 29 | 30 | 31 |
| Total censored | | | | | | | | | | |
| BCD-100 3 mg/kg | 0 | 0 | 1 | 1 | 3 | 4 | 4 | 13 | 26 | 32 |

Fig. 7

Fig. 8

Fig. 9

Fig. 10

Fig. 11

Fig. 12

## INTERNATIONAL SEARCH REPORT

| International application No. |
| --- |
| PCT/RU 2020/050197 |

**A.   CLASSIFICATION OF SUBJECT MATTER**
See supplemental sheet

According to International Patent Classification (IPC) or to both national classification and IPC

**B.   FIELDS SEARCHED**

Minimum documentation searched (classification system followed by classification symbols)
A61K, A61P

Documentation searched other than minimum documentation to the extent that such documents are included in the fields searched

Electronic data base consulted during the international search (name of data base and, where practicable, search terms used)
RUPTO, DWPI, Espacenet, PatSearch, USPTO, Google Patents

**C.   DOCUMENTS CONSIDERED TO BE RELEVANT**

| Category* | Citation of document, with indication, where appropriate, of the relevant passages | Relevant to claim No. |
| --- | --- | --- |
| X | WO 2018/013017 A1 (ZAO «BIOKAD») 18.01.2018, points 21-24; p. 3, lines 26-28; p. 45, line 19; p. 46, lines 8-13; p. 47, lines 5-11, 18-23, 31-34; p. 48, lines 9-16; p. 51, lines 7-11, 14-15; p. 56, lines 26-30; p. 56, line 35 - p. 57, line 1; p. 57, line 33- p. 58, line 2; examples 17, 19 | 89-92, 95-98 |
| Y | | 1-51,64-87,93-94 |
| Y | US 2018/0369377 A1 (RINALDI G. et al.) 27.12.2018, [0065], [0067], [0068], [0070], [0128]-[0134], [0143]-[0150], [0269]-[0301] | 1-51,64-87,93-94 |
| Y | WO 2017/198741 A1 (BOEHRINGER INGELHEIM INTERNATIONAL GMBH) 23.11.2017, p. 48, lines 2-5, 9-10, 13-15; p. 50, line 25- p. 51, line 9; p. 51, line 14 - p. 52, line 18; examples 14, 15 | 9-36,40-42,45-46 |

☐   Further documents are listed in the continuation of Box C.          ☐   See patent family annex.

| | |
| --- | --- |
| *         Special categories of cited documents: | "T"   later document published after the international filing date or priority date and not in conflict with the application but cited to understand the principle or theory underlying the invention |
| "A"   document defining the general state of the art which is not considered to be of particular relevance | |
| "E"   earlier application or patent but published on or after the international filing date | "X"   document of particular relevance; the claimed invention cannot be considered novel or cannot be considered to involve an inventive step when the document is taken alone |
| "L"   document which may throw doubts on priority claim(s) or which is cited to establish the publication date of another citation or other special reason (as specified) | "Y"   document of particular relevance; the claimed invention cannot be considered to involve an inventive step when the document is combined with one or more other such documents, such combination being obvious to a person skilled in the art |
| "O"   document referring to an oral disclosure, use, exhibition or other means | |
| "P"   document published prior to the international filing date but later than the priority date claimed | "&"   document member of the same patent family |

| Date of the actual completion of the international search | Date of mailing of the international search report |
| --- | --- |
| 02 November 2020 (02.11.2020) | 19 November 2020 (19.11.2020) |
| Name and mailing address of the ISA/ RU | Authorized officer |
| Facsimile No. | Telephone No. |

Form PCT/ISA/210 (second sheet) (April 2005)

## INTERNATIONAL SEARCH REPORT

| International application No. |
|---|
| PCT/RU 2020/050197 |

**Box No. II      Observations where certain claims were found unsearchable (Continuation of item 2 of first sheet)**

This international search report has not been established in respect of certain claims under Article 17(2)(a) for the following reasons:

1. [X] Claims Nos.: 52-63
   because they relate to subject matter not required to be searched by this Authority, namely:

2. [ ] Claims Nos.:
   because they relate to parts of the international application that do not comply with the prescribed requirements to such an extent that no meaningful international search can be carried out, specifically:

3. [X] Claims Nos.: 52-63
   because they are dependent claims and are not drafted in accordance with the second and third sentences of Rule 6.4(a).

**Box No. III      Observations where unity of invention is lacking (Continuation of item 3 of first sheet)**

This International Searching Authority found multiple inventions in this international application, as follows:

1. [ ] As all required additional search fees were timely paid by the applicant, this international search report covers all searchable claims.

2. [ ] As all searchable claims could be searched without effort justifying additional fees, this Authority did not invite payment of additional fees.

3. [ ] As only some of the required additional search fees were timely paid by the applicant, this international search report covers only those claims for which fees were paid, specifically claims Nos.:

4. [ ] No required additional search fees were timely paid by the applicant. Consequently, this international search report is restricted to the invention first mentioned in the claims; it is covered by claims Nos.:

**Remark on Protest**      [ ] The additional search fees were accompanied by the applicant's protest and, where applicable, the payment of a protest fee.

[ ] The additional search fees were accompanied by the applicant's protest but the applicable protest fee was not paid within the time limit specified in the invitation.

[ ] No protest accompanied the payment of additional search fees.

Form PCT/ISA/210 (continuation of first sheet (2)) (January 2015)

*A61K 39/395* (2006.01)

*A61K 9/08* (2006.01)

*A61K 47/12* (2006.01)

*A61K 47/18* (2017.01)

*A61K 47/26* (2006.01)

*A61P 35/00* (2006.01)

Form PCT/ISA/210 (extra sheet) (January 2015)

**REFERENCES CITED IN THE DESCRIPTION**

*This list of references cited by the applicant is for the reader's convenience only. It does not form part of the European patent document. Even though great care has been taken in compiling the references, errors or omissions cannot be excluded and the EPO disclaims all liability in this regard.*

**Patent documents cited in the description**

- WO 2018013017 A

**Non-patent literature cited in the description**

- **OKAZAKI et al.** *Curr. Opin. Immunol.,* 2002, vol. 14, 391779-82
- **BENNET et al.** *J Immunol,* 2003, vol. 170, 711-8
- **HUTLOFF et al.** *Nature,* 1999, vol. 397, 263-266
- **HANSEN et al.** *Immunogenics,* 1980, vol. 10, 247-260
- **ISHIDA et al.** *EMBO J,* 1992, vol. 11, 3887-95
- **AGATA et al.** *Int Immunol,* 1996, vol. 8, 765-72
- **THOMAS, M.L.** *J Exp Med,* 1995, vol. 181, 1953-6
- **DAERON, M.** *Immunol Today,* 1997, vol. 18, 286-91
- **FREEMAN et al.** *J Exp Med,* 2000, vol. 192, 1027-34
- **LATCHMAN et al.** *Nat Immunol,* 2001, vol. 2, 261-8
- **CARTER et al.** *Eur J Immunol,* 2002, vol. 32, 634-43
- **DONG et al.** *Nat. Med.,* 2002, vol. 8, 787-9
- **DONG et al.** *J. Mol. Med.,* 2003, vol. 81, 281-7
- **BLANK et al.** *Cancer Immunol. Immunother.,* 2005, vol. 54, 307-314
- **KONISHI et al.** *Clin. Cancer Res.,* 2004, vol. 10, 5094-100
- **IWAI et al.** *Proc. Nat'l. Acad. Sci. USA,* 2002, vol. 99, 12293-7
- **BROWN et al.** *J. Immunol.,* 2003, vol. 170, 1257-66
- **OKAZAKI et al.** *Curr Opin Immunol,* 2002, vol. 14, 391779-82
- **BENNETT et al.** *J Immunol,* 2003, vol. 170, 711-8
- **NISHIMURA et al.** *Immunity,* 1999, vol. 11, 141-51
- **NISHIMURA et al.** *Science,* 2001, vol. 291, 319-22
- **SALAMA et al.** *J Exp Med,* 2003, vol. 198, 71-78
- **PROKUNINA ; ALARCON-RIQUELME.** *Hum Mol Genet,* 2004, vol. 13, R143
- **NIELSEN et al.** *Lupus,* 2004, vol. 13, 510
- **OKAZAKI et al.** *PNAS,* 2001, vol. 98, 13866-71
- **LANGER.** *Science,* 1990, vol. 249, 1527-1533
- **NONOMURA Y ; OTSUKA A ; HAKASHIMA C ; SEIDEL JA ; KITOH A ; DAINICHI T ; HAKAJIMA S ; SAWADA Y ; MATSUSHITA S ; AOKI M.** Peripheral blood Th9 cells are a possible pharmacodynamic biomarker of nivolumab treatment efficacy in metastatic melanoma patients. *Oncoimmunology,* 18 October 2016, vol. 5 (12), e1248327